(19) Europäisches Patentamt
European Patent Office
Office européen des brevets

(11) **EP 3 769 842 A1**

(12) **EUROPÄISCHE PATENTANMELDUNG**

(43) Veröffentlichungstag:
27.01.2021 Patentblatt 2021/04

(21) Anmeldenummer: 20194310.7

(22) Anmeldetag: **13.07.2018**

(51) Int Cl.:
*B01L 3/02* (2006.01)   *G01N 35/10* (2006.01)
*G01N 21/25* (2006.01)   *G01N 35/00* (2006.01)
*G01N 35/04* (2006.01)   *G01N 35/02* (2006.01)
*B01L 3/00* (2006.01)   *G01N 21/00* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.07.2017   AT 505932017
14.07.2017   AT 505952017
23.04.2018   AT 503402018
23.04.2018   AT 503412018**

(62) Dokumentnummer(n) der früheren Anmeldung(en) nach Art. 76 EPÜ:
**18748848.1 / 3 651 905**

(71) Anmelder: **Meon Medical Solutions GmbH & Co. KG**
**8010 Graz (AT)**

(72) Erfinder:
• **LIMBACH, Berthold**
**4932 Lotzwil (CH)**
• **BARTEL, Arnold**
**8051 Graz (AT)**
• **HUEMER, Herfried**
**8330 Feldbach (AT)**
• **MARIK, Reinhard**
**8020 Graz (AT)**
• **SPRENGERS, Wolfgang**
**8081 Vasoldsberg (AT)**
• **KRAUS-FÜREDER, Patrick**
**8042 Graz (AT)**
• **SCHOLZ-MAREICH, Robert**
**8054 Graz (AT)**

(74) Vertreter: **Babeluk, Michael**
**Florianigasse 26/3**
**1080 Wien (AT)**

Bemerkungen:
Diese Anmeldung ist am 03-09-2020 als Teilanmeldung zu der unter INID-Code 62 erwähnten Anmeldung eingereicht worden.

(54) **AUTOMATISCHER ANALYSATOR UND VERFAHREN ZUR DURCHFÜHRUNG VON CHEMISCHEN, BIOCHEMISCHEN UND/ODER IMMUNCHEMISCHEN ANALYSEN**

(57) Die Erfindung betrifft ein Verfahren und eine Vorrichtung zur Durchführung von chemischen, biochemischen und/oder immunchemischen Analysen von flüssigen Proben, die in einem Probenlager (920) eines automatischen Analysators (100) vorliegen, unter Zuhilfenahme von flüssigen Reagenzien, die in zumindest einem Reagenzienlager (950a, 950b) des Analysators (100) vorliegen, mit Küvetten (201) zur Aufnahme der flüssigen Proben und Reagenzien, wobei eine Vielzahl von Küvetten als zumindest ein stationäres, lineares Küvettenarray (200) im Analysator angeordnet ist. Der Analysator weist verfahrbare und stationäre Automatenkomponenten auf, wobei zumindest zwei Automatenkomponenten unabhängig voneinander entlang oder parallel zu der durch das lineare Küvettenarray (200) definierten Bewegungslinie in x-Richtung verfahrbar ausgeführt sind und jeweils Zugriff auf unterschiedliche Küvetten (201) oder Gruppen von Küvetten (201) in frei wählbarer Reihenfolge aufweisen.

Fig. 3a

EP 3 769 842 A1

**Beschreibung**

Die Erfindung betrifft einen automatischen Analysator zur Durchführung von chemischen, biochemischen und/oder immunchemischen Analysen von flüssigen Proben, die in einem Probenlager des Analysators vorliegen, unter Zuhilfenahme von flüssigen Reagenzien, die in zumindest einem Reagenzienlager des Analysators vorliegen, sowie ein Verfahren zur automatischen chemischen, biochemischen und/oder immunchemischen Analyse von flüssigen Proben.

Automatisierte Analysatoren bzw. Analysengeräte werden routinemäßig, beispielsweise in der klinischen Diagnostik, der Analytik und der Mikrobiologie, verwendet, wobei die Notwendigkeit besteht, verschiedene Eigenschaften und Inhaltsstoffe von flüssigen Proben vor allem mit optischen Verfahren schnell, exakt und reproduzierbar zu bestimmen.

Bei den bekannten Analysegeräten kommen unterschiedliche Messprinzipien zur Anwendung. Zum einen werden Geräte mit einer stationären Detektionseinheit, beispielsweise einem stationären Photometer, sowie einem scheibenförmigen, drehbaren Halter mit Küvetten zur Aufnahme der zu vermessenden Reaktionsgemische aus Proben und Reagenzien verwendet. Die Küvetten werden sukzessive an der Detektionseinheit vorbeigeführt und durchgemessen. Demzufolge muss das Küvettenkarussell jedes Mal anhalten, wenn eine neue Probe oder ein Reagenz in eine Küvette eingebracht wird oder die Küvette gewaschen und für einen neuen Test bereitgestellt werden soll. Mit den konzeptionell starr vorgegebenen Zykluszeiten geht eine deutliche Effizienzeinbuße einher. Nähere Ausführungen dazu sind der Diskussion zum Stand der Technik (siehe Punkt A) zu entnehmen.

Fotometrie

Bei dem der fotometrischen Messung zugrundeliegenden physikalischen Effekt handelt es sich um die Absorption von Licht bestimmter Wellenlängen durch bestimmte, in einer Flüssigkeit vorhandene Substanzen. Die daraus folgende Verringerung der Intensität des durch die Küvette durchgehenden Lichts wird messtechnisch erfasst und erlaubt eine quantitative Ermittlung der Konzentration einer Substanz unter Einbeziehung der folgenden Gleichungen:

$$T = I \ / \ I_0 \qquad \text{(Eq 1)}$$

$$E = - \log T = \log (I_0 \ / \ I) \qquad \text{(Eq 2)}$$

$$E = \varepsilon \cdot c \cdot d \qquad \text{(Eq 3)}$$

Lambert-Beer'sches Gesetz
wobei

| | |
|---|---|
| $T$ | Transmission |
| $E$ | Extinktion |
| $I_0$ | Intensität in Abwesenheit der Licht absorbierenden Substanz |
| $I$ | Intensität in Anwesenheit der Licht absorbierenden Substanz |
| $c$ [mol / l] | Stoffmengenkonzentration |
| $d$ [cm] | Dicke der absorbierenden Flüssigkeitsschicht |
| $\varepsilon$ [l mol$^1$ cm$^{-1}$] | molarer Extinktionskoeffizient (stoffabhängige Größe) |

Die Stoffmengenkonzentration $c$ lässt sich also direkt aus dem Ergebnis einer Extinktions- bzw. Transmissionsmessung berechnen. Diese Art der Messung kommt bei chemischen und enzymatischen Reaktionen zur Bestimmung der Stoffmengenkonzentration bestimmter in der Probe (Blutplasma, Harn, etc.) vorhandener Analyte zum Einsatz. Dabei entstehen oder verschwinden Licht absorbierende Substanzen (Farbstoffe), aus deren Extinktion oder Änderungen der Extinktion dann auf die Stoffmengenkonzentration der zu bestimmenden Analyte geschlossen wird.

Im Bereich der klinisch-chemischen Analyse erfolgt die Bestimmung zahlreicher Parameter mit Hilfe fotometrischer Methoden, z. B. die Bestimmung von Enzymen (AP, GOT, GPT, $\gamma$-GT, Amylase, CK), Elektrolyten (Na$^+$, K$^+$, Ca$^{2+}$, Cl$^-$, Mg$^{2+}$), organspezifischen Substanzen (Herz, Leber, Niere) und zahlreichen Stoffwechselgrößen (Bilirubin, Gesamt-, HDL- und LDL-Cholesterin, Triglyzeride, Glukose, Harnsäure, Kreatinin, Harnstoff und Laktat).

## Turbidimetrie und Nephelometrie

Diese Art der Messung kommt bei homogenen Immunoassays zum Einsatz, wobei bestimmte Analyte, wie beispielsweise Metabolite, Enzyme, Peptide oder Proteine, mit Antikörpern, in Reaktion gebracht werden. Dabei entstehen größere Strukturen, die eine erhöhte Lichtstreuung bzw. eine Trübung des Reaktionsgemisches verursachen.

Während sich bei der Transmissionsmessung mit zunehmender Analytkonzentration die Intensität des durchgehenden Lichtstrahls in Folge der zunehmenden Trübung abschwächt, erhöht sich in einem Detektionswinkel von beispielsweise 90° die Intensität des gestreuten Lichtstrahls mit zunehmender Trübung.

Die Trübungsmessung in Form der Transmissionsmessung wird als Turbidimetrie bezeichnet. Die betreffende Messvorrichtung als Turbidimeter. Die in einem Winkel von beispielsweise 90° zum durchgehenden Lichtstrahl erfolgende Streulichtmessung wird als Nephelometrie bezeichnet, die betreffende Messvorrichtung als Nephelometer.

## Lumineszenz/Chemilumineszenz

Bei der Lumineszenz (z.B. Fluoreszenz, Phosphoreszenz, Chemolumineszenz) wird das von Molekülen emittierte Licht gemessen. Im Fall der Chemolumineszenz erfolgt die Lichtemission infolge einer chemischen Reaktion. Luminometrische Methoden sind sehr empfindlich und daher gut für die Detektion von Markern in Immunoassays geeignet.

Zum besseren Verständnis der Erfindung werden einige wesentliche in der gegenständlichen Anmeldung verwendete technische Begriffe näher definiert:

Analysator: Gerät zur Durchführung von chemischen, biochemischen und/oder immunchemischen Analysen flüssiger Proben, die in einem, im Analysator vorhandenen Probenlager vorliegen, unter Zuhilfenahme flüssiger Reagenzien, die in zumindest einem, im Analysator vorhandenen Reagenzienlager vorliegen.

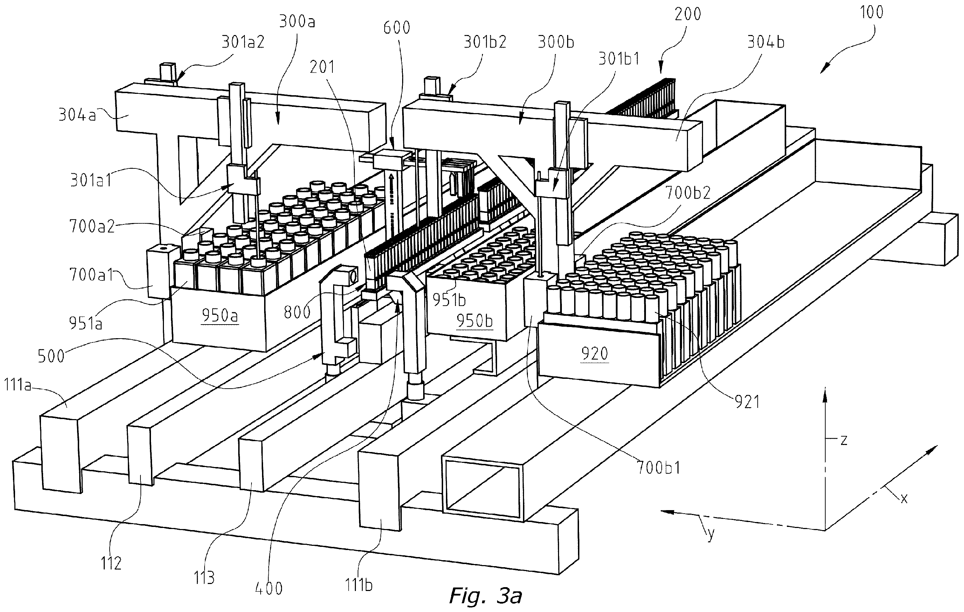

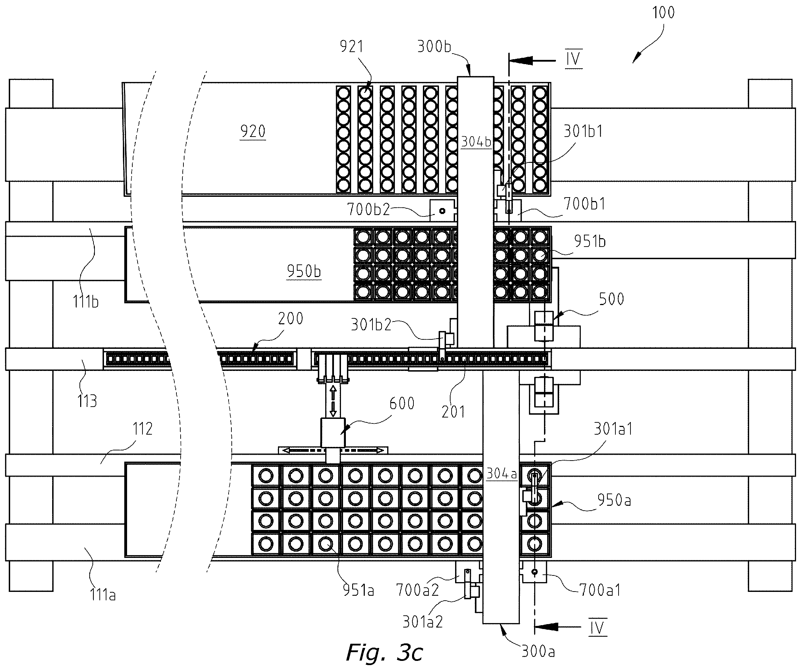

x-, y- und z-Achse: x-Achse bedeutet die horizontal verlaufende Längsachse, y-Richtung die horizontal verlaufende Breiten- oder Tiefenachse, und z- die vertikal verlaufende Höhenachse des Analysators (siehe z.B. Fig. 3a).

Küvette: Eine Küvette im Sinne der vorliegenden Erfindung bezeichnet ein allseitig verschlossenes, nach oben hin offenes und thermostatisierbares Gefäß zur Aufnahme von Proben- und Reagenzienflüssigkeiten und der sich daraus ergebenden Reaktionsgemische und dient zur Vermessung der Reaktionsgemische mittels photometrischer und/oder lumineszenzoptischer Verfahren. Eine Küvette im Sinne der vorliegenden Erfindung weist zumindest ein für das angewandte optische Messverfahren durchlässiges, in einer Seitenwand der Küvette angeordnetes Fenster auf oder ist zur Gänze optisch transparent ausgeführt.

stationäres Küvettenarrav: Bezeichnet eine Vielzahl aneinander gereihter Küvetten, welche ortsfest im Analysator angeordnet sind und während des gewöhnlichen Messbetriebs entlang keiner der x-, y- und z-Achsen bewegt werden.

lineares Küvettenarray: Bezeichnet eine einzige, entlang einer geraden Line angeordnete Reihe einer Vielzahl an Küvetten.

Reaaenzienaefäß: Gefäß bzw. Behälter zur Aufnahme von Reagenzien, die für die Durchführung der Analyse benötigt werden.

Probenaefäß: Gefäß bzw. Behälter, welches bzw. welcher im Analysator die Analysenprobe (die zu analysierende Probe) enthält, aus welchem für die Analyse einzelner Analyte oder Parameter mehrfach kleinere Probenmengen (Aliquote) entnommen werden können. Die Analyse erfolgt hierbei nicht im Gefäß der Analysenprobe, sondern nach Zugabe der Reagenzien in der Küvette, welche in diesem Sinne als Reaktionsgefäß dient.

Analysenprobe: Als Analysenprobe (meist nur Probe oder Stoffprobe genannt) wird das in den Analysator eingebrachte, zu untersuchenden Material bezeichnet. Dieses Material ist ein flüssiges Stoffgemisch und kann beispielsweise eine Körperflüssigkeit wie z.B. Blutserum, Blutplasma, Urin und Liquor sein. Weitere Stoffgemische sind z.B. Trinkwasser, Abwässer, Wein, Bier und Fruchtsäfte sowie Flüssigkeiten aus chemischen und biochemischen Herstellungsprozessen.

Analyt: Als Analyt bzw. Analyte (auch als Parameter) bezeichnet werden diejenigen in einer Analysenprobe enthaltenen Stoffe, über die mit einem Analysator über eine chemische Analyse unter Zuhilfenahme flüssiger Reagenzien eine Aussage getroffen werden soll, d.h. welche unter Angabe der Konzentration quantitativ bestimmt werden.

Analyse: Als Analyse bzw. Test (bei immunchemischen Analysen auch als Immunoassay) bezeichnet werden die mit einem Analysator automatisch durchgeführten qualitativen und/oder quantitativen Bestimmungen eines in der Analysenprobe nachzuweisenden bzw. enthaltenen Analyten unter Zuhilfenahme flüssiger Reagenzien.

Pipettiereinheit: Bezeichnet das Gesamtsystem einer automatischen Pipettiervorrichtung zum Flüssigkeitstransfer zwischen verschiedenen Gefäßen, welches einen oder mehrere bewegliche Pipettoren samt aller für deren Funktion notwendigen mobilen und stationären Komponenten, inklusive zuführender Fluidik (Schlauchverbindungen, Pumpen, Ventile, Behälter, etc.), Sensorik, Steuerung und Stromversorgung umfasst.

Pipettor: Beschreibt eine in Bezug auf die Aufnahmegefäße (Küvetten, Probengefäße, Reagenziengefäße) horizontal in mindestens einer Richtung linear bewegliche oder schwenkbare Komponente der Pipettiereinheit. Der Pipettor beinhaltet eine Aufhängungskomponente mit mindestens einer Pipettiernadel, welche allein oder gemeinsam mit dem Pipettor beweglich und in ein Aufnahmegefäß absenkbar ist.

Pipettiernadel: Bezeichnet eine, am Pipettor angebrachte Kanüle bzw. Hohlnadel samt deren Halterung zum Aufsaugen von Proben aus den Probengefäßen und/oder zum Aufsaugen von Reagenzien aus den Reagenziengefäßen und zur dosierten Abgabe der aufgesaugten Flüssigkeiten in die Küvetten.

Stationäre Automatenkomponente: Automatenkomponente, welche ortsfest im Analysator angeordnet ist und während des gewöhnlichen Messbetriebs nicht entlang des linearen Küvettenarrays bewegt (verfahren) wird.

Verfahrbare Automatenkomponente: Bezeichnet eine Automatenkomponente, welche nicht ortsfest im Analysator angeordnet ist und während des gewöhnlichen Messbetriebs zumindest entlang des linearen Küvettenarrays mittels gesteuertem Antrieb bewegt und positioniert werden kann.

optische Elemente zur Kollimation: Dabei handelt es sich um optische Elemente zur Erzeugung eines möglichst parallelen Strahlenverlaufs. Grundsätzlich wird dabei das Licht einer mehr oder weniger punktförmigen Quelle in ein paralleles Strahlenbündel verwandelt. Optische Elemente die das von einer LED ausgehende Licht im Wesentlichen parallel ausrichten, sind beispielsweise Sammellinsen, TIR-Linsen, Parabolspiegel, und Blendenanordnungen.

optische Elemente zur Filterung: Dabei handelt es sich um optische Bauelemente, insbesondere um Interferenzfilter, um das transmittierte Licht wellenlängen- bzw. frequenzabhängig, d.h. farbabhängig für sichtbares Licht, zu filtern. Verwendung finden Bandsperrfilter, Langpassfilter, Kurzpassfilter, Bandpassfilter und dichroitische Interferenzfilter. Besonders bevorzugt sind Bandpassfilter, da sie einen hohen Transmissionsgrad für ein bestimmtes Wellenlängenband aufweisen, während kürzere oder längere Wellenlängen absorbiert werden.

Kondensor bzw. Kondensorlinsen: Dabei handelt es sich um eine Anordnung von ein bis zwei Linsen die einen möglichst großen Teil des Lichts einer LED in eine Küvette einstrahlen bzw. um eine derartige Anordnung, die einen möglichst großen Teil des aus der Küvette austretenden Lichts auf eine Fotodiode lenken.

Thermostatisieren flüssiger Medien: Das Thermostatisieren von flüssigen Medien im Sinne der Erfindung umfasst sowohl das Aufheizen eines Proben-Reagenzgemischs als auch von partikelhaltigen Medien oder Gemischen (Suspensionen) inklusive der Stabilisierung einer erreichten Zieltemperatur.

Analyt/Antigen: Als Analyt - im Fall von Immunoassays auch als Antigen bezeichnet - wird hier ein, in einer Probe qualitativ und/oder quantitativ zu bestimmender Inhaltsstoff bezeichnet. Der Analyt liegt bei Immunoassays in einer flüssigen Phase vor, meist gelöst in einem Puffer, in verdünnten Körperflüssigkeiten oder anderen Probenflüssigkeiten. Darüber hinaus kann der Analyt auch eine, in einer Suspension vorliegende, durch Immunoassays nachweisbare partikuläre Struktur mit antigenen Oberflächenmerkmalen, wie zum Beispiel Bakterien, Viren, Zellen oder Materialpartikel sein.

Immunoassay: Als Immunoassay (deutsch auch.: *Immunassay)* werden zusammenfassend eine Reihe von Methoden in der Bioanalytik bezeichnet, deren gemeinsames Grundprinzip die Erkennung und damit der Nachweis eines Analyten (Antigen) in einer flüssigen Phase durch die Bindung eines Antigens an einen Antikörper ist. Immunoassays werden beispielsweise in der Labormedizin für die Bestimmung einer Vielzahl von Analyten in verschiedenen Körperflüssigkeiten wie Blut, Serum, Urin oder Liquor verwendet.

Kompetitiver Immunoassay: Ein kompetitiver Immunoassay wird zur Bestimmung eines Antigens genutzt, wenn für dieses entweder nur ein einzelner spezifischer Antikörper zur Verfügung steht oder wenn das Antigen nicht über ausreichende Bindungsstellen für die ungehinderte Bindung von zwei Antikörpern verfügt. Dabei wird beispielsweise als Erkennungskomponente ein Antikörper (Fängerantikörper) und als kompetitive Komponente ein mit einem Markermolekül markiertes Antigen verwendet.

Sandwich-Assay: Für den Nachweis eines Antigens mittels eines auch als Sandwich-Assay bezeichneten, nicht-kompetitiven Assays werden zwei verschiedene Antikörper benötigt, die das Antigen erkennen und sich dabei nicht in ihrer Bindung an das Antigen gegenseitig behindern. Von Vorteil beim Vergleich mit dem kompetitiven Immunoassay ist insbesondere die bei den meisten Anwendungen höhere Sensitivität.

heterogener Immunoassay: Bei einem heterogenen Immunoassay der gegenständlichen Erfindung findet - im Gegensatz zum homogenen Immunoassay - im Verlauf des Prozesses ein Wechsel der flüssigen Phase statt. Bei Verwendung magnetischer Partikel mit daran gebundenen Fängerantikörpern zur selektiven Bindung des Antigens kann dies z.B. dadurch erreicht werden, dass die Partikel durch ein Magnetfeld an die Gefäßwand abgeschieden werden, die erste Flüssigkeit durch eine zweite Flüssigkeit ersetzt wird, und die Partikel in der zweiten Flüssigkeit resuspendiert werden. Nach dem Entfernen der ersten Flüssigkeit können an den Partikeln beliebige Waschschritte mit der zweiten Flüssigkeit oder einer speziellen Waschflüssigkeit erfolgen. Die Waschschritte ermöglichen die Entfernung von unspezifisch an den Partikeln gebundenen Substanzen sowie von, in der ersten Flüssigkeit vorhandenen störenden Substanzen, wobei durch die Entfernung störender Substanzen der Assay wesentlich empfindlicher wird und niedrige Nachweisgrenzen und Konzentrationsbereiche für das zu bestimmende Antigen erzielt werden.

magnetische Partikel (magnetic Beads): Dabei handelt es sich um typischerweise wenige $\mu$m große, in einer wässrigen Pufferlösung suspendierte Magnetpartikel, die für immunchemische Tests mit dem Fängerantikörper beschichtet sind.

Fänaerantikörper ("capure Antibody"): Das sind Antikörper, die an zumindest ein Epitop des Analyten binden und an der festen Phase, - im Fall der gegenständlichen Erfindung - an der Oberfläche fester magnetischer Partikel gebunden sind.

Tracer-Antikörper (labeled Antibody, Konjugat): Dabei handelt es sich um zweiten Antikörper, an den chemisch ein Markermolekül (Label) gebunden ist und beim Assay durch Antigen-Antikörper Wechselwirkungen selektiv an Analytmoleküle bindet, oder mit diesem um Bindungsstellen an einem Antigen konkurriert (kompetitiver Assay). Das Markermolekül kann ein Farbstoff sein, der nach Zugabe ein oder mehrerer chemischer Substanzen Licht aussendet (Chemilumineszenz).

Bound/Free-Waschen, bzw. (B/F) - Waschen: Ein Prozessschritt eines heterogenen Immunoassays, bei dem der nicht gebundene Rest der im Überschuss zugegebenen markierten Tracer-Antikörper (labeled antibody) durch Waschen von der Oberfläche der magnetischen Partikel entfernt wird.

Dispensor (bzw. Injektor): Ein Dispensor dient zur Abgabe definierter Flüssigkeitsmengen aus einem Vorratsgefäß über eine Versorgungsleitung die in einer Düse, Abgabeöffnung oder Dispensornadel endet, in ein Gefäß, beispielsweise in eine Küvette.

Dokumente zum Stand der Technik:

A) Analvsensvsteme mit auf Drehtellern kreisförmig angeordneten, verfahrbaren Reaktionsgefäßen/Küvetten (Karussellanordnung)

Aus der US 8,911,685 B2 (HITACHI) ist ein typischer automatischer Analysator zur Durchführung chemischer und biochemischer Analysen flüssiger Proben mittels photometrischer Messverfahren bekannt. Wesentliches Merkmal dieser Analysatoren sind die am peripheren Umfang eines Drehtellers angeordneten Reaktionsgefäße, die zugleich als Küvetten fungieren, sowie stationär entlang des Drehtellerumfangs angeordnete Gerätekomponenten, wie z.B. Pipettoren (Probendispensor, Reagenziendispensor), Mischvorrichtung, optische Messvorrichtung und Küvettenwascheinheit. Die Thermostatisierung der Küvetten kann beispielsweise in Form eines temperaturgeregelten Wasserbads im Drehteller integriert sein. Die Probenbehälter sind auf einem Proben-Drehteller angeordnet, die Reagenzien auf einem Reagenzien-Drehteller lokalisiert.

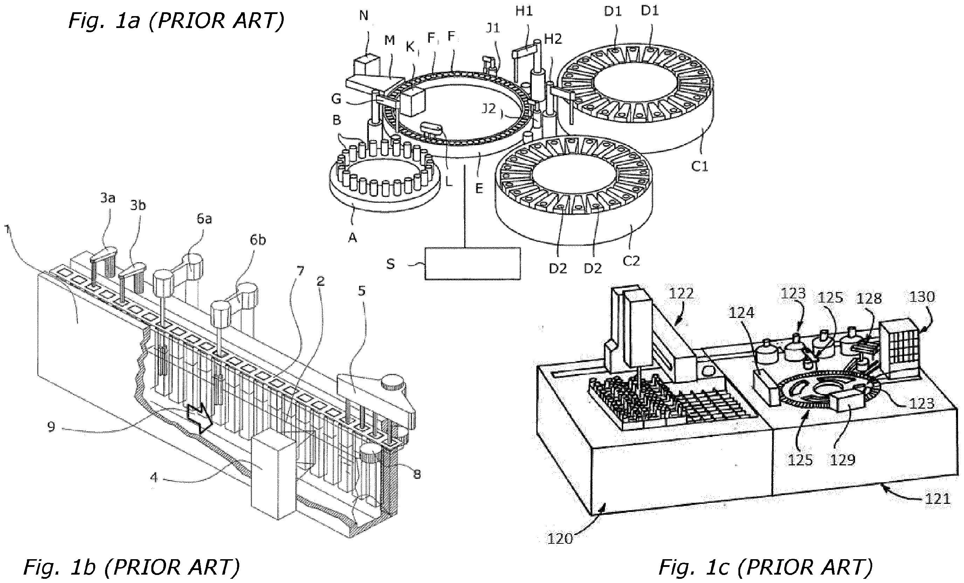

Aus der DE 11 2009 002 702 B4 (HITACHI) ist ein weiterer automatischer Analysator bekannt, dessen Probenbehälter und Reagenzienbehälter in einer Karussellanordnung vorliegen. Wie in **Fig. 1a** der gegenständlichen Anmeldung gezeigt, umfasst der Analysator eine Probenscheibe A, auf der eine Anzahl von Probenbehältern B für die Aufnahme einer Probe angebracht werden kann; eine erste Reagenzienscheibe C1 und eine zweite Reagenzienscheibe C2, auf der jeweils eine Anzahl von Reagenzienbehältern D1 bzw. D2 für die Aufnahme eines ersten Reagenz bzw. eines zweiten Reagenz angeordnet werden kann; und eine Reaktionsscheibe E, auf der entlang der Umfangsrichtung eine Anzahl von Küvetten bzw. Reaktionsbehältern F angeordnet ist.

Zwischen der Reaktionsscheibe E und der Probenscheibe A ist eine Probenabgabevorrichtung G vorgesehen, die eine am Probenbehälter B aufgesaugte Probe in den Reaktionsbehälter F abgibt. Weiterhin ist wischen der Reaktionsscheibe E und der ersten Reagenzienscheibe C1 eine erste Reagenzienabgabevorrichtung H1 vorgesehen, die ein vom Reagenzienbehälter D1 an der ersten Reagenzienscheibe C1 aufgesaugtes Reagenz in den Reaktionsbehälter F abgibt. Gleichermaßen ist zwischen der Reaktionsscheibe E und der zweiten Reagenzienscheibe C2 eine zweite Reagenzienabgabevorrichtung H2 vorgesehen, die ein vom Reagenzienbehälter D2 an der zweiten Reagenzienscheibe C2 aufgesaugtes Reagenz in den Reaktionsbehälter F abgibt. Die Probenabgabevorrichtung G und die beiden Reagenzienabgabevorrichtungen H1 und H2 sind ortsfest an definierten Punkten entlang des Umfangs der Reaktionsscheibe E angeordnet.

Am äußeren Umfang der Reaktionsscheibe E sind zwei ortsfeste Rührer J1, J2, die nach der Abgabe des ersten Reagenz und des zweiten Reagenz die Flüssigkeit in den Reaktionsbehältern F umrühren, eine Lichtquelle K, die Licht durch die Reaktionsbehälter F schickt, und ein Behälter-Reinigungsmechanismus L zum Reinigen der Reaktionsbehälter F, in dieser Reihenfolge in der Rotationsrichtung der Reaktionsscheibe E vorgesehen.

In einer Position gegenüber der Lichtquelle K ist ein ortsfestes, spektroskopisches System M derart angeordnet, dass sich die Reaktionsscheibe E dazwischen befindet. In der Nähe des spektroskopischen Systems ist eine Signalverarbeitungsschaltung N vorgesehen, die die Signale von dem spektroskopischen System M verarbeitet. Die Signalverarbeitungsschaltung N ist mit einem nicht weiter dargestellten Computer verbunden. Der automatische Analysator umfasst weiterhin auch eine Steuerung S, die den Betrieb des Analysators steuert.

Derartige Analysatoren zeichnen sich dadurch aus, dass alle Prozesse durch starre Taktzyklen des Karussells vorgegeben sind und in vorbestimmten Zeitfenstern ablaufen müssen. Aktionen wie Dispensieren, Mischen, Messen und Waschen können nur dann erfolgen, wenn die jeweiligen Küvetten sich an den Positionen der jeweiligen Gerätekomponenten befinden.

So kann eine Probe (nicht jederzeit, sondern) nur dann in eine leere Küvette dispensiert werden, wenn die leere Küvette an der Position des Probenpipettors vorbeifährt und das Küvetten-Karussell an dieser Position stoppt. Ein Reagenz kann nur dann in eine die Probe enthaltende Küvette dispensiert werden, wenn die betreffende Küvette an der Position des Reagenzienpipettors vorbeifährt und das Küvetten-Karussell an dieser Position stoppt. Analoges gilt für das Rühren von Reaktionsgemischen aus der Probe und den Reagenzien in den Küvetten mit mechanischen Rühren und für die optische Messung an der Position der optischen Messeinrichtung.

So kann beispielsweise eine bestimmte Küvette auch nicht jederzeit oder nicht wiederholt in kleinen Zeitintervallen optisch gemessen werden, da erst abgewartet werden muss, bis sich die betreffende Küvette an der Position der optischen Messeinheit befindet bzw. an dieser "on the fly" während der Messung vorbei geführt wird.

Bei abgeschlossenen Reaktionen kann nicht unmittelbar gemessen werden und im Fall kinetischer Messungen sind die Zeitintervalle zwischen den einzelnen Messungen relativ groß (zumindest eine Tellerumdrehung). Bei abgeschlossenen Messungen kann eine Küvette nachteiliger Weise nicht sofort gewaschen und für einen neuen Test bereitgestellt werden. Eine Küvette kann erst dann gewaschen und für einen neuen Test bereit gestellt werden, wenn sich die betreffende Küvette an der Position der Küvettenwaschstation befindet und zu einem fixen Zeitpunkt bzw. einer fixen Zeitdauer ab Testbeginn zu dem/der, entsprechend der konzeptionell starr vorgegebenen Zykluszeiten, an der betreffenden Position ein Waschstop erfolgt (vorgesehen ist). Dadurch sind alle Küvetten gleich lang "blockiert", unabhängig davon, ob die Messdauer an den jeweiligen Tests kurz oder lang ist.

Die rotatorisch organisierte Karussell-Anordnung mit bewegten Proben, Reagenzien und Küvetten, insbesondere aber das Karussell-Konzept mit verfahrbaren Küvetten und stationären Automatenkomponenten, resultiert in verhältnismäßig hohen Durchlaufzeiten für die einzelnen Tests und begrenzt die Anzahl der Tests die pro Stunde auf einem Gerät mit einer bestimmten Anzahl an Küvetten durchgeführt werden können.

**B)** Analysensysteme mit kreisförmig angeordneten, stationären Reaktionsoefäßen/Küvetten

Aus der US 5,178,833 A (BIOSEMA) ist ein automatischer Analysator mit kreisförmig angeordneten, relativ zum Gerät stationär ausgebildeten Messküvetten und Reagenziengefäßen bekannt, wobei die Messküvetten in einem äußeren Ring und die Reagenziengefäße in zwei inneren Ringen angeordnet sind. Im Zentrum der ringförmig angeordneten Reagenziengefäße ist die Drehachse eines stationären Pipettors positioniert, der von einem ringförmigen Waschgefäß für die absenkbare Pipettiernadel des Pipettors umgeben ist. Die Probengefäße des Analysators befinden sich auf einem separaten Drehteller an der Peripherie des stationären Küvettenrings. Eine optische Messeinheit erreicht die Messküvetten mittels einer Drehbewegung um die zentrale Achse des Analysators. Der optische Pfad führt durch die Flüssigkeitsoberfläche entlang der Längsachse der einzelnen Messküvetten. Die Pipettiernadel erreicht die Probengefäße, die Messküvetten, die Reagenziengefäße und das Waschgefäß mittels Drehbewegungen zweier horizontaler Arme des Pipettors um eine erste, zentrale Achse und eine weitere Achse.

Nachteilig ist, dass die geoffenbarte Konfiguration nur eine unabhängig bewegbare Pipettiernadel für Proben- und Reagenzien zulässt, dass das Reagenzienlager auf die Fläche der inneren stationären Ringe begrenzt ist und dass der optische Pfad durch die Oberfläche Reaktionsflüssigkeit verläuft. Nachteilig ist insbesondere, dass die Messküvetten nicht gewaschen werden können, sondern nach Gebrauch sektorweise mit dem äußeren Ring ausgetauscht werden müssen.

**C)** Analysensysteme mit linear angeordneten, verfahrbaren Reaktionsaefäßen/Küvetten

Aus der GB 1 321 754 A ist ein automatischer Analysator mit an linear verfahrbaren, umlaufenden Endlosbändern fixierten Reaktionsgefäßen/Küvetten bekannt.

Aus der US 2014/0287523 A1 (ABBOTT) ist ebenfalls ein Analysator mit auf Bändern linear angeordneten Reaktionsgefäße bzw. -küvetten bekannt. Die linearen Endlosbänder sind auf zwei Umlenkrollen aufgespannt, wobei in Längsrichtung beispielsweise in einer "pretreatement lane" und in einer "primary process lane" entsprechende Reaktionsgefäße befestigt sind. Durch Drehung der Rollen können die Reaktionsgefäße bzw. Küvetten in Laufrichtung des Bandes hin und her bewegt werden und auch die Rollen auf der Unterseite umfahren. Die Anordnung läuft auf eine "lineare Variante" der klassischen Karussellanordnung hinaus, bei welcher sich die Reaktionsgefäße bzw. Küvetten auf einer Kreisbahn bewegen. Beiden Varianten gemeinsam ist jedoch, dass die Reaktionsgefäße bzw. Küvetten nach wie vor relativ zum Gerät bewegt und zu den Bearbeitungsstationen (Automatenkomponenten) hin gefahren werden. Es treten daher im Wesentlichen dieselben Nachteile auf, die bereits zu Punkt **A)** angeführt wurden.

Die WO 99/046601 A1 (HITACHI) zeigt ein lineares, verfahrbares Küvettenarray mit stationären Gerätekomponenten (Dispensoren für Probenflüssigkeit und Reagenzien, mechanische Rührwerke, Photometer und Küvettenwaschstation).

Wie in **Fig. 1b** der gegenständlichen Anmeldung dargestellt ist, sind in der WO 99/046601 A1 eine Vielzahl von Küvetten bzw. Reaktionsgefäßen 2 in einem Stützrahmen bzw. einer Transportleiste 7 in vorbestimmten Abständen in einer thermostatisierten Kammer (Wasserbad) 1 angeordnet. Die Mischung des Küvetteninhalts erfolgt beispielsweise mittels Ultraschall. Die Transportleiste mit den Reaktionsgefäßen 2 wird mit Hilfe einer Antriebseinheit 8 in Pfeilrichtung 9 linear bewegt. Ferner sind neben der thermostatisierten Kammer 1 eine Probenpipettiereinheit 3a, eine Reagenzinjektionseinheit 3b, eine optische Messeinheit 4, eine Küvettenwascheinheit 5, sowie ein erster Rührmechanismus 6a und ein zweiter Rührmechanismus 6b zum erneuten Rühren des Inhalts der Reaktionsgefäße 2 vorgesehen. Der Rührmechanismus 6a bzw. 6b kann auch als Ultraschallgenerator ausgeführt sein, der über das Wasserbad in der Kammer 1 auf die Reaktionsgefäße 2 einwirkt. Bei dieser Ausführungsvariante wird das Wasser in der thermostatisierten Kammer 1 auf konstanter Temperatur gehalten, bei welcher die Reaktionen ablaufen und die optische Messung durchgeführt werden kann.

Im Betrieb der Vorrichtung stoppt ein Reaktionsgefäß 2 bei der Probenpipettiereinheit 3a, welche die Probe in das Reaktionsgefäß 2 abgibt. Ebenso entlädt die Reagenzinjektionseinheit 3b das für die Untersuchung verwendete Reagenz in das entsprechende Reaktionsgefäß 2. Zusätzlich rührt der erste Rührmechanismus 6a zum Mischen der Reaktionslösung und der zweite Rührmechanismus 6b erneut die Mischung im Reaktionsgefäß 2. Die optische Messeinheit 4 misst die Absorption im entsprechenden Reaktionsgefäß. Weiterhin entsorgt die Küvettenwascheinheit 5 die getestete Reaktionslösung und reinigt das Reaktionsgefäß 2. Nach der Beendigung dieser Vorgänge wird die Bewegung der Reaktionsbehälter 2 durch die Antriebseinheit 8 gestartet. Während sich die Reaktionsbehälter 2 weiterbewegen, werden die Probenpipettiereinheit 3a, die Reagenzinjektionseinheit 3b sowie der erste und der zweite Rührmechanismus 6a, 6b in einer Reinigungseinheit gewaschen. Eine Anzahl von chemischen Analysen wird durch Wiederholen des obigen Vorgangs durchgeführt. Wie aus dem obigen Vorgang ersichtlich ist, müssen die einzelnen Komponenten der Vorrichtung in der genannten Reihenfolge entlang der Bewegungsrichtung 9 angeordnet sein.

Nachteilig an diesem Konzept ist, dass die Transportleiste 7 zwangsläufig links bzw. rechts der stationären Gerätekomponenten 3a, 3b, 6a, 6b und 5 viel Freiraum für die lineare Bewegung der Reaktionsgefäße 2 benötigt. Damit vergrößert sich die Längsachse des Analysators zwangsläufig um zumindest das Doppelte der Länge der Transportleiste 7.

Die Küvetten bzw. Reaktionsgefäße 2 der Vorrichtung gemäß der WO 99/046601 A1 werden somit - analog der oben beschriebenen Drehtellervariante - an den stationären Gerätekomponenten vorbei bewegt. Das System ist unflexibel, es treten im Wesentlichen jene Nachteile auf, die bereits zu Punkt **A)** angeführt wurden.

**D)** Systeme mit kreisförmig und/oder linear angeordneten stationären Reaktionsaefäßen/Küvetten

Aus der EP 2 309 251 A1 (SIEMENS) ist ein automatischer Analysator mit stationären, in kreisförmiger oder linearer Anordnung vorliegenden Probengefäßen bzw. Küvetten bekannt, wobei die optische Messeinheit auf einer drehbaren Einrichtung entlang der Probengefäße verfahrbar ausgeführt ist. Gemäß einer Ausführungsvariante kann die drehbare Einrichtung, die die Lichtquelle in Form einer LED und den Photodetektor in Form einer Photodiode trägt, unterhalb der Aufnahme der Probengefäße angeordnet sein, wodurch es jederzeit möglich ist, auf die Probengefäße mittels eines Greifarms zuzugreifen. Die drehbare Einrichtung kann auch mehrere LEDs unterschiedlicher Wellenlängen und mehrere Photodioden aufweisen, damit die Proben bei mehreren Wellenlängen gemessen werden können. Die Photodioden können durch ein CCD-Element ersetzt sein.

Die in der EP 2 309 251 A1 beschriebene Anordnung ist für klinisch chemische Analysatoren (CC-Analysatoren) ungeeignet und auf einen Analysator für hämostatische Messungen (zur Bestimmung der Blutgerinnung) gerichtet. Diese Anordnung kann auch Teil eines Systems aus mehreren Geräten (z.B. PCR-Analysator, Kühlgerät) sein. Die Probengefäße werden nicht wiederverwendet sondern gegebenenfalls zu weiteren Komponenten eines Systems weitergereicht, z.B. mittels eines Greifarms oder nach der Bestimmung der Gerinnungsparameter entsorgt.

Bei Gerinnungsmessungen kommt als Probe nur Vollblut (Blutplasma mit den darin enthaltenen Blutzellen) in möglichst unverdünnter Form in Frage. Hingegen ist Vollblut für die photometrischen Messungen des gegenständlichen CC-Analysators vollkommen ungeeignet, da die Blutzellen das Licht streuen, und damit die Messergebnisse verfälscht würden. Daher verwenden CC-Analysatoren immer Blutplasma oder Blutserum, welches zudem durch den Zusatz von Reagenzien stark verdünnt wird.

Gemäß der EP 2 309 251 A1 werden die Gefäße mit den eingehenden Proben (gegebenenfalls nach der Zugabe von Reagenzien) direkt für die optische Messung verwendet.

Bei einem CC-Analysator wird immer mit zellfreiem Blutplasma/Blutserum gemessen, welches mittels Probengefäßen in das Gerät eingebracht wird, wonach Aliquote der Proben mittels Pipettor zusammen mit Reagenzien in separate Küvetten überführt werden, welche danach photometrisch vermessen werden.

**E)** Laborroboter und automatische Pipettier- und Analysegeräte zum Aufbereiten und/oder analysieren von Proben mit stationären Reaktionsaefäßen/Küvetten in 2D Anordnung (Mikrotiterplatte)

Ein typisches Analysengerät zur Durchführung biochemischer Analysen flüssiger Proben mit Hilfe von Mikrotiterplatten ist z.B. aus der EP 0 259 386 B1 (TECAN) bekannt. Das Analysengerät umfasst ein Primärrack zur Aufnahme einer Vielzahl von Probengefäßen, einen neben dem Primärrack in x-y Richtung positionierbaren Kreuztisch zur Aufnahme einer Mikrotiterplatte, einen über dem Primärrack und dem Kreuztisch angeordneten, in einer oberen Horizontalebene beliebig positionierbaren Probenverteilerarm und ein innerhalb des Positionierbereichs des Kreuztischs angeordnetes Photometer, dessen Strahlengang die x-y Ebene des Kreuztisches senkrecht durchstößt.

Ein weiteres Beispiel für einen Automaten zum automatischen Aufbereiten und Analysieren von Proben in den Kavitäten (Wells) einer Mikrotiterplatte ist aus der DE 10 2004 057 450 B4 (CYBIO) bekannt.

Es gibt eine Vielzahl von Automaten dieser Art, die Mikrotiterplatten für den Nachweis und die Bestimmung von Substanzen verwenden. Mikrotiterplatten enthalten viele voneinander isolierte Kavitäten ("wells") in Reihen und Spalten (2D-Arrays). Sie werden für die unterschiedlichsten Arbeitsgänge eingesetzt. Die Pipettierung erfolgt entweder manuell, oder bei Hochdurchsatz-Screening (HTS) mit Hilfe von Pipettierrobotern. Photometrische Bestimmungen, z.B. Absorptionsmessungen an Mikrotiterplatten im Durchlicht mit Photometern erfolgen so, dass der Strahlengang das Well in senkrechter Richtung durch die Flüssigkeitsoberfläche durchwandert. Für genaue quantitative Bestimmungen ist es jedoch unumgänglich, die Lichtstrahlen durch die Messflüssigkeit über möglichst genau definierte und bekannte Wege und Wegstrecken zu leiten. Jede Lichtstreuung an Partikeln, Trübungen, Eintrittsflächen, Oberflächen (z.B. Flüssigkeitsoberfläche, Küvettenwandung) führt zu Lichtverlusten die andererseits das Messergebnis verfälschen.

Aus der EP 2 410 342 A2 (HOFFMANN-LA ROCHE) ist eine Pipettiervorrichtung bekannt, die einen Pipettor mit mehreren, flach bauenden, nebeneinander angeordneten Rahmenelementen aufweist, welche mit deren Pipettiernadeln auf einem Hauptrahmenkörper gemeinsam in einer horizontalen, normal auf den Hauptrahmenkörper stehenden x-Richtung beweglich sind. Die Pipettiervorrichtung dient dazu, um Proben oder Reagenzien von einer ersten Reihe von Gefäßen zu einer in x-Richtung versetzten zweiten Reihe von Gefäßen zu transferieren. Die Pipettiernadeln werden zunächst in y-Richtung auf den Abstand der Gefäße der ersten Reihe justiert, um Proben- oder Reagenzflüssigkeit aufzunehmen und danach - zur Abgabe der Proben- oder Reagenzflüssigkeit - an den Abstand der zweiten Reihe von Gefäßen angepasst. Eine unabhängige Bewegung zweier Pipettiernadeln in x- und y-Richtung ist jedoch nicht vorgesehen. Verfahrmodule für die y-Richtung und die z-Richtung (Heben und Senken der Pipettiernadeln) sind in flachen, benachbarten Rahmenelementen auf Lücke angeordnet, um den Abstand der einzelnen Pipettiernadeln zueinander gering zu halten. Eine unabhängige Bewegung der Pipettiernadeln in y-Richtung ist jedoch nur begrenzt möglich. So ist beispielsweise ein aneinander Vorbeifahren der Rahmenelemente auf dem Transferarm nicht möglich, woraus eine gegenseitige Einschränkung der y-Bewegungsfreiheit der Pipettiernadeln resultiert. Eine sinnvolle Anwendung finden derartige Pipettiervorrichtungen vor allem im Zusammenhang mit Mikrotiterplatten.

Aus der EP 1 230 553 B1 (MAXMAT) ist ein chemischer oder biologischer Analysator bekannt, der ein Lagermodul für Probenröhrchen und Röhrchen für Reagenzien aufweist. Weiterhin ist ein Analysemodul mit einem Reaktionsbehälter in Form einer Mikrotiterplatte, sowie ein auf einer Schiene verfahrbares Entnahmemodul (Pipettor) mit zwei in einem fixen Abstand zueinander angeordneten Pipettiernadeln vorgesehen, die zur automatischen Probenentnahme unabhängig voneinander in z-Richtung arbeiten und jeweils mit einer einziehbaren Ansaugpipette zum Übertragen vorbestimmter Mengen von Proben und Reagenzien vom Lagermodul zum Analysemodul ausgestattet sind. In der horizontalen x- /y-Ebene sind die beiden Pipettiernadeln nur gemeinsam verfahrbar.

Das Analysenmodul weist eine Heizplatte für die Mikrotiterplatte auf, die nahe dem unteren Bereich der Vertiefungen (Wells) der Mikrotiterplatte angeordnet ist, um den Inhalt der Wells durch Konvektion zu erwärmen. Die Entnahmeeinheit umfasst ferner eine Mischvorrichtung, die von einem Elektromagneten gesteuert wird, um eine abwechselnde Hin-und Herbewegung der Pipettiernadel zu bewirken, wenn sich diese in abgesenkter Stellung in einem Well der Mikrotiterplatte befindet, um das Gemisch aus Proben und Reagenzien zu durchmischen.

Die US 5 897 837 A (TOA MEDICAL) offenbart einen Pipettierautomaten, der für die Probenvorbehandlung eines Immunoassay-Analysators geeignet ist, welcher über einen ersten, horizontal in x- und y-Richtung verfahrbaren Block eines Pipettors verfügt, welcher nebeneinander mit zwei Pipettiernadeln ausgestattet ist, die unabhängig voneinander abgesenkt oder angehoben werden können. Hierbei kann eine der beiden Nadeln Reagenzien, die andere Nadel Proben zugeordnet sein. Zusätzlich ist auch ein zweiter, in x-y-Richtung verfahrbarer Block mit einer absenkbaren Pipettiernadel vorhanden. Für die Nadelreinigung muss eine stationäre Nadelwaschstation angefahren werden. In der horizontalen x- /y-Ebene sind die beiden Pipettiernadeln des ersten verfahrbaren Blocks nachteiliger Weise nur gemeinsam verfahrbar. Dies hat den Nachteil, dass die Massen der RoboticKomponenten des Pipettors nicht auf die beiden horizontalen Verfahrachsen x und y aufgeteilt werden können, sodass für das Anfahren von Positionen in y-Richtung die Masse der zweiten Pipettiereinheit stets mitbeschleunigt werden muss. Ebenso muss auch die Masse der Nadelwascheinheit samt Nadelwaschgefäß in beiden horizontalen Richtungen stets mitbeschleunigt werden. Weiters ist es aufgrund der gemeinsamen horizontalen Bewegung nicht möglich, beide Nadeln gleichzeitig für Pipettierungen an unterschiedlichen, nicht benachbarten Positionen einer Gefäßreihe einzusetzen.

**F)** Optische Systemkomponenten für automatische Analysatoren

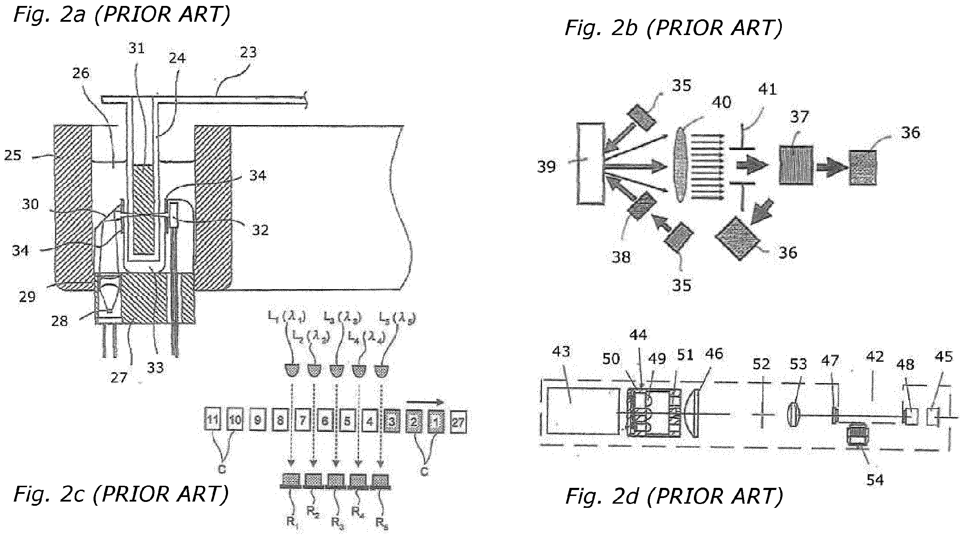

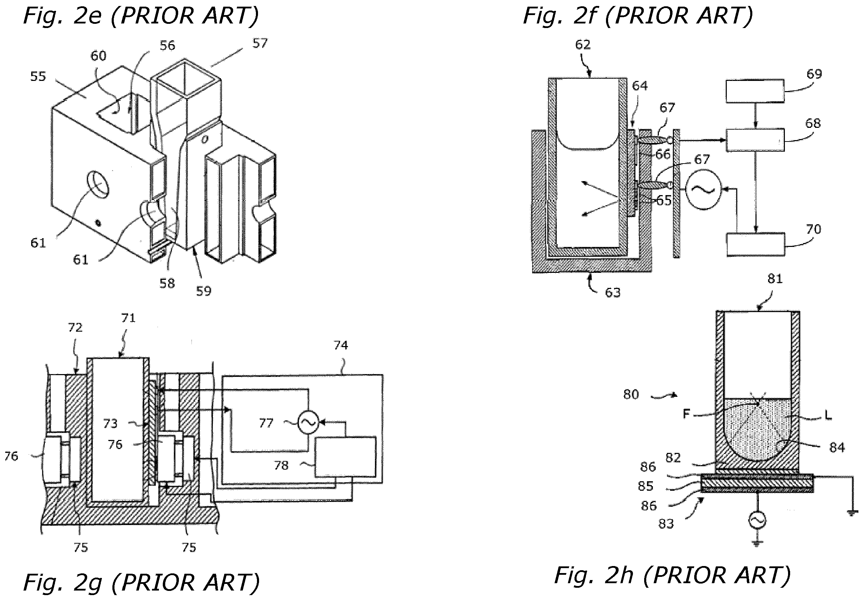

In der US 8,675,187 B2 (Hitachi) wird eine optische Messeinheit zur Gewinnung von Messsignalen von flüssigen Medien und ein damit ausgestattetes Analysesystem beschrieben. Wie in **Fig. 2a** der gegenständlichen Anmeldung dargestellt, wird eines von mehreren an einer Drehscheibe 23 kreisförmig angeordneten Reaktionsgefäßen 24 in ein Temperaturbad 25 eingetaucht, das mit Wasser 26 konstanter Temperatur gefüllt ist. Ein fix im Temperaturbad 25 angeordnetes Fotometer 27 weist eine LED-Lichtquelle 28 auf, deren Licht mittels einer Kondensorlinse 29 und eines Umlenkspiegels 30 in die im Reaktionsgefäß 24 vorliegende Probe 31 eingestrahlt wird. Als Lichtquelle kann auch ein Halbleiterlaser verwendet werden. An der gegenüberliegenden Seite des Reaktionsgefäßes 24 ist ein Fotodetektor 32 des Fotometers 27 angeordnet. Ein- und austrittsseitig des Reaktionsgefäßes 24 sind in der Messposition 33 des Fotometers 27 Blenden 34 für die Ein- und Austrittsstrahlung vorgesehen. Nachteilig ist der mechanische und messtechnische Aufwand im Zusammenhang mit Reaktionsgefäßen die kreisförmig an einer Drehscheibe angeordnet sind, da die einzelnen Reaktionsgefäße 24 zur Vermessung der Proben in eine Messposition des Fotometers 27 bewegt werden müssen.

Die US 2013/0301051 A1 (Pogosyan) beschreibt ein kostengünstiges, portables Fotometer, welches - wie in **Fig. 2b** der gegenständlichen Anmeldung dargestellt - als Lichtquellen 35 mehrere LEDs mit verschiedenen Wellenlängen und als Detektor 36 eine Fotodiode oder einen Fotomultiplier aufweist. Das Fotometer kann zur Untersuchung von chemischen, biologischen oder pharmazeutischen Proben verwendet werden, welche sich in einem Probenhalter 37 zwischen den Lichtquellen 35 und dem Detektor 36 befinden. Das Licht der Lichtquellen 35 wird - ggf. nach der Passage eines Interferenzfilters 38 - auf eine lichtstreuende Fläche 39 gerichtet und gelangt über eine Kollimatorlinse 40 und eine Schlitzblende 41 in die im Probenhalter 37 vorliegende Probe. Der Detektor 36 kann - wie dargestellt - von einer ersten Position in eine zweite Position verschwenkt werden. In der dargestellten Geometrie funktioniert eine Kollimatorlinse optimal, wenn die streuende Fläche sehr klein, quasi punktförmig, gewählt wird, was jedoch die Lichtausbeute schmälert.

Die US 8,064,062 B2 (Beckmann) offenbart - wie in **Fig. 2c** der gegenständlichen Anmeldung dargestellt - ein Fotometer mit einem stationären LED-Array mit den Lichtquellen L1 bis L5 und einem stationären Detektor-Array mit den Fotodioden R1 bis R5, wobei jeder Lichtquelle eine Fotodiode zugeordnet ist. Die sich auf einem Drehteller befindlichen Küvetten C sind zwischen dem LED-Array und dem Detektor-Array angeordnet. Bei einer rotatorischen Bewegung der Küvetten C in Pfeilrichtung werden die optischen Strahlengänge gekreuzt und die Proben in den Küvetten C können nacheinander mit dem Licht der unterschiedlichen Wellenlängen λ1 bis λ5 beaufschlagt werden.

Die AT 510 631 B1 (SCAN Messtechnik) beansprucht ein Spektrometer mit multiplen LEDs als Lichtquelle 44, wie in **Fig. 2d** der gegenständlichen Anmeldung dargestellt. Das Spektrometer dient zur Untersuchung der Inhaltsstoffe eines Fluides 42, mittels der Lichtquelle 44 und einem Detektor 45, wobei das Licht der Lichtquelle 44 mit einem vorgegebenen Spektralbereich durch ein Eintrittsfenster 47 durch das zu untersuchende Fluid 42 und durch ein Austrittsfenster 48 zum Detektor 45 geführt wird. Die Lichtquelle 44 wird durch mehrere in einer Halterung 50 angeordnete, mit einer Steuerelektronik 43 verbundene LEDs 49 gebildet, die zur Aussendung von Licht unterschiedlicher Wellenlängenbereiche innerhalb des vorgegebenen Spektralbereichs ausgebildet sind. Die Steuerelektronik 43 ist zur sequentiellen Ansteuerung der Leuchtdioden 49 ausgebildet, wobei in der Halterung 50 gegenüber den Leuchtdioden 49 ein mit der Steuerelektronik 43 verbundener Kompensationsdetektor 51 angeordnet ist. Im Strahlengang zwischen der Lichtquelle 44 und dem Eintrittsfenster 47 sind eine Linse 46, eine Blende 52 sowie eine Sammellinse 53 angeordnet. Zur Messung des Streulichts des zu untersuchenden Fluides kann quer zur Messstrahlung ein weiterer Detektor 54 angeordnet sein.

Die WO 2010/122203 A1 (Biosystems) offenbart ein auf einer Anordnung von mehreren LEDs als Lichtquelle basiertes Fotometer zur Messung der Absorption und der Trübung einer in einer Küvette vorliegenden Probe. Dabei wird das Licht der einzelnen LEDs mittels Strahlteiler samt Bandpassfilter in den Strahlengang vor der Probe eingekoppelt. Auf der Seite der Lichtquelle ist weiters eine Referenzphotodiode angeordnet. Im Strahlengang nach der Probe, auf der Detektionsseite ist eine Fotodiode angeordnet. Die einzelnen Küvetten werden am Fotometer vorbeigeführt. Nachteiliger Weise ist die Lichtquelle sehr komplex aufgebaut und besteht aus vielen Einzelkomponenten. Zudem muss das Licht der LEDs, die weiter von der Küvette entfernt sind, mehrere Strahlteiler passieren, was zu Intensitätsverlusten führt.

In der US 4,234,539 (Coulter Elelectronics) wird ein automatischer Analysator mit Drehscheiben für Proben-, Reagenzien- und Reaktionsgefäße (Küvetten) mit dazwischen eingebauten Pipettierarmen zum Transfer der Medien beschrieben. Konzentrisch zu einer Küvetten-Drehscheibe ist ein Rotor angeordnet, auf dem zueinander fix positionierte Paare von Lichtquellen und Fotodetektoren angeordnet sind. Bei entsprechender Positionierung bzw. Drehung kommen die einzelnen Küvetten zwischen Lichtquelle und Fotodetektor zu liegen. In einer alternativen Ausführungsform ist an der Rotationsachse zentral eine einzelne Lichtquelle positioniert und die Fotodetektoren befinden sich (in radialer Richtung gesehen) an der gegenüber liegenden Seite der Küvetten. Während sich nun die Küvetten-Drehscheibe nur langsam dreht, vollführt der Rotor mit der Lichtquelle eine viel raschere Drehbewegung, was zu einer deutlichen Erhöhung der Messfrequenz führt. Weiters kann der Rotor ein Filterrad mit unterschiedlichen Filtern aufweisen, die in den Strahlengang zwischen der zentralen Lichtquelle und der Küvette bringbar sind. Der Rotor muss jedoch bei jeder Küvette stoppen, wonach das jeweilige Filter durch Drehung des Filterrades ausgewählt wird. Es bestehen jedoch auch hier die bereits eingangs beschriebenen Nachteile von Drehtellersystemen bzw. von an Drehscheiben befestigten Küvetten.

Aus der EP 2 309 251 A1 (Siemens Healthcare) ist ein automatischer Analysator mit stationären, in kreisförmiger oder linearer Anordnung vorliegenden Probengefäßen bzw. Küvetten bekannt, wobei die optische Messeinheit auf einer drehbaren Einrichtung entlang der Probengefäße verfahrbar ausgeführt ist. Gemäß einer Ausführungsvariante kann die drehbare Einrichtung, die die Lichtquelle in Form einer LED und den Fotodetektor in Form einer Fotodiode trägt, unterhalb der Aufnahme der Probengefäße angeordnet sein, wodurch es jederzeit möglich ist, auf die Probengefäße mittels eines Greifarms zuzugreifen. Die drehbare Einrichtung kann auch mehrere LEDs unterschiedlicher Wellenlängen und mehrere Fotodioden aufweisen, damit die Proben bei mehreren Wellenlängen gemessen werden können. Die Fotodioden können durch ein CCD-Element ersetzt sein.

G) Systemkomponenten zum Mischen und Thermostatisieren für automatische Analysatoren

Aus der DE 27 26 498 A1 (HELLMA) ist eine temperierbare Küvettenanordnung bekannt geworden. Wie in **Fig. 2e** der gegenständlichen Anmeldung dargestellt, ist ein thermostatisierbarer Küvettenblock 55 mit mehreren Aufnahmeschächten 56 vorgesehen, in die Küvetten 57 eingesetzt werden können. Die nach unten konisch zulaufenden, seitliche Messfenster 58 aufweisenden Küvetten 57 sind formschlüssig in einen U-förmigen, gut wärmeleitenden Adapter 59 eingesetzt, der über die Wände 60 des Aufnahmeschachts 56 den thermischen Kontakt zum Küvettenblock 55 herstellt. Das Proben-Reagenziengemisch in jeder der Küvetten 57 kann jeweils durch einen Messkanal 61 im Küvettenblock 55 optisch vermessen werden.

Nachteilig dabei ist, dass sich die Temperatur des Proben-Reagenziengemisches nur langsam auf die Temperatur des Küvettenblocks aufheizt. Somit wird das Erreichen eines hohen Probendurchsatzes in einem Analysator erschwert, da die Thermostatisierung bei der Analyse einer Probe stets zu den Prozessen mit dem höchsten Zeitbedarf zählt.

Die JP 2007-303964 A (OLYMPUS) offenbart - wie in **Fig. 2f** der gegenständlichen Anmeldung dargestellt - eine Vorrichtung zur Thermostatisierung von Küvetten 62, die in Aufnahmen eines drehbaren Karussells 63 angeordnet sind. Die Vorrichtung weist ein an der Seitenwand jeder Küvette 62 befestigtes piezoelektrisches Substrat 64 auf, auf welchem sowohl eine Elektrodenstruktur eines Interdigitalwandlers (IDT) als Ultraschall-Wandler 65, als auch ein Temperatursensor 66 zur nichtinvasiven Messung der Temperatur des Küvetteninhalts integriert ist. Eine über Schleifkontakte 67 angeschlossene Temperatur-Regeleinheit 68 einer Steuereinheit 69 bildet zusammen mit der Treibereinheit 70 für den Ultraschall-Wandler 65 einen Regelkreis, um eine Reaktionsmischung in der Küvette 62 zu thermostatisieren. Dabei wird das Proben-Reagenzgemischs durch Absorption von Ultraschallenergie direkt bis zur Zieltemperatur erwärmt.

Nachteilig ist hierbei, dass jede Küvette 62 ein angeklebtes piezoelektrisches Substrat 64 mit integriertem Temperatursensor 66 benötigt, der mit einer elektronischen Regeleinheit 68 Kontakt gebracht werden muss. Weiters kann die auf dem Substrat des Ultraschall-Wandlers 65 gemessene Temperatur durch die Eigenerwärmung des Ultraschall-Wandlers verfälscht werden und entspricht somit nicht der Temperatur des Proben-Reagenzgemischs in der Küvette 62.

Weiters befindet sich der Temperatursensor 66 nicht in Kontakt mit der Flüssigkeit, sondern kann die Temperatur der Flüssigkeit nur indirekt über die Wärmeleitung der Gefäßwand der Küvette 62 aufnehmen, wodurch insbesondere bei einer sehr schnellen Erwärmung der Flüssigkeit ein Temperaturanstieg in der Flüssigkeit nicht mit ausreichender Schnelligkeit und Genauigkeit gemessen werden kann, um eine dauernde oder vorübergehende Überschreitung der Zieltemperatur um einen für die Probenbestandteile kritischen Wert ausschließen zu können.

Aus der EP 1 995 597 A1 (OLYMPUS) ist eine Vorrichtung zum Rühren von Flüssigkeiten in Küvetten 71 bekannt, die - wie in **Fig. 2g** der gegenständlichen Anmeldung dargestellt - auf einem drehbaren Karussell 72 angeordnet sind, wobei an der Seitenwand jeder Küvette ein Schallerzeuger 73 (Interdigitalwandler (IDT)) zur Einstrahlung von Ultraschallenergie in die Küvette 71 angeklebt ist. Gemäß EP 1 995 597 A1 müssen jedoch Maßnahmen getroffen werden, um eine durch Schallabsorption auftretende, unerwünschte Temperaturerhöhung des Küvetteninhalts zu begrenzen und eine Verfälschung der Analyseergebnisse durch thermische Schädigung zu verhindern.

Der durch den Betrieb des Schallerzeugers 73 bedingte kritische Wärmeeintrag wird durch in einer Steuereinheit 74 gespeicherte thermische Charakteristika des Küvetteninhalts errechnet. Der Wärmeeintrag kann durch Begrenzung der Betriebsdauer, der Amplitudenmodulation oder Variation der Betriebsfrequenz des Ultraschallgenerators auf einen nicht-schädlichen Wert begrenzt werden. Gemäß einer weiteren Maßnahme zur Begrenzung des Wärmeeintrags kann mittels eines Aktuators 75 für jede Küvette 71 ein eigenes Peltier-Element 76 direkt an das Substrat des angeklebten Schallerzeugers 73 angelegt werden, um dieses während des Betriebs aktiv zu kühlen. Die Steuerung der Leistung des Peltier-Elements 76 erfolgt über gespeicherte Betriebsparameter, wobei am Peltier-Element keine Temperaturmessung vorgesehen ist. Der Signalgenerator 77 für den Schallerzeuger 73 wird von einer Treibereinheit 78 der Steuereinheit 74 angesteuert.

Eine präzise Thermostatisierung der Flüssigkeiten in den Küvetten 71 durch entsprechende Parametrisierung alleine ist hierdurch nicht möglich oder vorgesehen, da ein vorberechneter Ultraschalleintrag zur Erreichung einer Zieltemperatur alleine zu ungenau wäre.

Um den Misch- bzw. Rührvorgang präziser zu regeln und zu gewährleisten, dass ein schädlicher Temperaturwert beim Rühren nicht überschritten wird, kann eine Temperaturmessung der Flüssigkeit von oben mit einem stationären InfrarotSensor durchgeführt werden, die aber nur an jeweils einer bestimmten Küvette des Karussells bei dessen Stillstand durchgeführt werden kann.

Eine Thermostatisierung mit den genannten technischen Merkmalen weist gegenüber einer Block-Thermostatisierung in einer Küvettenaufnahme konstanter Temperatur den Nachteil auf, dass das System im Hinblick auf eine Überschreitung der Zieltemperatur beim Aufheizen und Einregeln als nicht als inhärent sicher angesehen werden kann.

Die JP 2007-010345 A (OLYMPUS) beschreibt eine Ultraschall-Rührvorrichtung, mit welcher der Inhalt L einer Küvette 81 vermischt werden kann. Wie in **Fig. 2h** der gegenständlichen Anmeldung dargestellt, ist an Boden 82 der Küvette 81 ein piezokeramischer Ultraschall-Erzeuger (Dickenschwinger 83) angeklebt, wobei die Form und das Material des Küvettenbodens eine akustische Linse 84 bildet, um die Ultraschallenergie im Punkt F knapp unterhalb die Flüssigkeitsoberfläche zu bündeln. Der Dickenschwinger 83 aus Blei-Zirkonat-Titanat ("sounding body") weist eine plane Scheibe 85 mit beidseitig flächiger, elektrischer Kontaktierung 86 auf, mit einem Durchmesser der größer ist als jener des Küvettenbodens 82.

**H)** <u>Systemkomponenten zur Durchführung luminometrischer Messungen für automatische Analysatoren</u>

Aus der US 7,998,432 B2 ist eine automatischer Analysator zur Durchführung von biochemischen (klinisch-chemischen) Tests und Blut-Koagulationstests bekannt, die fotometrisch vermessen werden, wobei der Analysator auch zur Durchführung von heterogenen Immunoassays mithilfe einer Lumineszenz-Detektion geeignet ist. Die in **Fig. 1c** der gegenständlichen Anmeldung beschriebene Vorrichtung ist im Wesentlichen in einen Bereich 120 zur Lagerung von Proben und Reagenzien sowie einen Bereich 121 zur Durchführung von optischen Messungen und Analysen unterteilt. Eine Pipettiervorrichtung 122 kann entlang der beiden Bereiche 120 und 121 verfahren und somit flüssige Proben und Reagenzien vom Lagerbereich 120 in die Küvetten auf einem drehbaren Küvettenkarussell 123 pipettieren. Das Küvettenkarussell 123 wird von unten mittels einer ringförmigen Thermostatisiereinrichtung auf konstante Temperatur gebracht. Über jeweils vorhandene Transfermechanismen können einzelne Küvetten - bei Stillstand des Küvettenkarussell - in radialer Richtung zwischen den schlitzförmigen Küvettenaufnahmen des Karussells und den rund um das Küvettenkarussell 123 angeordneten stationären Stationen des Analysators ausgetauscht werden. Vorgesehen ist eine Station 124 zur fotometrischen Messung, eine Station 125 zur Abgabe zu entsorgender Küvetten, eine Station 126 mit einem Dispensor zur Abgabe von beschichteten, magnetischen Nanopartikel aus einem Lagerbereich 127, in dem sich auch Waschreagenzien und Triggerreagenzien für die Lumineszenzmessung befinden. Weitere Stationen dienen zur magnetischen Sedimentation und B/F-Waschen 128, Lumineszenzmessung 129, Koagulationsmessung oder Verdünnung von Proben. Mit 130 ist ein Magazin zur Bereitstellung von Einwegküvetten bezeichnet. Nachteilig ist der große mechanische Aufwand, der mit dem Transfer der Küvetten zwischen den Aufnahmen des Küvettenkarussells 123 und den einzelnen Stationen des Analysators verbunden ist. Obwohl in einzelnen Stationen (siehe 128, 129) - durch die Auslagerung der Küvetten - Mess- und Vorbereitungsschritte ablaufen, die von der Taktrate des Küvettenkarussells 123 entkoppelt sind, ist der Küvettentransfer in bzw. aus diesen Positionen nach wie vor von dieser Taktrate abhängig, ebenso wie jene Handlungen, bei welchen die Küvetten im Karussell verbleiben (fotometrische Messung in der Station 124 sowie Zugabe der magnetischen Beads in der Station 126). Es gelten somit die bereits in Punkt A) im Zusammenhang mit Karussell-Anordnungen dargelegten Nachteile.

Aus der US 6,333,008 B1 ist eine Messanordnung bekannt, die zur Durchführung luminometrischer Reihenanalysen an Flüssigproben dient, in denen nachzuweisende Zielsubstanzen und mit diesen in einer immunchemischen Nachweisreaktion verbindbare Markiersubstanzen ("labeling substances") sowie magnetisierbare Trägerpartikel enthalten sind. Die Flüssigproben werden in Vertiefungen einer Mehrfachküvette entlang einer Förderstrecke zu einer optischen Messstation transportiert, wobei während des Transports als drehbare Doppelmagnete ausgebildete Dauermagnete und Trennstationen, die zur Abtrennung überschüssiger Markiersubstanz bestimmt sind, auf die Mehrfachküvette einwirken. In den einzelnen Trennstationen findet mit Hilfe eines Injektors und einer Saugnadel jeweils ein (B/F) - Waschschritt statt. In der Messstation wird die Lumineszenzstrahlung durch einen Fotodetektor erfasst. Nachteilig bei der bekannten Messanordnung ist die Notwendigkeit, die Flüssigproben während des Analyseprozesses zu verschiedenen, an einem Prozesspfad ortsfest verteilten Automatenkomponenten befördern zu müssen. Weiters müssen bestimmte Komponenten, wie als drehbare Doppelmagnete ausgebildete Dauermagneten und Trennstationen mit Injektoren und Saugnadeln mehrfach ausgebildet werden.

Derartige Vorrichtungen zeichnen sich dadurch aus, dass alle Prozesse durch starre Taktzyklen des Küvetten-Fördermechanismus vorgegeben sind und in vorbestimmten Zeitfenstern ablaufen müssen. Aktionen wie Dispensieren, Mischen, Separieren und Messen können nur dann erfolgen, wenn die jeweiligen Küvetten sich an den Positionen der jeweiligen Gerätekomponenten befinden.

So kann eine Probe (nicht jederzeit, sondern) nur dann in eine leere Küvette dispensiert werden, wenn die leere Küvette an der Position des Probenpipettors vorbeifährt und der Küvetten-Beförderungsmechanismus an dieser Position stoppt. Ein Reagenz oder eine Waschflüssigkeit kann nur dann in eine die Probe enthaltende Küvette dispensiert werden, wenn die betreffende Küvette an der Position des Reagenziendispensors vorbeifährt und der Küvetten-Fördermechanismus an dieser Position stoppt. Analoges gilt für das Rühren von Reaktionsgemischen aus der Probe und den Reagenzien in den Küvetten mit mechanischem Rühren und für die optische Messung an der Position der optischen Messeinrichtung.

So kann beispielsweise eine bestimmte Küvette auch nicht jederzeit oder nicht wiederholt in kleinen Zeitintervallen optisch gemessen werden, da erst abgewartet werden muss, bis sich die betreffende Küvette an der Position der optischen Messeinheit befindet.

Aufgabe der Erfindung ist es, bei automatischen Analysatoren zur Durchführung von chemischen, biochemischen und/oder immunchemischen Analysen von flüssigen Proben, die oben - vor allem im Zusammenhang mit dem durch starre Taktzyklen vorgegeben und in vorbestimmten Zeitfenstern ablaufen Prozessen eingeschränkten Probendurchsatz bekannter Systeme - genannten Nachteile zu vermeiden und Verbesserungen vorzuschlagen, die den Probendurchsatz erhöhen, ohne die Einzelanalyse oder den Analysator wesentlich zu verteuern, wobei die Qualität der Analyse zumindest beibehalten werden soll. Weiterhin soll ein Verbessertes Verfahren zur automatischen chemischen, biochemischen und/oder immunchemischen Analyse von flüssigen Proben vorgeschlagen werden.

Diese Aufgabe wird erfindungsgemäß durch einen Analysator mit Küvetten zur Aufnahme der flüssigen Proben und Reagenzien gelöst, wobei eine Vielzahl von Küvetten als zumindest ein stationäres, lineares Küvettenarray im Analysator angeordnet ist, mit verfahrbaren und stationären Automatenkomponenten, zumindest umfassend:

- einen entlang einer durch das lineare Küvettenarray definierten Bewegungslinie in x-Richtung verfahrbar ausgeführten Pipettor, der mit zumindest einer Pipettiernadel ausgestattet ist, die in z-Richtung in die Küvetten absenkbar ausgeführt ist und in einer auf die x-Richtung im Wesentlichen normal stehenden y-Richtung zwischen den Küvetten und dem Probenlager und/oder dem Reagenzienlager verfahrbar ausgeführt ist,

- eine Mischereinheit zur Vermischung der Proben und Reagenzien in den Küvetten,

- eine optische Messeinheit, welche - zur Gewinnung eines Messsignals - durch ein seitlich an der Küvette angeordnetes Messfenster austretende Messstrahlung empfängt,

- eine in x-Richtung verfahrbar ausgeführte Küvettenwascheinheit zur Reinigung der Küvetten,

- eine Nadelwascheinheit zur Reinigung der zumindest einen Pipettiernadel, sowie

- eine stationäre Thermostatisiereinheit zur Einstellung einer vorgebbaren Messtemperatur in den Küvetten,

wobei zumindest zwei Automatenkomponenten unabhängig voneinander entlang oder parallel zu der durch das lineare Küvettenarray definierten Bewegungslinie in x-Richtung verfahrbar ausgeführt sind und jeweils Zugriff auf unterschiedliche Küvetten oder Gruppen von Küvetten in frei wählbarer Reihenfolge aufweisen.

Das erfindungsgemäße Verfahren zur automatischen chemischen, biochemischen und/oder immunchemischen Analyse von flüssigen Proben, die in einem Probenlager eines Analysators vorliegen, unter Zuhilfenahme von flüssigen Reagenzien, die in zumindest einem Reagenzienlager des Analysators vorliegen, zur Ermittlung zumindest einer Analytkonzentration in der Probe, zeichnet sich durch folgende Schritte aus:

- Transferieren einer vorbestimmten Menge einer flüssigen Probe von einem Probengefäß im Probenlager in eine Küvette eines stationären, linearen Küvettenarrays mittels eines entlang des Küvettenarrays verfahrbaren, ersten Pipettors;

- Transferieren einer vorbestimmten Menge einer Reagenzflüssigkeit von einem Reagenziengefäß des Reagenzienlagers in die Küvette des stationären, linearen Küvettenarrays mittels des ersten Pipettors oder mittels eines zweiten, unabhängig vom ersten verfahrbaren Pipettors;

- Vermischen und Thermostatisieren der Flüssigkeiten in der Küvette;

- gegebenenfalls Transferieren einer vorbestimmten Menge einer weiteren Reagenzflüssigkeit von einem Reagenziengefäß des Reagenzienlagers in die Küvette des stationären, linearen Küvettenarrays mittels des ersten oder des zweiten Pipettors;

- gegebenenfalls nochmaliges Vermischen und Thermostatisieren der Flüssigkeiten in der Küvette;

- optische Vermessung des Inhalts der Küvette mittels einer optischen Messeinheit und Ermittlung zumindest eines Messwertes;

- Berechnen und Anzeigen der Analytkonzentration basierend auf den ermittelten Messwerten und vorbekannten oder vorbestimmten Referenz- und Kalibrierwerten;

- Waschen und Trocknen der Küvette mittels einer entlang des Küvettenarrays verfahrbaren Küvettenwascheinheit; sowie

- Bereitstellen der Küvette für eine nachfolgende Analyse.

Erfindungsgemäß sind somit zwingend zwei Automatenkomponenten unabhängig voneinander in x-Richtung verfahrbar ausgeführt: der Pipettor (im einfachsten Fall ein einziger Pipettor mit einer einzigen Pipettiernadel) und die Küvettenwascheinheit. Die Mischereinheit und die optische Messeinheit können stationär oder verfahrbar sein, die Thermostatisiereinheit ist zwingend stationär ausgeführt. Es ist noch anzumerken, dass zwei verschiedene, verfahrbare Automatenkomponenten, die auf die Küvettenöffnungen zugreifen, nicht gleichzeitig auf ein und dieselbe Küvette zugreifen können. In der Praxis ist es aber ohnehin nicht erforderlich, dass beispielsweise Pipettor und Küvettenwascheinheit "gleichzeitig" auf ein und dieselbe Küvette zugreifen. Weiters ist anzumerken, dass stationär ausgebildete Automatenkomponenten so ausgeführt sind, dass diese ohnehin auf jede Küvette zugreifen, beispielsweise dadurch, dass jeder Küvette oder Gruppe von Küvetten eine derartige Automatenkomponente zugeordnet ist.

Durch den wahlfreien Zugriff der in x-Richtung verfahrbaren Automatenkomponenten, insbesondere der Küvettenwascheinheit auf beliebige Küvetten und des zumindest einen Pipettors (mit zumindest einer Pipettiernadel) auf beliebige Probengefäße, Reagenziengefäße und Küvetten, erhöht sich der Durchsatz im Vergleich zu einem rotatorisch organisierten Automaten mit gleicher Anzahl an Küvette erheblich.

Gemäß einer vorteilhaften Ausführungsvariante der Erfindung weist der Analysator zwei unabhängig voneinander in x-Richtung verfahrbare Pipettoren auf.

Gegenüber der Variante mit einem Pipettor ergibt sich eine weitere Durchsatzsteigerung dadurch, dass der erste Pipettor Proben in eine erste Küvette pipettieren kann, während der zweite Pipettor gleichzeitig Reagenzien in eine beliebig wählbare, zweite Küvette pipettieren kann.

Erfindungsgemäß ist weiterhin vorgesehen, dass zumindest ein Pipettor zwei unabhängig voneinander, parallel zueinander in y-Richtung verfahrbare Pipettiernadeln aufweist. Die beiden Pipettiernadeln eines Pipettors können somit unabhängig voneinander entlang derselben Wegstrecke in y-Richtung aneinander vorbeifahren, ohne zu kollidieren.

Gemäß dieser vorteilhaften Variante können auch zwei unterschiedliche Nadeltypen verwendet werden (z.B. für unterschiedliche Pipettiervolumina, mit speziellen Beschichtungen für unterschiedliche Proben- und Reagenzienarten, ohne dass man einen weiteren Pipettor oder eine Nadelaustauschstation benötigt).

Eine besonders vorteilhafte Variante der Erfindung sieht vor, dass die Nadelwascheinheit am Pipettor angeordnet und mit diesem verfahrbar ausgeführt ist.

Weiter durchsatzsteigernd ist die Maßnahme, dass eine Pipettiernadel pipettieren kann, während zeitgleich die zweite gereinigt wird. Vorteile ergeben sich auch bei nur einer Pipettiernadel am Pipettor, da der Pipettor nicht jedes Mal eine stationäre Nadelwascheinheit anfahren muss. Da die y-Bewegung der jeweiligen Pipettiernadel unabhängig von der am Pipettor mitgeführten Nadelwascheinheit erfolgen kann, ist eine Aufteilung der bewegten Massen der Robotikkomponenten auf die beiden horizontalen Achsen möglich, sodass nur in x-Richtung eine Mitbeschleunigung der Nadelwascheinheit erfolgen muss.

Eine weitere Aufgabe der Erfindung besteht darin, eine optische Messeinheit sowie ein optisches Messverfahren zur Gewinnung von Messsignalen von flüssigen Medien, die in aneinander gereihten Küvetten aufgenommen sind, dahingehend zu verbessern, dass im Verlauf der chemischen Reaktionen in den einzelnen Küvetten und in kurzer zeitlicher Abfolge eine Vielzahl von Messungen bei unterschiedlichen Wellenlängen durchgeführt werden kann, wobei der kinematische Aufwand, bedingt durch translatorische und/oder rotatorische Relativbewegungen zwischen einzelnen Komponenten des Messsystems, möglichst reduziert werden soll.

Diese weitere Aufgabe wird erfindungsgemäß dadurch gelöst, dass die optische Messeinheit mit einer Lichtbereitstellungseinheit ausgestattet ist, die mehrere im UV/VIS/NIR-Wellenlängenbereich spektral unterschiedlich emittierende LED-Lichtquellen aufweist, sowie mit einer stationären Detektionseinheit, die so ausgelegt ist, dass jeder Küvette des Küvettenarrays zumindest eine Fotodiode fix zugeordnet ist.

Von Vorteil ist insbesondere, dass die Küvetten als unbewegliches, stationäres Küvettenarray angeordnet sind, wobei jeder Küvette ihre individuellen Detektoren (Durchlichtdetektor (für fotometrische und turbidimetrische Messungen) und/oder Streulichtdetektor (für nephelometrische Messungen)) fix zugeordnet sind und dass das aus den einzelnen Küvetten austretende Licht - also auch allfällige Dunkelsignale und ggf. einfallendes Umgebungslicht - einer jeden Küvette zeitlich unbegrenzt zwecks Korrektur gemessen werden kann. Somit ist es nicht erforderlich im Vorbeifahren der Detektoren zu messen oder einen Detektor in sequenzieller Abfolge vor mehreren Küvetten im Stop-and-Go-Betrieb zu positionieren. Dadurch können präzisere Messergebnisse in sehr kurzen Zeitabständen erhalten, und Messabläufe wesentlich flexibler gestaltet werden.

Gemäß einer ersten Variante der Erfindung weist die Lichtbereitstellungseinheit zumindest eine stationäre Lichtverteilereinrichtung auf, die das Licht der einzelnen LED-Lichtquellen auf die einzelnen Küvetten des Küvettenarrays verteilt, wobei die Lichtverteilereinrichtung einen Hohlraum aufweist, dessen innere Flächen zumindest teilweise verspiegelt und/oder diffus reflektierend ausgeführt sind, und wobei die Lichtverteilereinrichtung für jede LED-Lichtquelle eine Eintrittsöffnung zur Einspeisung des Lichts in den Hohlraum aufweist und wobei die Lichtverteilereinrichtung für jede Küvette des Küvettenarrays eine Austrittsöffnung zur Einspeisung des Lichts in die Küvette aufweist.

Dabei handelt es sich um eine kompakte, kostengünstige Variante, da die Lichtverteilereinrichtung, die mehrere LED-Lichtquellen unterschiedlicher Wellenlänge aufnimmt, stationär einer Reihe von Küvetten zugeordnet ist. Bei Küvettenarrays mit einer großen Anzahl von Küvetten kann das stationäre Küvettenarray segmentiert sein, wobei jedem Segment eine separate Lichtverteilereinrichtung fix zugeordnet ist. Insgesamt wird somit eine optische Messeinheit realisiert, die keine beweglichen Komponenten aufweist.

Zur besseren Verteilung des von den einzelnen LED-Lichtquellen unterschiedlicher Wellenläge in die Lichtverteilereinrichtung eingestrahlten Lichts ist die den Eintrittsöffnungen der LED-Lichtquellen gegenüberliegende, innere Fläche der Lichtverteilereinrichtung bevorzugt gewellt und reflektierend ausgeführt. Obwohl sich unterschiedliche Lichtwege zwischen einzelnen LED-Lichtquellen und Küvetten ergeben, können - bedingt durch die konstanten geometrischen Verhältnisse - Intensitätsunterschiede rechnerisch, durch Parametrierung des Hardware-Setups und/oder durch Kalibriermessungen ausgeglichen werden.

Zur Homogenisierung der in die Küvetten gelangenden Messstrahlung ist die den Austrittsöffnungen zu den Küvetten gegenüberliegende, innere Fläche der Lichtverteilereinrichtung diffus reflektierend ausgeführt.

Gemäß einer zweiten Variante der Erfindung weist die Lichtbereitstellungseinheit zumindest ein eindimensionales, stabförmiges Lichtquellenarray mit mehreren LED-Lichtquellen auf, das entlang des stationären Küvettenarrays ausgerichtet ist und entlang des stationären Küvettenarrays verfahrbar ist, derart, dass jeder Küvette des stationären Küvettenarrays jede LED-Lichtquelle des Lichtquellenarrays zuordenbar ist.

Diese Variante profitiert davon, dass detektorseitig die den einzelnen Küvetten des stationären Küvettenarrays fix zugeordneten Fotodioden als stationäres, lineares Fotodiodenarray vorliegen und vorzugsweise auf einer gemeinsamen Platine angeordnet sind. Der geringfügige Nachteil, eines entlang des stationären Küvettenarrays verfahrbaren, stabförmigen Lichtquellenarrays, wird durch eine kostengünstige Herstellung (nur ein Lichtquellenarray für eine Vielzahl von Küvetten) aufgewogen.

Gemäß einer dritten Variante der Erfindung sind die LED-Lichtquellen der Lichtbereitstellungseinheit als 2D-LED-Array angeordnet, wobei jeder Küvette des stationären Küvettenarrays ein stationäres 2D-LED-Array fix zugeordnet ist.

Diese Variante genießt die Vorteile der oben beschriebenen ersten Variante, da die optische Messeinheit ohne bewegliche Komponenten realisierbar ist und jede Küvette über ein individuelles Fotometer, aufweisend ein fix zugeordnetes 2D-LED-Array als Lichtquelle und eine fix zugeordnete Fotodiode als Detektor, verfügt.

Ein erfindungsgemäßes optisches Messverfahren zur Gewinnung von Messsignalen von flüssigen Medien, insbesondere im Zusammenhang mit der ersten Variante der Erfindung, zeichnet sich durch folgende Schritte aus:

- Aufnahme der flüssigen Medien in aneinander gereihte Küvetten, die ein stationäres Küvettenarray bilden,

- Bereitstellen einer in die Küvetten einstrahlenden Eintrittsstrahlung mit Hilfe zumindest einer stationären Lichtverteilereinrichtung, die zumindest ein Segment des Küvettenarrays optisch kontaktiert,

- wobei in zeitlicher Abfolge nacheinander durch mehrere im UV/VIS/NIR-Wellenlängenbereich spektral unterschiedlich emittierende LED-Lichtquellen Licht in die Lichtverteilereinrichtung eingestrahlt und auf die einzelnen Küvetten verteilt wird, und

- Detektieren der aus den Küvetten austretenden Messstrahlung mit Hilfe von zumindest einer - jeder Küvette fix zugeordneten - Fotodiode einer stationären Detektionseinheit.

Die aus den Küvetten austretende Messstrahlung wird in ein elektrisches Messsignal umgewandelt und nach entsprechender Aufbereitung in einer Anzeigeeinheit dargestellt.

Der Analysator kann auch eine optische Messeinheit aufweisen, die als eine entlang des linearen, stationären Küvettenarrays verfahrbare Einheit, beispielsweise als Spektrometereinheit, ausgebildet ist.

Eine weitere Aufgabe der Erfindung besteht darin, Verfahren und Vorrichtungen zum Mischen und/oder Thermostatisieren flüssiger Medien, die in aneinander gereihten Küvetten eines Küvettenarrays eingebracht werden, derart zu verbessern, dass die Zeitdauer vom Einbringen der flüssigen Medien in die Küvette bis zum Erreichen einer vorgegebenen Zieltemperatur verkürzt wird, ohne dass Gefahr besteht, das Proben-Reagenziengemisch thermisch zu schädigen. Weiters soll das Proben-Reagenziengemisch beim Erreichen der Zieltemperatur optimal vermischt sein.

Diese Aufgabe wird einerseits dadurch gelöst, dass die Thermostatisiereinheit einen auf eine vorgegebene Zieltemperatur geregelten Küvettenblock aufweist, der mit einer Thermostatisiereinrichtung ausgestattet ist und mit den einzelnen Küvetten in thermischem Kontakt steht, sowie andererseits dadurch, den Küvetten zur Vermischung der Proben und Reagenzien stationäre Mischereinheiten zugeordnet sind, wobei an jeder Küvette als stationäre Mischereinheit zumindest ein Ultraschall-Wandler zum Einbringen von Ultraschallenergie in die Küvetten befestigt ist, sowie dass der Ultraschall-Wandler als piezoelektrischer Schwinger ausgeführt ist und mit einer Steuereinheit in Verbindung steht, die den zumindest einen Ultraschall-Wandler in Abhängigkeit von Parameterwerten der flüssigen Medien ansteuert.

Das erfindungsgemäße Verfahren zum Mischen und Thermostatisieren flüssiger Medien, die in aneinander gereihten Küvetten eines Küvettenarrays eingebracht werden, wobei die Küvetten des Küvettenarrays in einem thermostatisierbaren Küvettenblock angeordnet sind, zeichnet sich durch folgende Schritte aus:

a) Erwärmen der Küvetten auf eine vorgegebene Zieltemperatur mit Hilfe des thermostatisierbaren Küvettenblocks,

b) Erwärmen der flüssigen Medien mit Hilfe des thermostatisierten Küvettenblocks, um die vorgegebene Zieltemperatur zu erreichen,

c) in der Erwärmungsphase gemäß Punkt b), vor dem Erreichen der Zieltemperatur, zusätzliche Einbringung einer vorbestimmten Menge an Ultraschallenergie mit Hilfe zumindest eines an jeder Küvette befestigten Ultraschall-Wandlers zur Erhöhung der Erwärmungsrate, sowie

d) gleichzeitiges Mischen der flüssigen Medien mit Hilfe der in Punkt c) eingebrachten Ultraschallenergie.

Insbesondere ist erfindungsgemäß vorgesehen, dass die in Punkt c) eingebrachte Menge an Ultraschallenergie in Abhängigkeit von vorbestimmten Parameterwerten, wie beispielsweise Art, Menge, Viskosität, thermische Leitfähigkeit und Temperatur, der zugegebenen flüssigen Medien ermittelt wird.

Die einzubringende Menge an Ultraschallenergie kann beispielsweise in einem Test- oder Kalibrierschritt werkseitig durch Versuchsmessungen und/oder Berechnungen ermittelt werden, wobei dann eine entsprechende Information anwenderseitig zu Verfügung gestellt wird.

Nach Fertigstellung der Kalibrierung für alle vorgesehenen Analytbestimmungen sind anwenderseitig, im laufenden Betrieb der Vorrichtung zum Mischen und Thermostatisieren flüssiger Medien, keine Maßnahmen erforderlich, die benötigte Menge an Ultraschall-Energie für die jeweilige Analytbestimmung zu ermitteln, da auf die entsprechenden Werte aus der Test- und Kalibrierphase zugegriffen werden kann.

Mit dem erfindungsgemäßen Verfahren werden allfällige, beim schnellen Aufheizen auftretende, lokale Hotspots effektiv unterbunden, da das Einbringen von Ultraschallenergie durch Steuercodes geregelt wird, die beispielsweise in einem Analyseprotokoll abgelegt sind und in Abhängigkeit von Parameterwerten der Flüssigkeit ermittelt wurden, derart, dass die Flüssigkeit in der Küvette erhitzt und stets gleichzeitig umgewälzt wird.

Ein wesentlicher Vorteil der Erfindung besteht somit darin, dass durch die Parametrisierung der eingebrachten Menge an Ultraschallenergie die Temperatur des Küvetteninhalts niemals größer werden kann als jene des auf eine für die Probe verträgliche Endtemperatur vorthermostatisierten Küvettenblocks. Hierdurch kann eine thermische Schädigung biologischer Proben und Reagenzien durch Hotspots oder eine kurzfristige Überschreitungen der Zieltemperatur weitgehend ausgeschlossen werden.

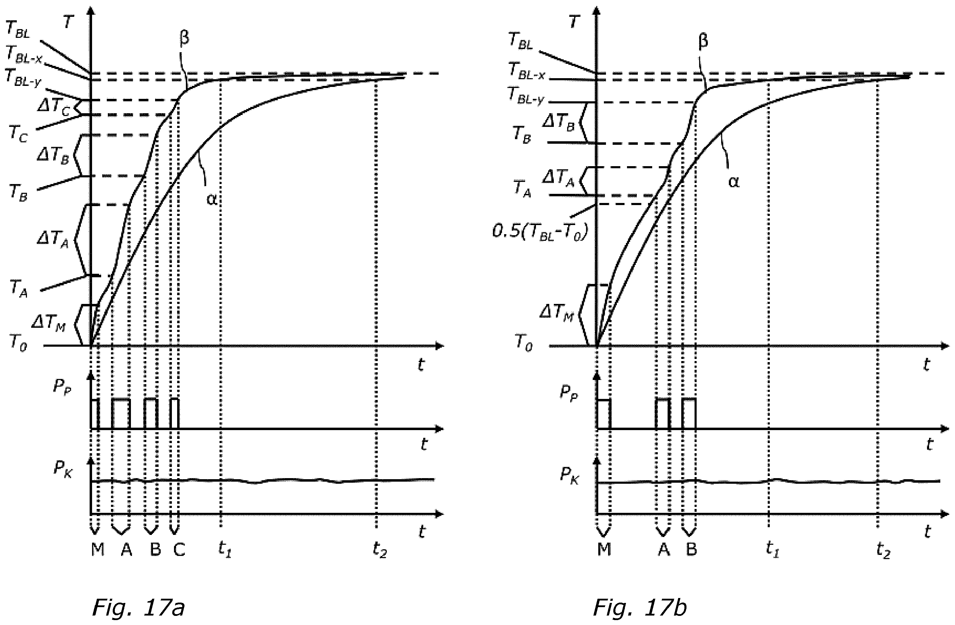

Technisch besonders einfach und zuverlässig ist die Temperierung aneinandergereihter Küvetten mithilfe eines Küvettenblocks aus einem zusammenhängenden, wärmeleitfähigen Material, wie beispielsweise einem Block aus eloxiertem Aluminium. Typisch für die Erwärmung des Küvetteninhalts aus einer vorthermostatisierten Wärmequelle ist eine asymptotische Annäherung an die Blocktemperatur $T_{BL}$, sodass die Erwärmung zunächst rasch, und dann immer langsamer erfolgt. Da die Blocktemperatur $T_{BL}$ nie ganz erreicht wird, wird bei einer Blockthermostatisierung eine etwas darunterliegende Temperatur von $T_{BL-x}$ als Zieltemperatur akzeptiert, die bei der Thermostatisierung von biologischen Proben im Rahmen einer optischen Messung bestimmter Analyte typischerweise im Bereich 0.1 - 0.5 °C unter der Blocktemperatur liegt und sich während der Analyse nicht um mehr als 0.1 °C ändern darf (siehe Fig. 17a, 17b).

Erfindungsgemäß kann die Ultraschall-Energie gemäß Punkt c) in mehreren Teilmengen (Boosts) gepulst in die flüssigen Medien eingebracht werden.

Weiterhin ist es von Vorteil, wenn zumindest eine Teilmenge der in Punkt c) eingebrachten Ultraschall-Energie im Hinblick auf die Pulsdauer, die Frequenz und die Amplitude zum Vermischen der flüssigen Medien in der Küvette optimiert wird.

Hierbei kann eine für ein kombiniertes Mischen (durch Erzeugung einer Konvektion in der Flüssigkeit) und Erhitzen (durch die Absorption von Ultraschall in der Flüssigkeit) günstige Signalform gewählt werden, ausgehend von einer Grundfrequenz des Ultraschall-Wandlers, welche durch eine aufgeprägte, im Vergleich niedrigere Frequenz (Frequenz-"Sweep") moduliert sein kann. Weiters kann auch die Amplitude der Grundfrequenz des Ultraschall-Wandlers durch eine aufgeprägte, im Vergleich niedrigere Frequenz moduliert sein, wobei die Amplitude zwischen einer Vollaussteuerung (100%) des Signals, bis zur Abschaltung des Signals (0%) variiert werden kann. Eine Amplitudenmodulation mit dem Amplitudenverhältnis (100:0) würde hierbei einem Burst-Muster entsprechen. Es können in beiden Fällen Modulationssignalformen wie Sinus, Rechteck, Sägezahn oder Ähnliches zum Einsatz kommen.

Besonders gute Ergebnisse im Hinblick auf das Vermischen der in die Küvette eingebrachten flüssigen Medien können erzielt werden, wenn der Ultraschall-Wandler mit einer Grundfrequenz von 200 kHz bis 200 MHz, beispielsweise bei Verwendung eines Dickenschwingers mit ca. 0,5 MHz bis 10 MHz, und bei Verwendung eines Interdigital-Wandlers mit ca. 50 MHz bis 150 MHz, betrieben wird.

Bevorzugt wird der Grundfrequenz des Ultraschall-Wandlers eine Modulationsfrequenz der Amplitude von 1 bis 100 Hz aufgeprägt.

Zur Vermischung und Erhitzung von wässrigen Reagenzien- und Probenflüssigkeiten bei der Durchführung von Analysen in entsprechenden Küvetten hängt die Grundfrequenz vorteilhaft einsetzbarer Ultraschall-Wandler von der Art des verwendeten Ultraschall-Wandlers ab. Werden angeklebte Dickenschwinger aus Piezokeramik verwendet, liegen Grundfrequenzen geeigneter Bauformen (je nach Größe und Dimension des Substrats) zwischen etwa 200 kHz und 10 MHz, vorzugsweise bei etwa 0,5 bis 10 MHz. Werden angeklebte Interdigital-Wandler verwendet, liegen Grundfrequenzen geeigneter Bauformen (je nach Größe und Dimension des Wandlers, sowie des Substrats) bei etwa 10 bis 200 MHz, vorzugsweise bei etwa 50-150 MHz.

Der Analysator kann auch eine Mischereinheit aufweisen, beispielsweise eine in Rotation oder Vibration versetzbare Pipettiernadel, die zur Vermischung der Proben und Reagenzien in die jeweiligen Küvetten abgesenkt werden kann.

Der Analysator weist eine Küvettenwascheinheit auf, die erfindungsgemäß als verfahrbare Automatenkomponente ausgeführt ist, die in jeder Waschposition auf eine Küvette oder eine Gruppe von Küvetten, vorzugsweise auf zwei bis fünf nebeneinander angeordnete Küvetten, gleichzeitig Zugriff hat.

Erfindungsgemäß weist der Analysator gemäß einer Variante eine Thermostatisiereinheit zur Einstellung einer vorgebbaren Messtemperatur auf, die Heizfolien umfasst, die einzelne Küvetten oder Gruppen von Küvetten thermisch kontaktieren und mit unterschiedlichen Temperaturniveaus beaufschlagbar sind.

Eine weitere Aufgabe der Erfindung besteht darin, einen Analysator vorzuschlagen, mit welchem ausgehend vom dargelegten Stand der Technik heterogene Immunoassays durchgeführt werden können, wobei Nachteile, vor allem im Zusammenhang mit dem - durch starre Taktzyklen vorgegeben und in vorbestimmten Zeitfenstern ablaufenden Prozessen - eingeschränkten Probendurchsatz bekannter Systeme vermieden werden und Verbesserungen erzielt werden, die den Probendurchsatz erhöhen, ohne die Einzelanalyse oder den Analysator wesentlich zu verteuern, wobei die Qualität der Analyse zumindest beibehalten werden soll.

Diese Aufgabe wird erfindungsgemäß dadurch gelöst, dass der Analysator eine Vorrichtung zur Durchführung von heterogenen Immunoassays aufweist, die Zugriff auf die Küvetten zumindest eines endständigen Segments des stationären, linearen Küvettenarrays hat.

Erfindungsgemäß weist die Vorrichtung zur Durchführung von heterogenen Immunoassays folgende Komponenten auf:

- zumindest einen, entlang des Küvettenarrays verfahrbaren und in Richtung der Füllöffnung einer ausgewählten Küvette absenkbaren Haltearm mit zumindest einer, in Richtung Boden der Küvette absenkbaren Absaugnadel, sowie mit zumindest einem, über oder in der jeweiligen Füllöffnung positionierbaren Dispensor zur Abgabe der flüssigen Medien in die Küvette, wobei zumindest ein Dispensor zur Abgabe einer Waschlösung für die magnetischen Partikel ausgebildet ist,

- zumindest eine, entlang des Küvettenarrays verfahrbare, auf den Inhalt der ausgewählten Küvette wirkende Magnetanordnung zur Separation der magnetischen Partikel an einer Innenfläche der Küvette, sowie

- zumindest eine, entlang des Küvettenarrays verfahrbare, und auf das Messfenster der ausgewählten Küvette ausrichtbare, optische Detektionseinrichtung zur Aufnahme eines einer Analytkonzentration in der ausgewählten Küvette proportionalen Messsignals.

Gemäß einer bevorzugten Ausführungsvariante der Erfindung weist der Haltearm für die Absaugnadel und den zumindest einen Dispensor eine Hub- und Dreheinrichtung auf, die auf einer entlang des Küvettenarrays verfahrbaren Plattform angeordnet ist, wobei auf der verfahrbaren Plattform eine gemeinsame Aufhängung für die Magnetanordnung und die Detektionseinrichtung angeordnet sein kann.

Besonders vorteilhaft ist es, wenn der auf der verfahrbaren Plattform angeordnete Haltearm samt Dispensorplattform zusammen mit der Magnetanordnung und der Detektionseinrichtung ein entlang des Küvettenarrays verfahrbares Mess- und Manipulationsmodul bildet, das sämtliche robotische, fluidische und messtechnische Komponenten für die Prozessschritte der magnetischen Separation der Beads, des sogenannten B/F - Waschens, sowie der Auslösung (Triggerung) und Messung der Lumineszenz, vereint.

Ein erfindungsgemäßes Verfahren zur Bestimmung eines Antigens mittels eines heterogenen Immunoassays ist dadurch gekennzeichnet, dass zunächst in einer ersten Schrittfolge A

eine Probe zur Bestimmung des Antigens,

eine Suspension magnetischer Partikel mit einem Fängerantikörper, sowie ggf. ein Tracer-Antikörpers oder ein markiertes Antigen

in eine ausgewählte Küvette eines stationären Küvettenarrays einpipettiert werden, sowie dass die folgenden Schritte B einer immunchemischen Analyse, wie

a) Separieren der magnetischen Partikel,

b) ein oder mehrmaliges Einbringen und Absaugen einer Waschlösung,

c) Zudosieren zumindest einer Trigger-Flüssigkeit, sowie

d) luminometrische Vermessung der Probe,

mit Hilfe eines entlang des Küvettenarrays verfahrbaren Mess- und Manipulationsmoduls erfolgen, das zur Durchführung einzelner oder aller Schritte a) bis d) bei der ausgewählten Küvette angehalten wird.

Ein besonderer Vorteil der Erfindung besteht darin, dass das Mess- und Manipulationsmodul während des Ablaufs zeitaufwändiger Schritte bei der immunchemischen Analyse, wie Inkubation, etc., in der ausgewählten Küvette, zu zumindest einer weiteren Küvette des Küvettenarrays verfahren werden kann, um in der weiteren Küvette einzelne oder alle Schritte B einer immunchemischen Analyse durchzuführen.

Insbesondere kann das erfindungsgemäße Mess- und Manipulationsmodul frei zwischen den Küvetten des stationären Küvettenarrays verfahren, um während eines nicht mit den Komponenten des Mess- und Manipulationsmoduls durchzuführenden Assay-Prozessschrittes in einer ersten Küvette, einen zweiten Prozessschritt in einer anderen Küvette durchzuführen.

Vor oder während der Anfahrt des Mess- und Manipulationsmoduls zu einer Küvette kann die Nadelgruppe der Dispensoren sowie die Absaugnadel in einer auf dem Mess- und Manipulationsmodul angeordneten Waschstation gewaschen werden.

So kann beispielsweise in einem Parallelisierungsbeispiel während eines Inkubationsschritts eines Assays in einer ersten Küvette eine magnetische Separation sowie B/F-Waschen in einer zweiten Küvette durchgeführt werden, um die Auslastung der Automatenkomponenten zu erhöhen, und Zeit bei der Abarbeitung der Assays zu sparen.

Erfindungsgemäß weisen die Küvetten, die im klinisch chemischen Bereich des Analysators eingesetzt werden, in einem bodennahen Bereich vorzugsweise planparallel zueinander angeordnete Ein- und Austrittsfenster auf, die für die Eintritts- und Austrittsstrahlung bzw. Messstrahlung der optischen Messeinheit durchlässig sind.

In jenem Bereich, der zur Durchführung heterogener Immunoassays dient, wobei die Detektion über Chemilumineszenz erfolgt, benötigen die Küvetten des Küvettenarrays in einem bodennahen Bereich lediglich ein seitliches Austrittsfenster, das für die Lumineszenzstrahlung optischen durchlässig ist,

Die Erfindung wird im Folgenden an Hand von zum Teil schematischen Ausführungsbeispielen näher erläutert. Es zeigen:

Fig. **1a**    einen automatischen Analysator mit auf Drehtellern kreisförmig angeordneten, verfahrbaren Reaktionsgefäßen bzw. Küvetten gemäß Stand der Technik,

Fig. 1b    einen automatischen Analysator mit linear angeordneten, verfahrbaren Reaktionsgefäßen bzw. Küvetten gemäß Stand der Technik,

Fig. 1c    einen automatischen Analysator für klinisch chemische Analysen und zur Durchführung heterogener Immunoassays gemäß Stand der Technik,

Fig. 2a    bis Fig. 2d optische Messeinheiten zur Gewinnung von Messsignalen von flüssigen Medien gemäß Stand der Technik,

Fig. 2e    bis Fig. 2h Vorrichtungen zum Mischen bzw. Rühren von Flüssigkeiten in Küvetten gemäß Stand der Technik,

Fig. 3a    eine erste Ausführungsvariante eines erfindungsgemäßen, automatischen Analysators zur Durchführung von chemischen, biochemischen und/oder immunchemischen Analysen von flüssigen Proben mit einem linearen, stationären Küvettenarray in einer dreidimensionalen Gesamtansicht,

Fig. 3b    eine Schnittdarstellung des Analysators gemäß Linie IV-IV in Fig. 3c,

Fig. 3c    eine vereinfachte Draufsicht auf den Analysator gemäß Fig. 3a,

Fig. 4    zwei unabhängig voneinander verfahrbare Pipettoren des automatischen Analysators gemäß Fig. 3a in einer dreidimensionalen Ansicht,

Fig. 5    eine verfahrbare, optische Messeinheit des automatischen Analysators gemäß Fig. 3a in einer Schnittdarstellung,

Fig. 6    eine verfahrbare Küvettenwascheinheit des automatischen Analysators gemäß Fig. 3a in einer dreidimensionalen Ansicht,

Fig. 7    eine Nadelwascheinheit des automatischen Analysators gemäß Fig. 3a in einer dreidimensionalen, teilweise aufgeschnittenen Ansicht,

Fig. 8    eine Thermostatisiereinheit für die Küvetten des automatischen Analysators gemäß Fig. 3a in einer dreidimensionalen, teilweise aufgeschnittenen Ansicht,

Fig. 9a     Fluidikelemente einer Pipettiernadel eines Pipettors gemäß Fig. 4 in einer schematischen Darstellung,

Fig. 9b     Fluidikelemente einer Nadelwascheinheit gemäß Fig. 7 in einer schematischen Darstellung, sowie

Fig. 9c     Fluidikelemente einer Küvettenwascheinheit gemäß Fig. 6 in einer schematischen Darstellung.

Fig. 10a     eine zweite Ausführungsvariante eines erfindungsgemäßen, automatischen Analysators zur Durchführung von chemischen, biochemischen und/oder immunchemischen Analysen von flüssigen Proben mit einem linearen, stationären Küvettenarray in einer dreidimensionalen Gesamtansicht,

Fig. 10b     eine Schnittdarstellung des Analysators gemäß Linie IV-IV in Fig. 10c,

Fig. 10c     eine vereinfachte Draufsicht auf den Analysator gemäß Fig. 10a,

Fig. 11a     eine erste Variante einer erfindungsgemäßen, optischen Messeinheit zur Gewinnung von Messsignalen von flüssigen Medien in einer dreidimensionalen Ansicht, mit Blickrichtung auf die Lichtbereitstellungseinheit gemäß Fig. 10a bis 10c,

Fig. 11b     die Ausführungsvariante gemäß Fig. 11a in einer dreidimensionalen Ansicht, mit Blickrichtung auf die Detektionseinheit,

Fig. 11c     eine Schnittdarstellung der Lichtbereitstellungseinheit gemäß Fig. 11a nach Linie II-II in Fig. 11d,

Fig. 11d     eine Schnittdarstellung der Lichtbereitstellungseinheit gemäß Fig. 11a nach Linie III-III in Fig. 11c,

Fig. 11e     eine dreidimensionale Detaildarstellung eines Röhrenkörpers der Lichtbereitstellungseinheit gemäß Fig. 11a,

Fig. 11f     eine vergrößerte Detaildarstellung aus Fig. 11c,

Fig. 12a     ein Blockschaltbild zur elektronischen Ansteuerung der optischen Messeinheit gemäß Fig. 11a,

Fig. 12b     ein erstes Diagramm zur Darstellung eines Messablaufs (Modi 1 und 2),

Fig. 12c     ein zweites Diagramm zur Darstellung eines Messablaufs (Modus 3),

Fig. 13a     eine zweite Variante einer erfindungsgemäßen optischen Messeinheit zur Gewinnung von Messsignalen von flüssigen Medien in einer dreidimensionalen Ansicht eines automatischen Analysators gemäß Fig. 10a bis 10c,

Fig. 13b     eine vergrößerte Schnittdarstellung durch die Achse einer Küvette, normal auf das Küvettenarray gemäß Fig. 13a,

Fig. 14a     eine dritte Variante einer erfindungsgemäßen optischen Messeinheit zur Gewinnung von Messsignalen von flüssigen Medien in einer dreidimensionalen Ansicht eines automatischen Analysators gemäß Fig. 10a bis 10c,

Fig. 14b     eine vergrößerte Schnittdarstellung durch die Achse einer Küvette, normal auf das Küvettenarray gemäß Fig. 14a,

Fig. 14c     eine vergrößerte Detaildarstellung aus Fig. 14a.

Fig. 15a     eine erfindungsgemäße Vorrichtung zum Mischen und Thermostatisieren flüssiger Medien in einer dreidimensionalen Darstellung eines automatischen Analysators gemäß Fig. 10a bis 10c,

Fig. 15b     die Vorrichtung gemäß Fig. 15a in einer Schnittdarstellung gemäß Fig. 15a,

Fig. 15c     eine Küvette samt Ultraschall-Wandler der erfindungsgemäßen Vorrichtung gemäß Fig. 15a in einer dreidimensionalen Ansicht,

Fig. 16    ein Blockschaltbild zur elektronischen Ansteuerung der Vorrichtung zum Mischen und Thermostatisieren flüssiger Medien gemäß Fig. 15a

Fig. 17a    ein Temperaturdiagramm zur Darstellung eines ersten Ausführungsbeispiels eines Thermostatisier- und Mischvorganges einer Flüssigkeit,

Fig. 17b    ein Temperaturdiagramm zur Darstellung eines zweiten Ausführungsbeispiels eines Thermostatisier- und Mischvorganges einer Flüssigkeit.

Fig. 18a    eine dritte Ausführungsvariante eines erfindungsgemäßen, automatischen Analysators zur Durchführung von chemischen, biochemischen und/oder immunchemischen Analysen von flüssigen Proben mit einem linearen, stationären Küvettenarray und einer Vorrichtungen zur Durchführung heterogener Immunoassays in einer dreidimensionalen Gesamtansicht,

Fig. 18b    eine Draufsicht auf den automatischen Analysator gemäß Fig. 18a,

Fig. 19a    die erfindungsgemäße Vorrichtung zur Durchführung heterogener Immunoassays gemäß Fig. 18a in einer dreidimensionalen Ansicht,

Fig. 19b    ein Detail der Vorrichtung gemäß Fig. 19a in einer vergrößerten Schnittdarstellung,

Fig. 20    ein schematisiertes Ablaufbeispiel eines heterogenen Immunoassays,

Fig. 21    ein Fluidschaltbild der Vorrichtung gemäß Fig. 19a, sowie

Fig. 22    ein Blockschaltbild zur elektronischen Steuerung der Vorrichtung gemäß Fig. 19a.

Funktionsgleiche Teile sind in den Ausführungsvarianten mit gleichen Bezugszeichen versehen.

Die in den Fig. 1a bis 1c sowie 2a bis 2h dargestellten automatischen Analysatoren und deren Komponenten betreffen Beispiele zum Stand der Technik und werden in der Beschreibungseinleitung ausführlich beschrieben.

Der in den Fig. 3a bis 3c dargestellte automatische Analysator 100 einer ersten Ausführungsvariante dient zur Durchführung von chemischen, biochemischen und/oder immunchemischen Analysen von flüssigen Proben. Zur Vereinfachung sind nur jene Komponenten des Analysators 100 dargestellt, die für die gegenständliche Erfindung wesentlich sind, wobei auf Analysatorkomponenten, wie Pumpen, Ventile, Auswerte-, Steuer- und Antriebseinheiten, nicht näher eingegangen wird.

Die flüssigen Proben liegen in Probengefäßen 921 in einem Probenlager 920 des Analysators 100 vor und werden unter Zuhilfenahme von flüssigen Reagenzien, die in Reagenziengefäßen 951a, 951b in zwei Reagenzienlagern 950a, 950b des Analysators 100 vorliegen, analysiert.

Die Küvetten 201 zur Aufnahme der flüssigen Proben und Reagenzien, sind in Form eines stationären, linearen Küvettenarrays 200 im Analysator 100 angeordnet und verbleiben während einer Vielzahl von Einzelanalysen an deren ursprünglichen Position. Das Küvettenarray 200 ist im dargestellten Beispiel zwischen dem ersten Reagenzienlager 950a und dem zweiten Reagenzienlager 950b angeordnet.

Der automatische Analysator 100 ist mit verfahrbaren und stationären Automatenkomponenten ausgestattet und zwar:

- mit zwei entlang einer durch das lineare Küvettenarray 200 definierten Bewegungslinie in x-Richtung verfahrbaren Pipettoren 300a, 300b, die jeweils mit zwei Pipettiernadeln 301a1, 301a2 bzw. 301b1, 301b2 ausgestattet sind, die in z-Richtung in die Küvetten 201, in die im Probenlager 920 befindlichen Probengefäße 921 und in die in den Reagenzienlagern 950a, 950b befindlichen Reagenziengefäße 951a, 951b absenkbar ausgeführt sind und in einer auf die x-Richtung im Wesentlichen normal stehenden y-Richtung zwischen den Küvetten 201 und dem Probenlager 920 und/oder den beiden Reagenzienlagern 950a, 950b verfahrbar ausgeführt sind;

- mit einer Mischereinheit 400 zur Vermischung der Proben und Reagenzien in den Küvetten 201;

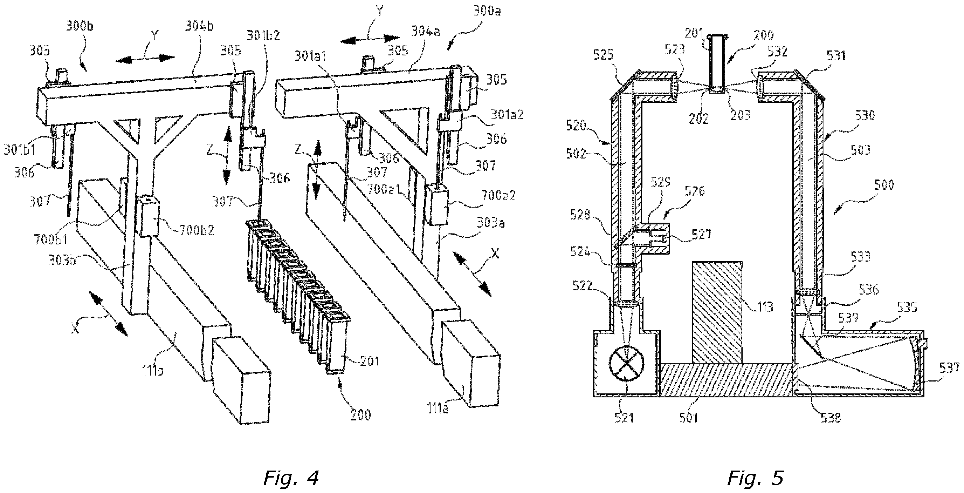

- mit einer optischen Messeinheit 500, welche - zur Gewinnung eines Messsignals - durch ein seitlich an der Küvette 201 angeordnetes Messfenster 202, 203 austretende Messstrahlung empfängt (siehe Fig. 5);

- mit einer Küvettenwascheinheit 600 zur Reinigung der Küvetten 201, die entlang der durch das Küvettenarray 200 definierten Bewegungslinie in x-Richtung verfahrbar ist,

- mit Nadelwascheinheiten 700a1, 700a2, 700b1, 700b2 zur Reinigung der Pipettiernadeln 301a1, 301a2, 301b1, 301b2 der beiden Pipettoren 300a, 300b; sowie

- mit einer stationären Thermostatisiereinheit 800 zur Einstellung einer vorgebbaren Messtemperatur in den Küvetten 201.

Die Pipettoren 300a, 300b sind mittels verfahrbaren Aufnahmeelementen (nicht dargestellt) an den parallel angeordneten Schienen lila, 111b befestigt, weiterhin ist eine entsprechende Schiene 113 samt verfahrbarer Aufnahme 501 für die optische Messeinheit 500 sowie eine Schiene 112 samt verfahrbarer Aufnahme 601 für die Küvettenwascheinheit 600 vorgesehen. Die verfahrbaren Aufnahmen der Pipettoren 300a, 300b, und die Aufnahmen 501 und 601 werden beispielsweise mittels hier nicht weiter dargestellten Zahnriemen und Steppermotoren an einem Ende der Schienen 112, 113, lila und 111b angetrieben.

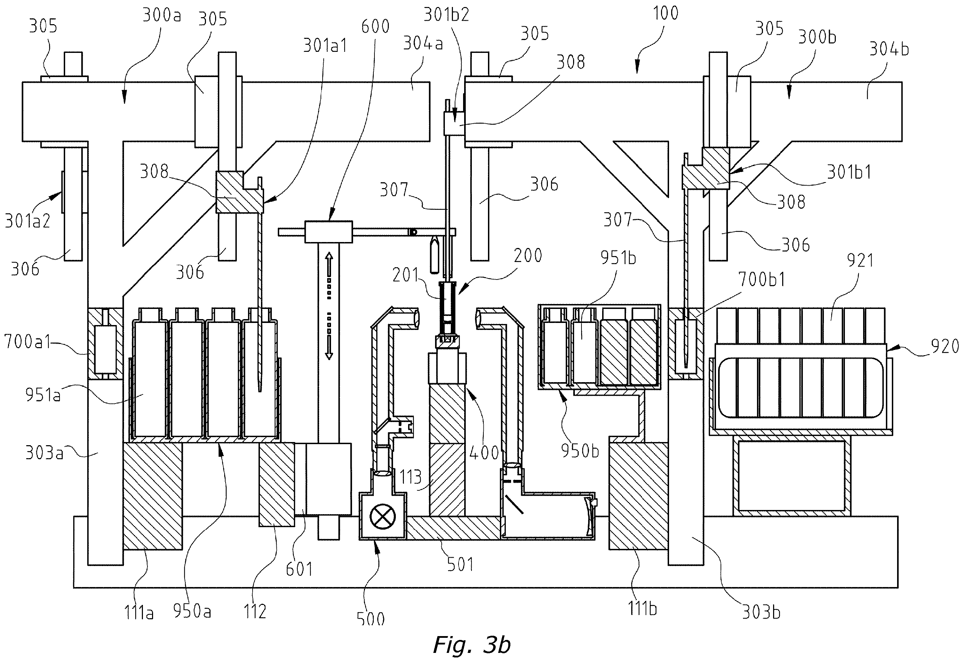

Wie insbesondere in Fig. 3b erkennbar ist, sind zumindest zwei - im dargestellten Beispiel mehrere - der Automatenkomponenten unabhängig voneinander entlang bzw. parallel zu der durch das lineare Küvettenarray 200 definierten Bewegungslinie in x-Richtung verfahrbar ausgeführt, und können jeweils auf unterschiedliche Küvetten 201 oder Gruppen von Küvetten 201 in frei wählbarer Reihenfolge zugreifen.

In der dargestellten Ausführungsvariante gemäß Fig. 3a bis 3c weist der Analysator 100 ein Probenlager 920, ein erstes Reagenzienlager 950a und ein zweites Reagenzienlager 950b auf. Die Lagerbereiche können ganz oder teilweise gekühlt sein.

Zur Beschickung des Analysators 100 mit Probenmaterial werden Gefäße 921 mit Analysenproben manuell oder mittels einer Robotik in vorbestimmte Positionen in das Probenlager 920 eingebracht. Die für die einzelnen Analysenproben gewünschten Analysen werden in die Steuerung des Analysators 100 eingegeben.

Zur Beschickung des Analysators mit Reagenzien werden Reagenziengefäße 951a, 951b mit Reagenzien für die Analyse unterschiedlicher Analyte manuell oder mittels einer Robotik in die beiden Reagenzienlager 950a, 950b des Analysators 100 in vorbestimmte Positionen eingebracht.

In die Proben- bzw. Reagenzienlager können auch Gefäße mit Kalibrierflüssigkeiten und Vergleichsproben eingebracht werden.

In der dargestellten Ausführungsvariante weist der Analysator zwei unabhängig voneinander in x-Richtung verfahrbare Pipettoren 300a, 300b auf, die - unter Ausnahme derselben Küvette - völlig unabhängig voneinander und bei frei wählbarer Reihenfolge auf einzelne Küvetten 201 des Küvettenarrays 200 zugreifen können.

Die beiden Pipettoren 300a, 300b gemäß Fig. 4 weisen jeweils einen vertikalen Turm 303a, 303b, sowie einen horizontal in y-Richtung ausgerichteten Arm 304a, 304b auf, sodass eine im Wesentlichen L-förmige Trägerstruktur (Pipettor 300a) für die beiden Pipettiernadeln 301a1, 301a2 bzw. T-förmige Trägerstruktur (Pipettor 300b) für die beiden Pipettiernadeln 301b1, 301b2 ausgebildet wird, die entlang der Schiene lila bzw. 111b in x-Richtung verfahrbar ist. Jeder Pipettor weist somit zwei unabhängig voneinander, parallel zueinander in y-Richtung verfahrbare Pipettiernadeln 301a1, 301a2 bzw. 301b1, 301b2 mit deren Kanülen bzw. Hohlnadeln 307 auf. Die Pipettiernadeln 301a1, 301a2 bzw. 301b1, 301b2 sind mittels einer in y-Richtung verfahrbaren Aufnahme 305 links und rechts des Arms 304a bzw. 304b befestigt und können dadurch ungehindert aneinander vorbeifahren. Jede Aufnahme 305 weist einen nach unten ragenden Schienenabschnitt 306 auf, an welchem die Nadel in z-Richtung in die Küvetten 201 des Küvettenarrays 200 abgesenkt werden kann.

Die einzelnen Pipettiernadeln 301a1, 301a2 bzw. 301b1, 301b2 weisen jeweils einen Nadelhalter 308 mit einem in Richtung des Küvettenarrays 200 auskragenden Bereich auf, der die Hohlnadel 307 trägt. Dadurch bleibt selbst bei einer fluchtend auf die Küvette 201 ausgerichteten bzw. abgesenkten Hohlnadel 307 der Pipettiernadel 301b2 genügend Freiraum für den L-förmigen Pipettor 300a um am T-förmigen Pipettor 300b vorbeifahren zu können (siehe Fig. 3b).

Im dargestellten Beispiel kann somit der Pipettor 300b bzw. dessen beide Pipettiernadeln 301b1, 301b2 nur auf die Probengefäße 921 im Probenlager 920 und auf die Reagenziengefäße 951b im Reagenzienlager 950b zugreifen, wohingegen der Pipettor 300a bzw. seine Pipettiernadeln 301a1 301a2 nur Zugriff auf die im Reagenzienlager 950a angeordneten Reagenziengefäße 951a hat. Alle Pipettiernadeln 301a1, 301a2 bzw. 301b1, 301b2 können bis zur Ebene des Küvettenarrays 200 verfahren und in die einzelnen Küvetten 201 abgesenkt werden.

Eine wesentliche Erhöhung des Probendurchsatzes kann dadurch erzielt werden, dass die Nadelwascheinheiten 700a1, 700a2 bzw. 700b1, 700b2 am Pipettor 300a bzw. 300b angeordnet und mit diesem verfahrbar ausgeführt sind. In der dargestellten Ausführungsvariante weist jede Pipettiernadel 301a1, 301a2, 301b1, 301b2 eine eigene Nadelwascheinheit 700a1, 700a2, 700b1, 700b2 auf, die beispielsweise jeweils am vertikalen Turm 303a bzw. 303b des Pipettors 300a bzw. 300b angeordnet sein kann. Es kann somit jeweils eine der Pipettiernadeln 301a1 bzw. 301b1 in der zugeordneten Nadelwascheinheit 700a1 bzw. 700b1 gewaschen werden, während die jeweils andere Pipettiernadel 301a2, 301b2 in eine Küvette 201 eintaucht (siehe Fig. 4).

Es sind auch einfache Ausführungsvarianten des Analysators denkbar, die nur einen Pipettor aufweisen. Dieser kann entweder als L-förmiger Pipettor 300a seitlich an einem Proben- oder Reagenzienlager verfahrbar ausgeführt sein und nur eine verfahrbare Pipettiernadel 301a1 aufweisen oder auch eine T-förmige Trägerstruktur aufweisen und zwischen einem Proben- und einem Reagenzienlager verfahrbar ausgeführt sein.

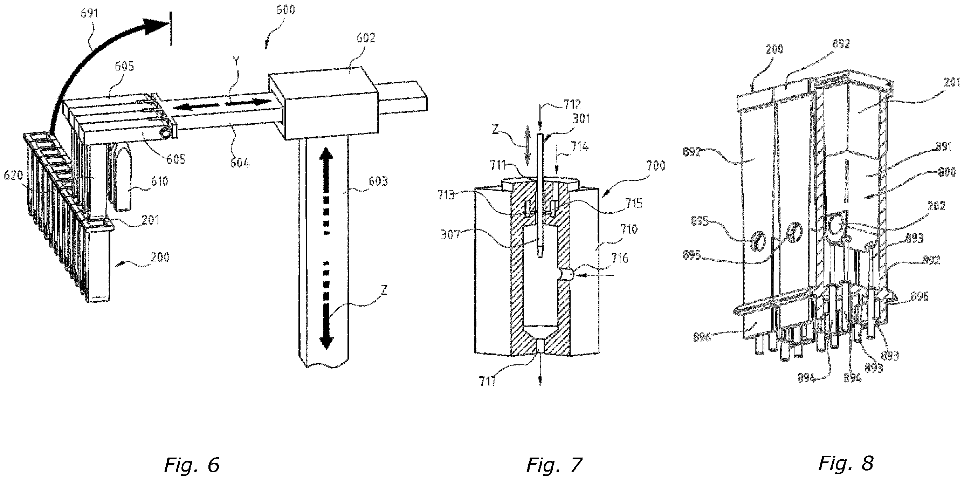

Die in Fig. 5 dargestellte optische Messeinheit 500 ist als eine entlang des linearen, stationären Küvettenarrays 200 an der Schiene 113 mit Hilfe der Aufnahme 501 verfahrbare Einheit ausgebildet. Diese besteht in dem in Fig. 5 dargestellten Beispiel aus einer Lichtbereitstellungseinheit (light supplying unit) 520 auf einer Seite des Küvettenarrays 200 und einer spektroskopischen Einheit 530 auf der anderen Seite, die über die Aufnahme 501 starr miteinander verbunden sind. Die optische Messeinheit 500 umfasst eine Lichtquelle 521, beispielsweise eine Halogenlampe, jeweils einen Strahlengang für die Eintritts- 502 und die Austritts- bzw. Messstrahlung 503 mit Linsen 522, 523, 532 533, Filtern 524, Umlenkspiegeln 525, 531 und einem Spektrometer 535, welches das Spektrum der Messstrahlung bzw. die Intensität der Messstrahlung bei einzelnen vorbestimmten Wellenlängen im Bereich von 300 bis 800 nm erfasst. Das Spektrometer 535 besteht in dem in Fig. 7 dargestellten Beispiel aus einem Polychromator umfassend einen Eintrittsspalt 536, einen Umlenkspiegel 539 und ein konkaves Beugungsgitter 537, welches das Spektrum der Messstrahlung 503 auf ein Sensorarray 538, beispielsweise ein Photodiodenarray, abbildet. Die in der Küvette 201 befindliche Flüssigkeit wird im dargestellten Beispiel im Durchlicht vermessen, wobei die Eintrittsstrahlung 502 durch ein seitliches Eintrittsfenster 202 in die Küvette 201 eintritt und durch ein gegenüberliegendes Austrittsfenster 203 aus der Küvette 201 austritt.

Bevorzugt umfasst die optische Messeinheit 500 einen Referenzdetektor 526 zwecks Messung und Kompensation von Schwankungen der Intensität des von der Lichtquelle 521 emittierten Lichts. Dieser besteht beispielsweise aus einem im Strahlengang für die Eintrittsstrahlung 502 befindlichen Strahlteiler 528, einer Blende 529 und einem Photodetektor 527, beispielsweise einer Photodiode.

Mit der oben beschriebenen, optischen Messeinheit 500 können verschiedene optische Messungen bei einzelnen und/oder multiplen Wellenlängen im Wellenlängenbereich des ultravioletten und sichtbaren Lichtbereichs durchgeführt werden. Beispiele dafür sind photometrische, turbidimetrische, und luminometrische Messungen.

Im Folgenden wird ein optischer Messvorgang am Beispiel einer photometrischen Messung beschrieben. Die von der polychromatischen Lichtquelle 521 stammende Eintrittsstrahlung 502 durchläuft die in der Küvette 201 befindliche Reaktionsmischung aus Probe und den für die jeweilige Analyse zugesetzten Reagenzien, tritt als Messstrahlung 503 in die spektroskopische Einheit 530 ein, und wird im Spektrometer 535 bezüglich der Wellenlängen am Beugungsgitter 537 aufgeteilt und vom Sensorarray 538 aufgenommen. Die einzelnen Licht aufnehmenden Elemente des Sensorarrays 538 des Spektrometers 535 beispielsweise Photodioden, sowie die Referenzphotodiode 527 des Referenzdetektors 526 geben einen ihrer jeweiligen Messwellenlänge entsprechenden Photostrom ab, der von einer Signalverarbeitungsschaltung und mittels AD-Konverter in einen digitalen Messwert umgewandelt wird. In einer Operationseinheit werden - abhängig von der jeweiligen Analyse - einzelne oder periodisch über die Zeit und bei einer oder mehreren Wellenlängen gemessene digitale Messwerte mit, den der jeweiligen Analyse zugeordneten vorbekannten Referenz- und Kalibrierwerten, zu einem Konzentrationswert des Analyten verrechnet.

Zur Vermischung der Proben und Reagenzien ist dem gesamten Küvettenarray 200, bevorzugt einzelnen Gruppen oder Segmenten 210 von Küvetten 201, eine stationäre Mischereinheit 400, zugeordnet (hier nicht näher dargestellt). Die in Fig. 6 dargestellte Küvettenwascheinheit 600 ist über eine Aufnahme 601 entlang der Schiene 112 (siehe Fig. 3b) in x-Richtung verfahrbar ausgeführt. Der Kopf 602 der Einheit 600 kann mit Hilfe eines vertikal ausgerichteten Schienenabschnitts 603, der in der Aufnahme 601 geführt ist, in z-Richtung auf und ab bewegt werden, um entweder die Waschkörper 610 oder die Trockenstempel 620 in die Küvetten 201 des Küvettenarrays 200 einzuführen. Über ein Verstellelement 604, das im Kopf 602 geführt ist und die beispielsweise vier Trockenstempel 620 sowie Waschkörper 610 trägt, kann durch eine Verschiebung in y-Richtung von der Waschposition in die Trocknungsposition umgeschaltet werden. Einzelne Finger 605, die die Waschkörper 610 und Trockenstempel 620 tragen, können - wie mit Pfeil 691 angedeutet - hochgeschwenkt werden, sodass nur eine oder wenige Küvetten 201 gleichzeitig gewaschen werden.

Fig. 7 zeigt in einer vergrößerten Schnittdarstellung den Aufbau einer mit dem allgemeinen Bezugszeichen 700 gekennzeichnete Nadelwascheinheit, die den im Wesentlichen baugleichen, an unterschiedlichen Positionen in den Fig. 3a bis 3c und 4 dargestellten Nadelwascheinheiten 700a1, 700a2, 700b1, 700b2 entspricht, und eine mit dem allgemeinen Bezugszeichen 301 gekennzeichnete Pipettiernadel, die den im Wesentlichen baugleichen, an unterschiedlichen Positionen in den Fig. 3a bis 3c und 4 dargestellten Pipettiernadeln 301a1, 301a2, 301b1, 301b2 entspricht. Die Hohlnadel 307 der Pipettiernadel 301, wird durch eine Aufnahmeöffnung 711 im Gehäuse 710 einer Nadelwascheinheit 700 eingeführt, wobei gleichzeitig das Lumen der Hohlnadel 307 mit einer Systemflüssigkeit 712 und die Außenseite der Nadel mit einer über seitliche Reinigungsdüsen 713 aus einer Ringkammer 715 zugeführten Spülflüssigkeit 714 gereinigt werden kann. Zur Innen- und Außenreinigung der Hohlnadel 307 durch wiederholtes Ansaugen und Ausstoßen von Waschlösung aus dem unteren Teil der Nadelwascheinheit 700, kann über einen radialen Einlass 716 Waschlösung vorgelegt werden, welche anschließend über eine Absaugöffnung 717 entleert werden kann.

Fig. 8 zeigt einen vergrößerten Ausschnitt aus dem linearen Küvettenarray 200 des Analysators 100 mit dem teilweise aufgeschnittenen Gehäuse 892 und einer darin angeordneten Küvette 201, welche zur Einstellung einer vorgebbaren Messtemperatur von einer Heizfolie 891 einer Thermostatisiereinheit 800 kontaktiert wird, deren elektrische Kontaktstifte 893 aus dem Gehäuse 892 austreten. Weitere elektrische Kontaktstifte 894 können für die Kontaktierung eines Temperatursensors vorgesehen sein. Die Küvette 201 weist seitlich in einem bodennahen Bereich, vorzugsweise planparallel zueinander angeordnete Messfenster, im dargestellten Beispiel Ein- und Austrittsfenster 202, 203 (Austrittsfenster nicht sichtbar) auf, die für die Eintrittsstrahlung und die Austritts- bzw. Messstrahlung der optischen Messeinheit 500 durchlässig sind. Im Bereich der Ein- und Austrittsfenster 202, 203 der Küvette 201 weist das Gehäuse 892 korrespondierende Öffnungen 895 auf. Die einzelnen Kontaktstifte 893, 894 rasten in entsprechende Kontaktöffnungen ein. Am Boden der Gehäuse 892 sind Rastelemente 896 angeformt, die zur Befestigung des Küvettenarrays 200 dienen.

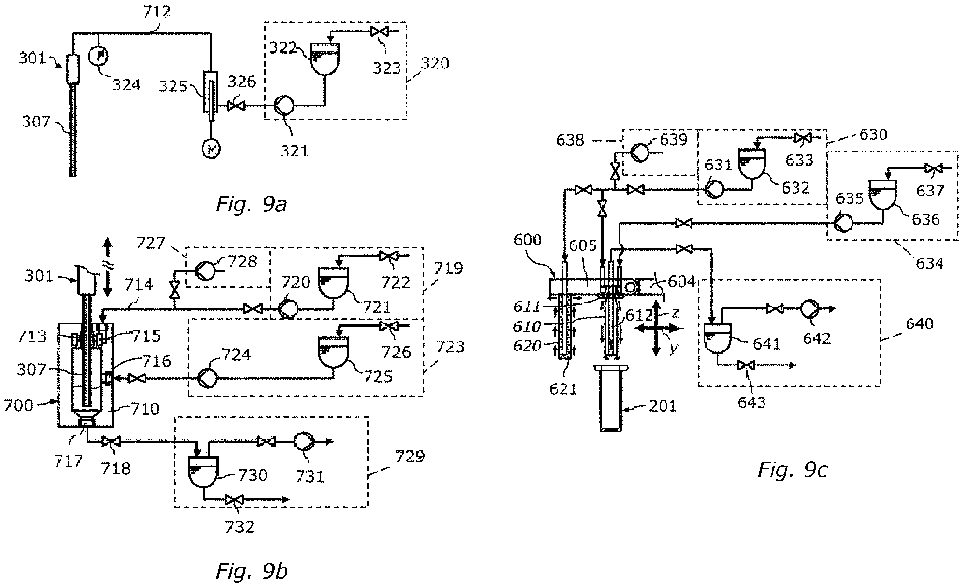

Fig. 9a zeigt das Fluidikschaltbild einer Pipettiernadel 301, deren Hohlnadel 307 über einen mit einer entgasten Flüssigkeit gefüllten Druckübertragungskanal 712 mit einer Präzisionskolbenpumpe 325, vorzugsweise einer von einem Schrittmotor angetriebenen Verdrängerpumpe (Dilutor), verbunden ist. Die Verdrängerpumpe verfügt seitlich über einen zusätzlichen Flüssigkeitsanschluss, der über ein Magnetventil 326 an eine Bereitstellungseinheit 320 für eine Systemflüssigkeit angeschlossen ist, die über eine Spülpumpe 321 aus einem Vorratsgefäß 322 z.B. entgastes, deionisiertes Wasser fördert, welches über ein Magnetventil 323 nachfüllbar, oder unter Druck setzbar ist.

Zur Detektion von Störungen verfügt der Druckübertragungskanal 712 in der Nähe der Pipettiernadel 301 über einen weiteren Anschluss zu einem Drucksensor 324, der mit einer hier nicht dargestellten Auswerte- und Kontrolleinheit, beispielsweise zur Detektion von Verstopfungen der Hohlnadel 307, verbunden ist.

Beschreibung eines Pipettiervoraanas

Für den Transfer einer definierten Flüssigkeitsmenge mit der Pipettiernadel 301 wird diese zunächst in horizontaler Richtung zu einem ersten Gefäß bewegt, 5 µL Luft (Spacer) in die Spitze der Hohlnadel 307 eingesaugt und die Pipettiernadel 301 in Richtung der Flüssigkeitsoberfläche des ersten Gefäßes abgesenkt. Um eine ausreichende, aber nicht zu große Eintauchtiefe der Pipettiernadel 301 zu gewährleisten, wird die Abwärtsbewegung der Hohlnadel 307 in definierter Eintauchtiefe durch ein Signal einer Flüssigkeitsoberflächen-Detektionsvorrichtung (nicht dargestellt), beispielsweise mit kapazitivem Detektionsprinzip, gestoppt. Zur Aspiration einer definierten Menge an Flüssigkeit mit hoher Genauigkeit im µL Bereich wird nun durch Abwärtsbewegung des Arbeitskolbens der in Fig. 9a dargestellten Verdrängerpumpe (Dilutor) ein Unterdruck in der Hohlnadel 307 der Pipettiernadel 301 erzeugt, welcher die Aspiration eines entsprechenden Flüssigkeitsvolumens aus einem ersten Gefäß bewirkt. Die Pipettiernadel 301 wird nun samt der aspirierten Flüssigkeit, welche durch eine Trennluftblase (Spacer) von der Systemflüssigkeit getrennt ist, zu einem zweiten Gefäß bewegt, wobei der Prozess nun in umgekehrter Richtung abläuft und die aspirierte Flüssigkeit über die Spitze der Hohlnadel 307 in das zweite Gefäß abgegeben wird. Zumindest zwischen zwei Pipettiervorgängen mit unterschiedlichen zu pipettierenden Flüssigkeiten erfolgt stets eine Innen- und Außenreinigung der Pipettiernadel 301 in einer Nadelwascheinheit 700 (siehe Fig. 7).

Fig. 9b zeigt das Fluidikschaltbild einer Nadelwascheinheit 700 gemäß Fig. 7 mit darin abgesenkter Hohlnadel 307 der Pipettiernadel 301. Das Gehäuse 710 der Nadelwascheinheit verfügt im oberen Bereich über eine konzentrisch umlaufende Ringkammer 715, die als Medienzuführung für mehrere innenliegende, konzentrisch ausgerichtete Reinigungsdüsen 713 fungiert, und die jeweils über Magnetventile mit einer Bereitstellungseinheit 719 für eine Spülflüssigkeit (beispielsweise deionisiertes Wasser), und eine Bereitstellungseinheit 727 für Trockenluft verbunden ist.

Ein in der Mitte der Höhe des Gehäuses 710 der Nadelwascheinheit 700 radial angeordneter Einlass 716 ist ebenfalls mit einem Magnetventil verbunden, und dient ausschließlich der Zufuhr von tensidhaltiger Waschlösung aus einer Bereitstellungseinheit 723.

Die Bereitstellungseinheiten 719 für eine Spülflüssigkeit und 723 für eine Waschlösung verfügen jeweils über eine Pumpe 720, 724, die eine tensidhaltige Waschlösung bzw. Spülflüssigkeit aus den jeweiligen Vorratsbehältern 721, 725 fördern, die jeweils über ein Magnetventil 722, 726 nachfüllbar, oder unter Druck setzbar sind. Die Bereitstellungseinheit 727 für Luft weist eine Luftpumpe 728 zur Bereitstellung komprimierter Luft und ggf. eine Trocknungsvorlage (nicht dargestellt) auf.

Die am Boden der Nadelwascheinheit 700 befindliche Absaugöffnung 717 ist über ein Magnetventil 718 mit der unter Unterdruck stehenden Abwassersammeleinheit 729 verbunden, welche im Wesentlichen aus einem Sammelbehälter 730 besteht, der im Gasraum über der Flüssigkeit über einen Anschluss zu einer Vakuumpumpe 731 verfügt, die über ein Magnetventil mit dem Sammelbehälter 730 verbunden ist.

Die gesammelten Abwässer können über ein Magnetventil 732 am Boden des Sammelbehälters 730 abgeführt werden und einer weiteren Abwasserbehandlung zugeführt werden.

Beschreibung eines Nadelwaschvoraanas

In einem typischen Waschprozess der Pipettiernadel 301 wird diese zunächst horizontal zur Nadelwascheinheit 700 bewegt und in die untere Halteposition der Waschkammer abgesenkt. Alle bei der Reinigung der Pipettiernadel 301 anfallenden Abwässer werden über die am Boden befindliche Absaugöffnung 717 abgesaugt, gesammelt, und gegebenenfalls nachbehandelt. Anschließend werden über die in Fig. 9a dargestellte Präzisionskolbenpumpe 325 der Pipettiernadel 301 zunächst in und an der Nadelspitze befindliche Restmengen der zuletzt pipettierten Flüssigkeit entleert und abgesaugt. Schließlich wird die abgesenkte Pipettiernadel 301 von hinten mittels der in Fig. 9a dargestellten Bereitstellungseinheit 320 für Systemflüssigkeit gespült.

In einem nächsten Schritt wird (bei geschlossenem Magnetventil 718 an der Absaugöffnung 717) durch den Einlass 716 im Gehäuse 710 der Nadelwascheinheit 700 ein definiertes Volumen tensidhaltiger Waschlösung eingeleitet, wodurch sich die Kammer im unteren Teil mit einem definierten Pegel an Waschlösung füllt. Die Hohlnadel 307 der Pipettiernadel 301 wird so weit abgesenkt, dass durch das Eintauchen in die Waschlösung eine Außenbenetzung der Nadel, und durch Aufsaugen der Waschlösung in das Nadelinnere eine Innenbenetzung der Hohlnadel 307 erfolgen kann. Anschließend wird die aspirierte Waschlösung wieder ausgestoßen, wobei der Prozess des Aufsaugens und Ausstoßens der Waschlösung mehrfach wiederholt werden kann, um die Reinigungswirkung zu verbessern.

In einem letzten Schritt wird die kontaminierte Waschlösung abgesaugt und das Innere der Hohlnadel 307 mit Systemflüssigkeit (z.B. entgastes, deionisiertes Wasser) gespült, während die Außenseite der Hohlnadel 307 gleichzeitig durch die obenliegenden, konzentrisch angeordneten Reinigungsdüsen 713 mit Spülflüssigkeit aus der Bereitstellungseinheit 719 gespült wird, wobei die Spitze der Hohlnadel 307 von unten nach oben bewegt wird, um die Reinigungswirkung zu verbessern.

Nach Beendigung der simultanen Innen- und Außenspülung wird die Hohlnadel 307 erneut in die untere Halteposition bewegt, die Medienzuführung der Reinigungsdüsen 713 auf die Bereitstellungseinheit 727 für komprimierte Luft umgeschaltet und die Spitze der Hohlnadel 307 erneut von unten nach oben bewegt, wodurch anhaftende Wassertropfen von der Nadeloberfläche rasch entfernt werden können. Die Pipettiernadel 301 kann nun aus der Nadelwascheinheit 700 bewegt werden, und ist nach der Aspiration eines Trennluft-Spacers (5 μL) erneut für eine Pipettierung bereit.

Fig. 9c zeigt das Fluidikschaltbild und den Längsschnitt eines am Verstellelement 604 angelenkten Fingers 605 der Küvettenwaschstation 600 mit einem Waschkörper 610 und einem Trockenstempel 620 (siehe auch Fig. 6), wobei die Beschreibungen der Bereitstellungseinheiten 630 (Spülflüssigkeit), 634 (Waschlösung) und 638 (Luft), sowie der Abwassersammeleinheit 640 den Bereitstellungseinheiten 719 (Spülflüssigkeit), 723 (Waschlösung), 727 (Luft) und 729 (Abwasser) der Figurenbeschreibung zu Fig. 9b entnommen werden können, die mit den in Fig. 9c dargestellten Einheiten funktionsgleich, bzw. baugleich sind.

Der Waschkörper 610, sowie der Trockenstempel 620 des Fingers 605 der Küvettenwaschstation 600 können durch horizontale und vertikale Translationsbewegungen nacheinander in die zu waschende Küvette 201 eines linearen Küvettenarrays abgesenkt werden, wobei nach dem Absenken in die Küvette 201 jeweils ein umlaufender Spalt von weniger als 1 mm zwischen der Innenseite der Küvette 201 und dem Waschkörper oder Trockenstempel frei bleibt, um eine kontrollierte Strömung der Reinigungsmedien entlang der inneren Küvettenwandung zu ermöglichen.

Der Waschkörper 610 verfügt an seinem oberen Ende über eine Elastomerdichtung 611, die ein Austreten der Reinigungsmedien zwischen dem oberen Küvettenrand und der Unterseite des Fingers 605 während des Waschvorgangs verhindert. Um den Schaft des in der Mitte des Waschkörpers 610 verlaufenden Steigkanals 612 zur Absaugung der Abwässer und Abluft herum ist eine ringförmig umlaufende Medienzuführung angeordnet, die ein Spülen der Küvetteninnenseite von oben nach unten ermöglicht (siehe Pfeile). Der Waschkörper 610 kann über entsprechende Magnetventile mit tensidhaltiger Waschlösung aus der Bereitstellungseinheit 634, Spülflüssigkeit (beispielsweise deionisiertes Wasser) aus der Bereitstellungseinheit 630, oder mit komprimierter Luft aus der Bereitstellungseinheit 638 beschickt werden, welche über die unter Unterdruck stehende Abwassersammeleinheit 640 abgeführt werden, in dem diese über ein Magnetventil mit der unter Unterdruck stehenden Abwassersammeleinheit 640 zugeführt werden. Die Abwassersammeleinheit 640 besteht im Wesentlichen aus einem Sammelbehälter 730, der im Gasraum über der Flüssigkeit über einen Anschluss zu einer Vakuumpumpe 642 verfügt, die über ein Magnetventil mit dem Sammelbehälter 641 verbunden ist. Die gesammelten Abwässer können über ein Magnetventil 643 am Boden des Sammelbehälters 641 abgeführt werden und einer weiteren Abwasserbehandlung zugeführt werden.

Der Trockenstempel 620 besteht aus einem porösen luftdurchlässigen Material, und weist im Inneren einen nicht ganz bis zum Boden reichenden Längskanal 621 auf, welcher der Zuführung und Verteilung der komprimierten Luft durch die Wand des porösen Trockenstempels 620 hindurch in die Küvette 201 dient. Der Trockenstempel 620 schließt an der Unterseite des Fingers 605 nicht mit einer Dichtung ab, sondern steht im abgesenkten Zustand etwas über und bildet zwischen der Oberseite der Küvette 201 und Fingerunterseite einen umlaufenden Luftaustrittsspalt (siehe waagrechte Pfeile). Der Trockenstempel 620 kann über ein Magnetventil mit komprimierter Luft aus der Bereitstellungseinheit 638 verbunden werden.

Beschreibung eines Küvettenwaschvoraanas

In einem die eigentliche Reinigung vorbereitenden Schritt wird der Waschkörper 610 in die zu waschende Küvette 201 abgesenkt und das Reagenzien/Probengemisch, welches sich nach der Analyse in der Küvette 201 befindet, über den zentralen Steigkanal 612 abgesaugt und der Abwassersammeleinheit 640 zugeführt.

In einem ersten Reinigungsschritt wird mit Waschlösung aus der Bereitstellungseinheit 634, Spülflüssigkeit aus der Bereitstellungseinheit 630 und schließlich komprimierter Luft aus der Bereitstellungseinheit 638 gespült, wobei diese Reinigungssequenz mit den genannten Medien mehrfach wiederholt werden kann, um die Reinigungswirkung zu verbessern.

Der Waschkörper 610 wird nun aus der gewaschenen, jedoch Restfeuchte enthaltenden Küvette 201 gehoben, und der Finger in y-Richtung bewegt.

In einem zweiten Reinigungsschritt wird nun der Trockenstempel 620 in z-Richtung in die Küvette 201 abgesenkt und mit trockener komprimierter Luft aus der Bereitstellungseinheit 638 für eine bestimmte Zeitspanne Luft an der Küvetteninnenseite vorbeigeblasen, wobei die hierfür benötigte Luft aus dem porösen Körper des Trockenstempels 620 gleichmäßig austritt, von unten nach oben an der Innenseite der Küvette 201 entlangstreicht, und am Schaft des Trockenstempels 620 austritt.

Beispiele:

Der automatische Analysator gemäß Fig. 3a bis 3c arbeitet beispielsweise wie folgt:

Im Vorfeld einer Analyse, d.h. der Bestimmung eines Analyten $A_x$ einer Analysenprobe $P_x$ stellt die Steuereinheit des Analysators aus den bekannten und vorher eingegebenen Informationen alle für die Analyse des Analyten $A_x$ benötigten Daten zusammen (Analysenprotokoll, Positionen der Gefäße 921, 951a, 951b mit der Analysenprobe und mit den für die Analyse erforderlichen Reagenzien, Position einer freien Küvette 201 im Küvettenarray 200, Küvettentemperatur, Auswahl des Messablaufs, der Kalibrierdaten, der Mess- und Auswertealgorithmen).

Beispiel: Einzelanalvse

Phase 1

Zu Beginn und während der Analyse wird die Temperatur der für die Analyse vorgesehenen Küvette 201 mittels der der Küvette 201 zugeordneten Thermostatisiereinheit 800 auf eine vorbestimmte Temperatur geregelt.

Von der ersten Pipettiernadel 301b1 des T-förmigen Pipettors 300b wird im Probenlager 920 aus einem ersten Probengefäß 921 eine vorbestimmte Menge einer ersten Analysenprobe aufgenommen und eine vorbestimmte Menge davon in eine freie Küvette 201 abgegeben. Im Anschluss an den Pipettiervorgang wird die Pipettiernadel 301b1 in der ersten Nadelwascheinheit 700b1 des Pipettors 300b gewaschen und bereitgestellt.

Phase 2

Von einer Pipettiernadel 301a1 des L-förmigen Pipettors 300a wird im Reagenzienlager 950a aus einem ersten Reagenziengefäß 951a eine vorbestimmte Menge einer ersten Reagenzflüssigkeit aufgenommen und eine vorbestimmte Menge in die Küvette 201 pipettiert. Danach werden die beiden Flüssigkeiten in der Küvette durch kurzzeitiges (wenige Sekunden) Einschalten der, der Küvette zugeordneten Mischereinheit 400 vermischt. Im Anschluss an den Pipettiervorgang wird die Pipettiernadel 301a1 in einer ersten Nadelwascheinheit 700a1 des L-förmigen Pipettors 300a gewaschen und bereitgestellt.

Phase 3

Abhängig vom jeweiligen Analyseprotokoll wird von der zweiten Pipettiernadel 301b2 des T-förmigen Pipettors 300b im Reagenzienlager 950b eine vorbestimmte Menge einer zweiten Reagenzflüssigkeit aus einem Reagenziengefäß 951b aufgenommen und davon eine vorbestimmte Menge in die Küvette 201 dispensiert. Danach wird der Inhalt der Küvette durch kurzzeitiges (wenige Sekunden) Einschalten der, der Küvette 21 zugeordneten Mischereinheit 400 vermischt. Im Anschluss an den Pipettiervorgang wird die Pipettiernadel 301b2 in der zweiten Nadelwascheinheit 700b2 des T-förmigen Pipettors 300b gewaschen und bereitgestellt.

Phase 4

Die Phase 4 beginnt mit den photometrischen Messungen an Küvette 201, in der Regel nach Abschluss der Phase 2.

Die optische Messeinheit 500 fährt das lineare Küvettenarray 200 periodisch ab und generiert beim Vorbeifahren ("on the fly") am Eintritts- 202 bzw. Austrittsfenster 203 der Küvette 201, - sofern vom Messprotokoll zum jeweiligen Zeitpunkt des Vorbeifahrens vorgesehen - einen Messwert. Alternativ dazu kann die optische Messeinheit 500 beim Vorbeifahren auch kurz anhalten und während des Anhaltens messen, um einen genaueren Messwert zu erhalten.

Während die chemische Reaktion in der Küvette 201 zwischen Probe und Reagenz abläuft, können in definierten Zeitabständen Messpunkte generiert werden. Abhängig vom jeweiligen Analyseprotokoll werden singuläre oder - bei kinetischen Messungen - zeitabhängige Messwerte bei einer oder mehreren Wellenlängen gewonnenen, und mit vorbekannten, der jeweiligen Analyse zugeordneten Referenz- und Kalibrierwerten, zu einem Konzentrationswert des Analyten verrechnet und angezeigt.

Abhängig von der Art der jeweiligen Analyse und Probe kann sich der Messvorgang - insbesondere bei kinetischen Messungen - über sehr unterschiedliche Zeiträume von wenigen Sekunden bis in den zweistelligen Minutenbereich erstrecken.

Unmittelbar nach dem Abschluss der photometrischen Messung wird die Küvette 201 zum Waschen mit der Küvettenwascheinheit 600 freigegeben. Der Waschprozess mittels Küvettenwascheinheit 600 erfolgt umgehend nach Freigabe der Küvette, vorzugsweise zusammen mit mehreren benachbarten, ebenfalls zum Waschen freigegebenen Küvetten 201 und nach "frei werden" der verfahrbaren Küvettenwascheinheit 600. Nach dem Waschen und Trocknen wird die Küvette 201 für die nächste Analyse bereitgestellt.

Beispiel: multiple Analysen

Im Vorfeld der Durchführung multipler Analysen wird das Probenlager 920 mit den Proben $P_1$ bis $P_n$ manuell oder automatisch beschickt. Die Art und Anzahl der für jede Probe $P_x$ durchzuführende Analysen $A_1$ bis $A_n$ wird in die Steuerung des Analysators 100 eingegeben. Gegebenenfalls werden die Reagenzienlager 950a, 950b mit den für die durchzuführenden Analysen erforderlichen Reagenzien beschickt oder nachbeschickt.

Für jede durchzuführende Analyse $P_xA_x$ werden die oben beschriebenen Phasen 1 bis 4 durchlaufen, jeweils beginnend mit der Phase 1.

Nachdem der Pipettor 300b in den Phasen 1 und 3 von der durchzuführenden Analyse $P_xA_x$ in Anspruch genommen wird, kann die Phase 1 der nachfolgenden Analysen $P_xA_{x+1}$ bzw. $P_{x+1}A_x$ erst nach Abschluss der Phase 1 und außerhalb der Phase 2 der laufenden Analysen beginnen, und zwar für so viele nachfolgende Analysen wie es "freie", d.h. nicht von anderen Analyseprozessen beanspruchte Küvetten, gibt.

Das erfindungsgemäße Konzept ermöglicht es - im Gegensatz zu den eingangs beschriebenen Systemen - dass nach abgeschlossener Messung eine Küvette umgehend gewaschen und für einen neuen Test bereitgestellt werden kann, ohne dass dadurch die Abläufe der noch laufenden Analyseprozesse nachteilig gestört werden.

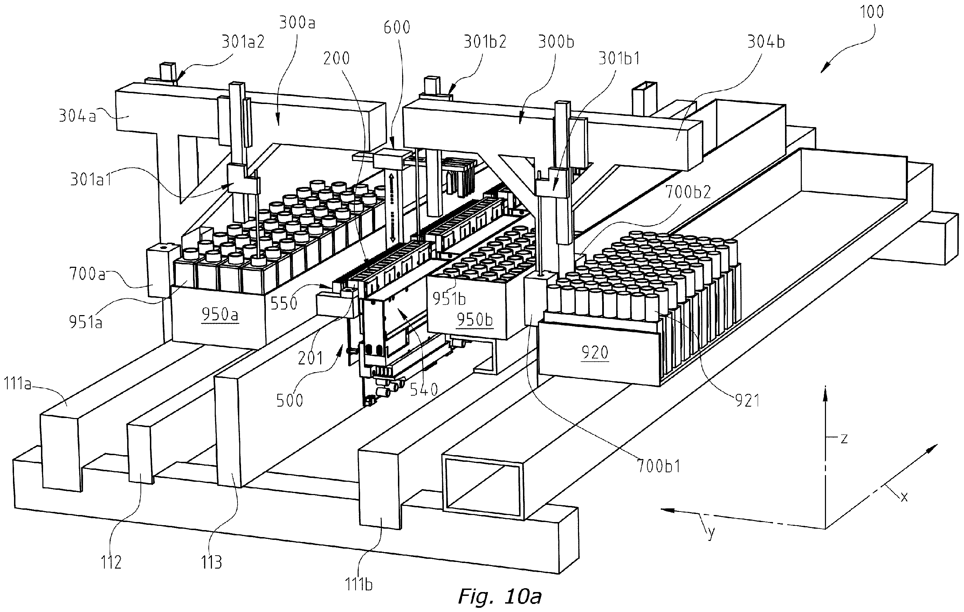

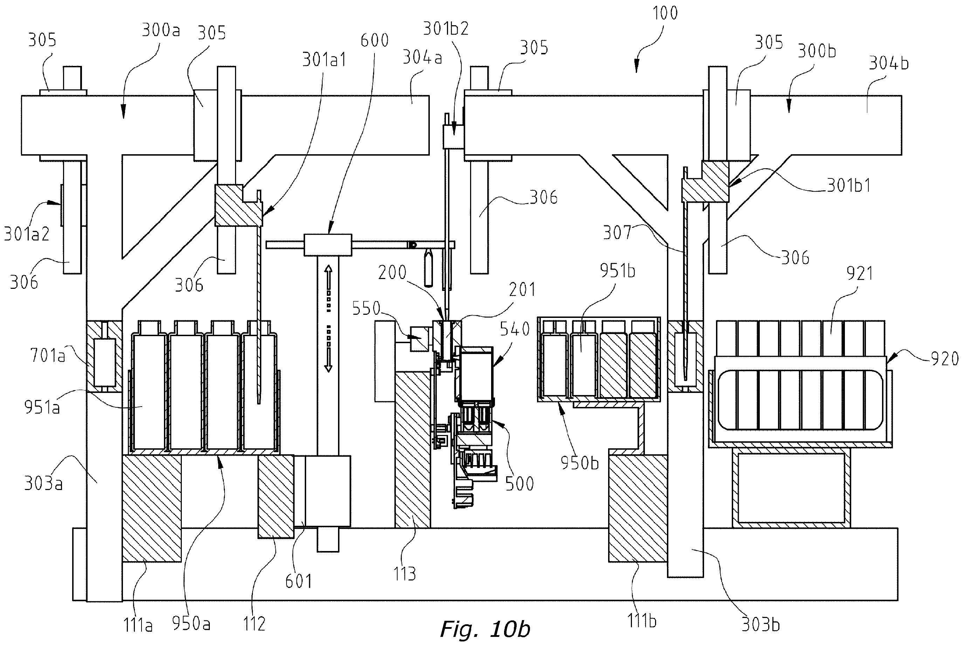

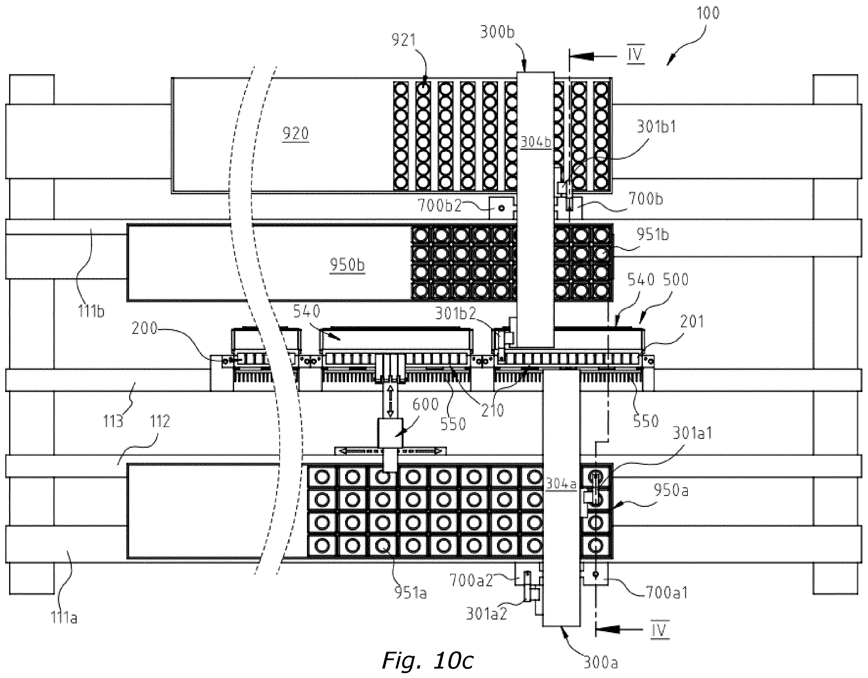

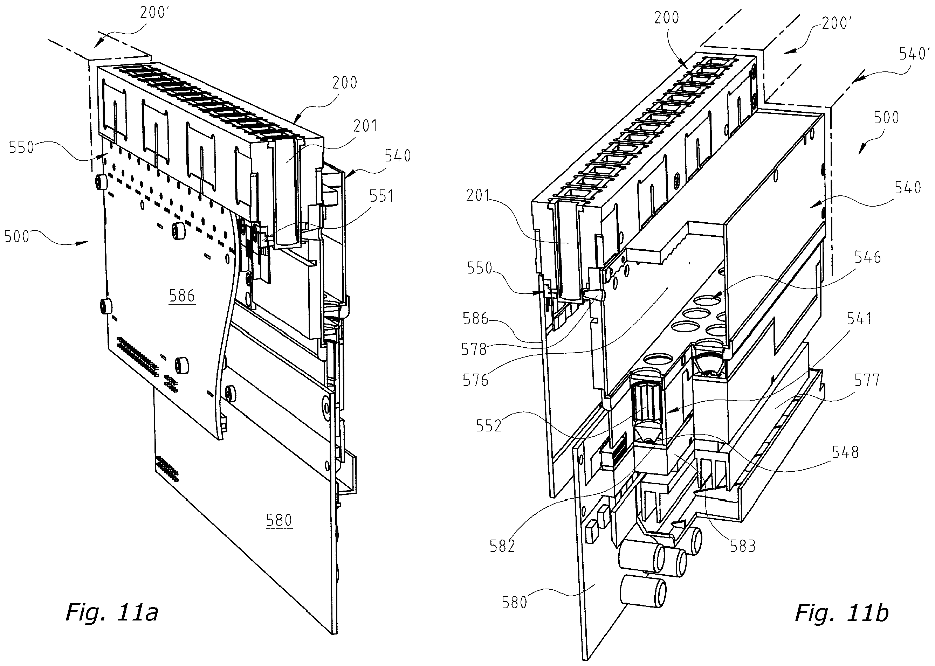

Die in den Fig. 10a bis 10c beschriebene, zweite Ausführungsvariante des automatischen Analysators 100 weist die schon im Zusammenhang mit der ersten Variante ausführlich erläuterten Komponenten, wie entlang des stationären Küvettenarrays 200 verfahrbare Pipettoren 300a, 300b, bevorzugt mit den Pipettoren 300a, 300b mitfahrende Nadelwascheinheiten 700a1 bis 700b2, sowie eine entlang des Küvettenarrays 200 verfahrbare Küvettenwascheinheit 600 auf und unterscheidet sich vor allem in der optischen Messeinheit 500, die gemäß einer ersten Variante (siehe Fig. 11a bis 11f) stationär ausgebildet und den einzelnen Küvetten 201 fix zugeordnet ist.

Die optische Messeinheit 500 weist folgende Grundelemente auf:

eine Lichtbereitstellungseinheit 540 zur Abgabe einer Eintrittsstrahlung in die Küvetten 201 des Küvettenarrays 200, wobei die Lichtbereitstellungseinheit 540 mehrere im UV/VIS/NIR-Wellenlängenbereich spektral unterschiedlich emittierende LED-Lichtquellen 541 aufweist, sowie

eine Detektionseinheit 550 zur Erfassung einer aus den Küvetten 201 des Küvettenarrays 200 austretenden Messstrahlung und Umwandlung der Messstrahlung in ein elektrisches Messsignal, wobei die Detektionseinheit 550 so ausgelegt ist, dass jeder Küvette 201 des Küvettenarrays 200 zumindest eine Fotodiode 551 fix und stationär zugeordnet ist.

Die in den Fig. 11a bis 11f dargestellte, erste Variante der erfindungsgemäßen, optischen Messeinheit 500 weist zumindest eine stationäre Lichtverteilereinrichtung 542 auf, die das Licht der einzelnen LED-Lichtquellen 541 auf die einzelnen Küvetten 201 des stationären Küvettenarrays 200 verteilt.

Die Lichtverteilereinrichtung 542 weist einen von Wänden gebildeten Hohlraum auf, dessen innere Flächen 543, 544, 545, sowie die Rückwand und die beiden Stirnflächen zumindest teilweise verspiegelt und/oder diffus reflektierend ausgeführt sind. Die Lichtverteilereinrichtung 542 weist für jede LED-Lichtquelle 541 in der Bodenfläche 545 eine Eintrittsöffnung 546 zur Einspeisung des Lichts in den Hohlraum auf und verfügt für jede Küvette 201 des Küvettenarrays 200 über eine Austrittsöffnung 547 zur Einspeisung des Lichts in die Küvette 201.

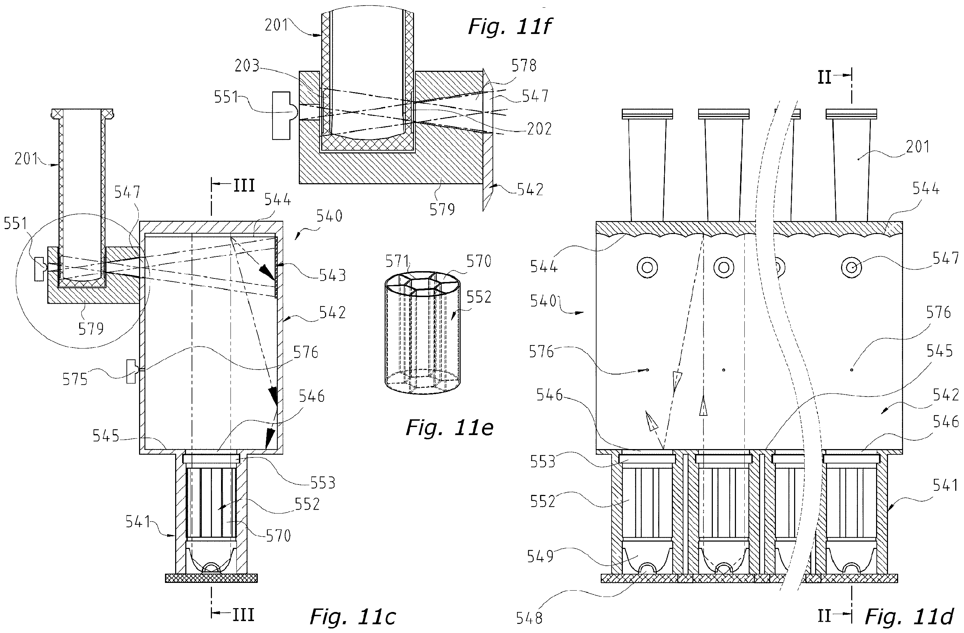

Erfindungsgemäß ist die den Eintrittsöffnungen 546 der LED-Lichtquellen 541 gegenüberliegende, innere Fläche 544 an der Deckfläche der Lichtverteilereinrichtung 542 gewellt und reflektierend ausgeführt, wobei die Wellen der gewellten Innenfläche 544 bevorzugt normal zur Längserstreckung der Lichtverteilereinrichtung 542 ausgerichtet sind, um das von den einzelnen LED-Lichtquellen 541 eintretende Licht in Längsrichtung der Lichtverteilereinrichtung 542 optimal zu verteilen (siehe Fig. 11d).

Um eine möglichst homogene Beaufschlagung der Küvetten 201 mit der Messstrahlung zu gewährleisten, ist die den Austrittsöffnungen 547 zu den Küvetten 201 gegenüberliegende, innere Fläche 543 der Lichtverteilereinrichtung 542 am oberen Teil diffus reflektierend ausgeführt (siehe Fig. 11c). Als Material zur Beschichtung der inneren Fläche 543 im Sichtbereich ausgehend vom Eintrittsfenster 202 der Küvette 201 eignet sich beispielsweise Bariumsulfat ($BaSO_4$).

Zumindest einzelne LED-Lichtquellen 541 der Lichtbereitstellungseinheit 540 zur Verbesserung der spektralen Charakteristik und zur Einspeisung des Lichts in die Lichtverteilereinrichtung 542 weisen optische Elemente zur Kollimation und ausgangsseitig ein schmalbandiges Filter auf.

Wie in Fig. 11a und im Detail in Fig. 11c dargestellt, kann die LED-Lichtquelle 541 eine LED 548, angeordnet in einer TIR-Linse 549, einen Röhrenkörper 552 zur Eliminierung nicht-paralleler Strahlanteile der LED, sowie eintrittsseitig in die Lichtverteilereinrichtung 542 ein schmalbandiges Filter, vorzugsweise ein Interferenzfilter 553, aufweisen.

Dabei kann der Röhrenkörper 552 parallel zur Längsachse der LED-Lichtquelle 541 verlaufende, längliche Durchgangsöffnungen 570 aufweisen, deren Wände 571 aus einem Licht absorbierenden Material bestehen oder mit einem derartigen Material beschichtet sind (siehe Detaildarstellung gemäß Fig. 11e). Es gelangen somit - innerhalb einer gewissen Toleranz - nur parallel ausgerichtete Strahlen auf das Interferenzfilter 553, da abweichende Strahlen vom Röhrenkörper 552 absorbiert werden.

Die Lichtführung bzw. Lichtlenkung in der optischen Messeinheit erfolgt in mehreren Schritten um den Anforderungen gerecht zu werden:

• Im ersten Schritt wird das räumlich breit abgestrahlte Licht der LEDs 548 mit Hilfe von optischen Linsen, TIR-Linsen 549 oder Parabolspiegeln gesammelt, parallelisiert und in Richtung des Innenraums der Lichtverteilereinrichtung 542 gelenkt.

• Im (optionalen) zweiten Schritt wird mit Hilfe des Röhrenkörpers 552 oder anderer röhrenartiger Bauelemente das weitere Fortschreiten nicht ausreichend parallelisierter Anteile des Lichtes verhindert.

• Im dritten Schritt sind optische Bandpassfilter, beispielsweise Interferenzfilter 553 vorgesehen, um ein vorgegebenes, schmalbandiges Lichtspektrum zu erhalten.

• Im vierten Schritt erfolgt im Innenraum der Lichtverteilereinrichtung 542 die möglichst homogene Verteilung und Lenkung des durch die einzelnen LED-Lichtquellen 541 erzeugten Lichts in die einzelnen Küvetten 201. Dafür ist die im Wesentlichen quaderförmige Lichtverteilereinrichtung 542 derart ausgestaltet, dass die Deckelfläche eine gewellte Struktur 544 aufweist (siehe Fig. 11d) und die übrigen Innenflächen eben und spiegelnd bzw. diffus reflektierend ausgeführt sind, sodass Licht über einen spektralen Bereich von ca. 340 bis 800nm möglichst effektiv reflektiert wird. Den Austrittsöffnungen 547 gegenüberliegend ist eine diffus reflektierende Fläche 543 angeordnet, alle anderen Innenflächen der Lichtverteilereinrichtung 542 weisen spiegelnde und/oder diffus reflektierende Oberflächen auf. In der Rückwand der Lichtverteilereinrichtung 542 sind die Austrittsöffnungen 547 angeordnet, durch die das Licht direkt zu den Eintrittsfenstern 202 der Küvetten 201 gelangen kann.

• Im fünften Schritt wird durch eine Durchführung 578, ggf. unter Zwischenschaltung einer oder mehrerer Blenden zwischen der Lichtverteilereinrichtung 542 und der Küvette 201 ein in das Innere der Küvette 201 gerichtetes Strahlenbündel erzeugt.

• Im sechsten Schritt wird die Messstrahlung vom Austrittsfenster 203 der Küvette 201 ggf. unter Zwischenschaltung einer Blende zur Fotodiode 551 der Detektionseinheit 550 gelenkt.

Erfindungsgemäß sind an der Lichtverteilereinrichtung 542 ausgangsseitig von in einer Wand, beispielsweise der Rückwand, der Lichtverteilereinrichtung 542 angeordneten Durchgangsöffnungen oder Lochblenden 576 Monitoroder Referenzdetektoren 575 angeordnet, mit welchen Schwankungen der Messstrahlung jederzeit erfasst werden können. Es kann jeder Küvette 201 eine Lochblende 576 samt Referenzdetektor 575 zugeordnet sein. Falls jeder Küvette 201 eine Referenzfotodiode zugeordnet ist, befinden sich diese vorzugsweise an den Austrittsöffnungen 547 der Lichtverteilereinrichtung 542. Es ist auch möglich in der Lichtverteilereinrichtung 542 nur zwei oder drei Lochblenden 576 samt Referenzdetektoren 575 vorzusehen (siehe Fig. 11a).

Wie in den Fig. 11a/b dargestellt, kann das stationäre Küvettenarray 200 segmentiert bzw. in mehrere Abschnitte unterteilt sein, wobei jedem Segment 210 eine separate Lichtbereitstellungseinheit 540 fix zugeordnet ist.

Jedem Segment 210 ist eine gemeinsame, über die gesamte Länge des Segments verlaufende Lichtverteilereinrichtung 542 zugeordnet, welche über eine hinreichende Anzahl von Einbaupositionen für LED-Lichtquellen 541 für bis zu 16 optische Kanäle mit Licht unterschiedlicher Wellenlänge ($\lambda$1 bis $\lambda$16) verfügt. Die einzelnen LEDs der LED-Lichtquellen 541 können bevorzugt in Form eines LED-Arrays auf einer gemeinsamen Leiterplatte 582, beispielsweise aus Aluminium, angeordnet sein. Benachbarte Einbaupositionen (siehe Fig. 11a) können zur Erhöhung der Intensität mit LED-Lichtquellen gleicher Wellenläge bestückt sein. Im Bereich des vorderen, der Lichtverteilereinrichtung 542 benachbarten Eintrittsfensters 202 jeder Küvette 201 besitzt die Lichtverteilereinrichtung 542 eine kreisrunde Öffnung, die sogenannte Austrittsöffnung 547, durch welche das von den LEDs erzeugte Licht durch das Eintrittsfenster 202 in das Innere der Küvette 201 eingestrahlt wird. Die Durchführung 578 in der Küvettenaufnahme 579, zwischen der Austrittsöffnung 547 und dem Eintrittsfenster 202 in die Küvette 201 kann kanalförmig ausgeführt sein, ggf. Blenden beinhalten und bevorzugt aus einem Licht absorbierenden Material bestehen (siehe Fig. 11f).

Durch die Verteilung des Lichts innerhalb der Lichtverteilereinrichtung 542 durch multiple Streuungen und Reflexionen an den Innenwänden gelangt das Licht eines jeden optischen Kanals der LED-Lichtquellen 541 durch die kreisrunden Austrittsöffnungen 547 in das Eintrittsfenster 202 einer jeden, zugeordneten Küvette 201.

Die Messung der Intensität **I** des durch die Küvetten 201 transmittierten Lichts erfolgt mittels eines stationären Arrays von Fotodioden 551 (zumindest eine Fotodiode pro Küvette), welche jeweils fix hinter dem hinteren, von der Lichtverteilereinrichtung 542 abgewandten Austrittsfenster 203 der Küvetten 201 platziert sind.

Optional kann an jeder Küvette 201 eine zweite Fotodiode (nicht dargestellt) in einem vom durchgehenden Strahlengang um beispielsweise 90° verdrehten Winkel zur Durchführung nephelometrischer Streulichtmessungen angeordnet sein.

Zwecks Gewährleistung einer konstanten Umgebungstemperatur der LED-Lichtquellen 541 wird ein massiver Alu-Block 583 beispielsweise mit Hilfe von Peltier-Bauteilen temperiert (Kühl- und Heizmöglichkeit) an der Leiterplatte 582 der LED-Lichtquellen 541 angebracht.

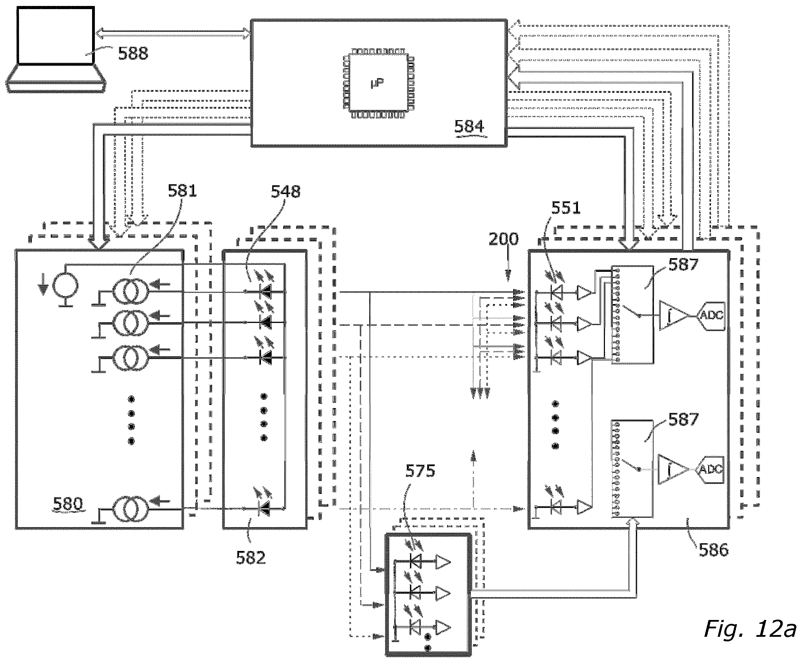

Die in Fig. 12a schematisch dargestellte Elektronik für die optische Messeinheit 500 besteht aus mehreren Schaltungseinheiten, die auf mehreren Leiterplatten verteilt angeordnet und entsprechend deren Funktion am stationären Küvettenarray 200 (siehe Pfeil) geometrisch platziert sind.

Die Leiterplatte der Sendeeinheit 580 enthält im dargestellten Beispiel 16 parallel aufgebaute Stromquellen 581, die jeweils einer bestimmten Lichtquelle (LED 548) mit einer bestimmten Wellenlänge zugeordnet sind. Die Stromquellen 581 können von einem Optik-Kontroller (584) in der Stromstärke und in der Pulslänge geregelt werden, sodass ein gewünschter Stromimpuls in Länge und Stärke für den Lichtimpuls eingestellt werden kann. Auch die LED-Versorgungsspannung kann für jeden LED-Kanal individuell geregelt werden. Die Platine der Sendeeinheit 580 wird zwecks Thermostatisierung mit einem Alu-Block 583 samt Kühlrippen 577 (siehe Fig. 11a) verschraubt und mittels Peltier Elementen auf eine einstellbare Temperatur, beispielsweise zwischen 29°C und 41°C, geregelt. Die thermische Drift der Stromquellen 581 kann dadurch auf ein Minimum reduziert werden. Die in den Stromquellen 581 anfallende Verlustleistung wird durch die zeitlich aufeinanderfolgende Ansteuerung vergleichmäßigt. Es wird immer nur eine Stromquelle 581 pro Zeiteinheit aktiviert, somit wird auch immer nur Licht mit einer bestimmten, vorgegebenen Wellenlänge erzeugt.

Die eigentlichen Lichtquellen werden auf einer separaten, gekühlten Alu-Leiterplatte 582 mittels 16 selektierten LEDs 548 mit den gewünschten 16 Wellenlängen realisiert. Die Alu-Leiterplatte 582 wird wegen der besseren thermischen Ankopplung der LEDs verwendet, mit dem Alu-Block 583 verschraubt und somit auch auf konstanter Temperatur (z.B. +37°C) betrieben. Die LEDs haben trotz unterschiedlicher Pulslängen konstante mittlere Temperatur und erzeugen damit einen geringen spektralen Shift.

Die Alu-Leiterplatte bzw. Platine 582 mit den LEDs ist direkt an der Lichtverteilereinrichtung 542 (siehe Fig. 11a) angeordnet, um bestmögliche Lichteinkopplung in die Lichtverteilereinrichtung 542 zu garantieren. Das Licht der LEDs 548 wird über TIR Linsen 549 und Röhrenkörper 552 zunächst parallel ausgerichtet, dann über optische Filter 553 spektral gefiltert und anschließend im Inneren der Lichtverteilereinrichtung 542 soweit gleichmäßig diffus verteilt, sodass das Licht auf 16 nebeneinanderliegenden Austrittsöffnungen 547 zu den 16 Küvetten 201 des stationären Küvettenarrays (siehe Pfeil 200 in Fig. 12a) ausgekoppelt werden kann.

Eine weitere Leiterplatte 585 ist mit bis zu 16 Monitor- oder Referenzfotodioden 575 ausgestattet, die das von den LEDs 548 erzeugte Licht vor der Passage der jeweiligen Küvette erfassen. Es können aber auch nur zwei globale Monitor bzw. Referenzfotodioden 575 zum Einsatz kommen. In diesem Fall wird das Licht nicht direkt vor jeder Küvette sondern an mehreren Stellen der Lichtverteilereinrichtung 542 gemessen. Aufgrund der konstanten geometrischen Verhältnisse kann das Licht vor jeder Küvette mit Hilfe eines Geometriefaktors umgerechnet werden.

Ausgangsseitig der Küvetten des Küvettenarrays 200 befindet sich die Leiterplatte 586 der Detektoreinheit 550. Diese Leiterplatte enthält 16 Fotodioden 551 für das aus den Küvetten 201 austretende Durchlicht. Die Detektoreinheit verarbeitet pro Küvette zwei Analogwerte der zwei zugeordneten Fotodioden 551, 575 von Durchlicht und Monitor- bzw. Referenzlicht. Für die Streulichtmessung (Nephelometrie) kann von jeder Küvette durch eine seitlich angeordnete Fotodiode ein dritter Analogwert erfasst werden, dessen Signalpfad jedoch aus Gründen der Übersichtlichkeit in Fig. 12a nicht weiter dargestellt ist.

Die zwei Signalpfade ausgehend von den Fotodioden 551, 575 werden durch zwei 16:1 Multiplexer 587, Inverter, Integratoren und ADCs zeitsynchron verarbeitet und in einen digitalen Messwert gewandelt. Die Multiplexer 587 ermöglichen es die beispielsweise 16 Küvettenkanäle auszuwählen und zeitlich nacheinander in konfigurierbarer Reihenfolge umzuschalten.

Falls das stationäre Küvettenarray 200 segmentiert ist, und jedem Segment 210 eine separate Lichtverteilereinrichtung 542 fix zugeordnet ist (siehe Fig. 11a/b) werden strichliert angedeutete, zusätzliche Leiterplatten bei der Sendeeinheit 580, der Leiterplatte für die LEDs 582, der Leiterplatte für die Monitor- bzw. Referenzdioden 575 und ggf. der Leiterplatte für die Detektoreinheit 586 eingesetzt. Beispielsweise können bei einer Anordnung von 96 Küvetten 201 im stationären Küvettenarray 200 sechs separate Lichtverteilereinrichtungen 540 mit jeweils 16 Austrittsöffnungen zu den fix zugeordneten Küvetten 201 vorgesehen sein.

Die zentrale Leiterplatte 584 für die optische Messeinheit 500 ist mit dem Optik-Kontroller bestückt. Die optische Steuereinheit wird durch eine programmierbare Logik (FPGA) als Statemachine realisiert und kann zeitgleich die Sendeeinheit 580 und die Detektoreinheit 586 bedienen. Für die Erzeugung des korrekten Zeitablaufs werden die einzelnen Lichtmessungen in Licht- und Dunkelmessungen zerlegt und können in einem Konfigurationsspeicher zeilenweise unterschiedlich parametriert werden. Die Statemachine arbeitet diese Konfigurationszeilen der Reihe nach ab, wobei auch Zeilen übersprungen werden können. Die Unterscheidung für Licht- und Dunkelmessung wird durch ein Flag in der Konfigurationszeile definiert, ebenso wie der gewünschte Küvettenkanal und die Lichtquelle. Des Weiteren sind in der Konfigurationszeile die gewünschten Delay Einstellungen, Stromstärke und Pulslänge enthalten, weiters die Auswahl der Referenz-Fotodiode, der LED-Versorgungsspannung, die Oversampling- und Averaging-Vorgabe sowie die Periodendauer.

Die Detektoreinheit 586 wird synchronisiert zur Sendeeinheit 580 angesteuert und kann durch globale Parameter mit Mittelung oder Oversampling Einstellungen gesetzt werden. Des Weiteren wird aus der Konfigurationszeile die gewünschte Integrationszeit ausgelesen, mit der das Lichtsignal integriert werden soll. Ebenso können hier mittels globaler Parameter die Delayzeit für den Integrator und die Integrationssteilheit gewählt werden, sodass man damit die Einschwingzeiten des Messsignals und die Integrationsgeschwindigkeit umschalten kann.

Der analoge Messwert wird somit aus der entsprechenden Fotodiode 551 mit Transimpedanzwandler über den Multiplexer 587 selektiert und mittels Inverter und Integrator und optionalem logarithmischen Verstärker gemessen und mit einem hochauflösenden ADC Messungen mit bzw. ohne Oversampling digitalisiert. Letztlich werden - falls auch eine Streulichtmessung erfolgt - drei Analogmesswerte (Durchlicht, Monitor- bzw. Referenzlicht, Streulicht) zeitgleich mit drei ADCs digitalisiert und als Rohmesswerte im internen Speicher zeilenweise abgelegt. Wesentlich ist, dass die Messung von Durchlicht und Monitor- bzw. Referenzlicht sowie ggf. Streulicht zeitgleich erfolgt.

Der interne Speicher enthält alle Rohdaten und wird vom Auswerteprozessor mittels Software zyklisch ausgelesen und durch einen Umrechnungsalgorithmus in einen endgültigen Messwert umgerechnet. Die Umrechnung berücksichtigt Dunkelwert und Lichtwert und auch die $I_0$ Messung und $I_1$ Messung vor und nach Hinzumischen der Reagenzien. Auch die zeitliche Veränderung der Messwerte kann durch aufeinanderfolgende Messungen erfasst werden. Wesentlich ist, dass die Messungen periodisch erfolgen und entsprechend der eingestellten Periodendauer einen wiederholbaren Messzyklus ergeben.

Die berechneten Daten werden pro Küvette in definierte Datenpakete verpackt und mittels lokaler Ethernet Schnittstelle an den Hauptrechner 588 übermittelt. Durch diese Datenreduktion ist es möglich alle Küvetten des Küvettenarrays 200 der optischen Messeinheit 500 zu bearbeiten und an den Hauptrechner 588 zu übergeben.

Im Messverfahren ist die Messung von $I$ bzw. $I_0$ in rascher Abfolge für jede Küvette mit einer hohen Abtastfrequenz (>1Hz) möglich. Dabei gibt es verschiedene Möglichkeiten die multiplen LED-Lichtquellen 541 und Fotodioden 551 der Detektionseinheit 500 anzusteuern bzw. auszulesen.

Das periodische Ansteuerungssignal der einzelnen LED-Lichtquellen 541 wird bezüglich Puls- und Integrationsdauer sowie der verwendeten Stromhöhe für jede Kombination aus Küvette und Wellenlänge für den verwendeten Messmodus festgelegt und während des Betriebs nicht verändert.

Im dargelegten Beispiel erfolgt die Ansteuerung von 16 LED-Lichtquellen 541 über 16 separate Stromquellen 581 und deren Umgebungshardware. Die Belichtung jeder Küvette mit jedem spektralen Kanal der LED-Lichtquellen 581 sowie die dabei verwendeten Integrationszeiten werden einzeln definiert (16 x 16 Kombinationen). Die einzelnen LEDs emittieren (bzw. in einzelnen Positionen zur Erhöhung der Intensität auch mehrere LEDs) im Zuge eines Messzyklus in sequentieller Reihenfolge jeweils einen Lichtpuls, der im Inneren der Lichtverteilereinrichtung 542 an den Innenwänden mehrfach reflektiert wird und schließlich durch die 16 Austrittsöffnungen 547 zu den 16 zugeordneten Küvetten 201 gelangt (siehe Fig. 11c).

Es sind verschiedene Messmodi vorgesehen:

Modus 1: Detektion des dynamischen LED-Blitzsignals mit konstanter Integrationszeit und variabler Stromstärke sowie Pulsdauer (256 Blitze)

Modus 2: Detektion des statischen LED-Signals mit variabler Integrationszeit (256 LED-Ansteuerungen) und variabler Stromstärke

Modus 3: Detektion des statischen LED-Signals mit variabler Integrationszeit (16 LED- Ansteuerungen)

Die Messung erfolgt für jede Kombination aus Küvette und Wellenlänge einzeln, wobei bei den Modi 1 und 2 für jeden Messpunkt ein Lichtpuls erzeugt wird.

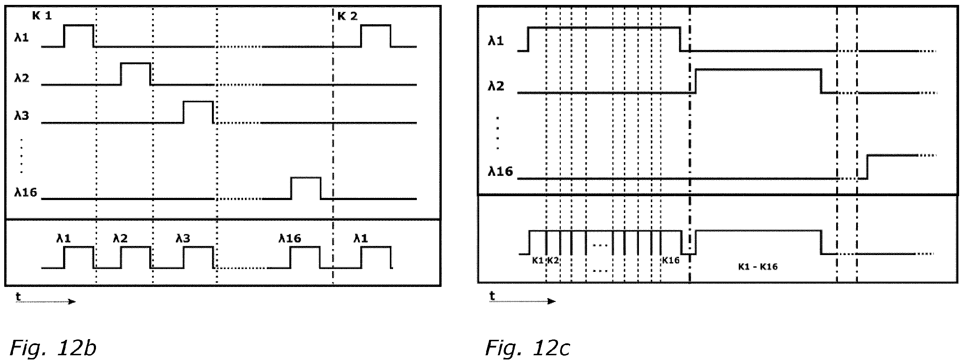

Wie in Fig. 12b dargestellt, werden in den Modi 1 und 2 die spektralen Kanäle ($\lambda$1 ... $\lambda$16) der einzelnen LED-Lichtquellen 581 in fester Reihenfolge aktiviert und deaktiviert. Die resultierenden Lichtblitze werden von der durch den Multiplexer 587 angewählten Fotodiode 551 detektiert und vermessen. Nach dem Durchlauf aller spektralen Kanäle wird auf die Sensorik von der Küvettenposition K1 auf die Küvettenposition K2 umgeschaltet und die hierfür benötigten Lichtblitze in derselben Reihenfolge erzeugt. Nach einem kompletten Durchlauf aller 16 Küvettenpositionen (also 16 x 16 Lichtblitzen) ist ein Sampling abgeschlossen und das nächste kann initiiert werden. Durch diesen Ablauf können bis zu vier Samplings pro Sekunde realisiert werden. In den Modi 1 und 2 werden abwechselnd Dunkel- und Lichtmessungen hintereinander ausgeführt, sodass in Summe 512 Einzelmessungen pro Sampling durchgeführt werden.

Das Messverfahren gemäß Modi 1 und 2 zeichnet sich somit dadurch aus, dass die spektralen Kanäle $\lambda$1 ... An der einzelnen LED-Lichtquellen 581 in einer vorgegebenen Reihenfolge aktiviert und deaktiviert werden, wobei jeweils die in einer ersten Küvettenposition K1 angeordnete Fotodiode 551 detektiert wird, sowie dass nach dem Durchlauf aller spektralen Kanäle in der ersten Küvettenposition K1 auf die nächste Küvettenposition K2 umgeschaltet wird. Die Zeitdauer für einen Zyklus in Messmodus 1oder 2 beträgt >= 0,25 Sekunden.

Im Messmodus 3, schematisch dargestellt in Fig. 12c, werden die LED-Lichtquellen 541 in anderer Reihenfolge als im Modus 1 bzw. 2 geschaltet.

Jede LED-Lichtquelle 541 bzw. jeder spektrale Kanal wird im Zyklus (angedeutet durch die strichpunktierte Linie) nur jeweils einmal eingeschaltet und danach alle 16 Küvetten hintereinander gemessen, wobei zwischen diesen Einzelmessungen keine Dunkelmessung erfolgt. Die erste Küvette K1 wird mit einem Delay vermessen, sodass die zugeordneten Fotodioden 551 der Detektoreinheit 550 genug Zeit zum Einschwingen haben. Die weiteren Küvetten K2 bis K16 können ohne zusätzliche Einschwingzeit schneller hintereinander gemessen werden.

Innerhalb eines Zyklus wird jede LED nur einmal eingeschaltet, wobei jeweils alle 16 Küvetten vermessen werden. Falls eine Dunkelmessung erforderlich ist, wird einmal, beispielsweise am Anfang oder Ende des Zyklus für die Vermessung der 16 Küvetten ein Dunkelwert gemessen.

Bei 16 Wellenlängen bzw. 16 spektralen Kanälen ($\lambda$1 ... $\lambda$16) und 16 Küvettenpositionen benötigt man 16 x 16 Lichtmessungen. Addiert man die 16 Dunkelmessungen (einmal pro Zyklus) ergibt das 272 Einzelmessungen. Die Zeitdauer für einen Zyklus in Messmodus 3 beträgt >= 0,5 Sekunden.

Das Messverfahren gemäß Modus 3 zeichnet sich somit dadurch aus, dass der spektrale Kanal $\lambda$1 der ersten LED-Lichtquellen 581 aktiviert wird, wobei in einer vorgegebenen Reihenfolge die in den Küvettenpositionen K1 ... Km angeordneten Fotodioden 551 detektiert werden, wobei nach dem Durchlauf aller Küvettenpositionen K1 ... Km der nächste spektrale Kanal $\lambda$2 der nächsten LED-Lichtquellen 581 aktiviert wird.

Vorteil von Modus 3:

- Modus 3 ist in Summe schneller als die 512 abwechselnd ausgeführten Dunkel/Lichtmessungen von Modus 1 und Modus 2, weil insgesamt weniger Messungen und weniger Einschwingzeiten für die Fotodioden notwendig sind.

- Die Einschwingzeit der Fotodioden muss nur vor der ersten Lichtmessung der Küvette K1 berücksichtigt werden, die restlichen 15 Küvetten K2 bis K16 können unmittelbar darauf folgen.

- Insgesamt kommt man daher auf deutlich kürzere Abtastzeiten pro Zyklus gegenüber Modus 1 oder 2.

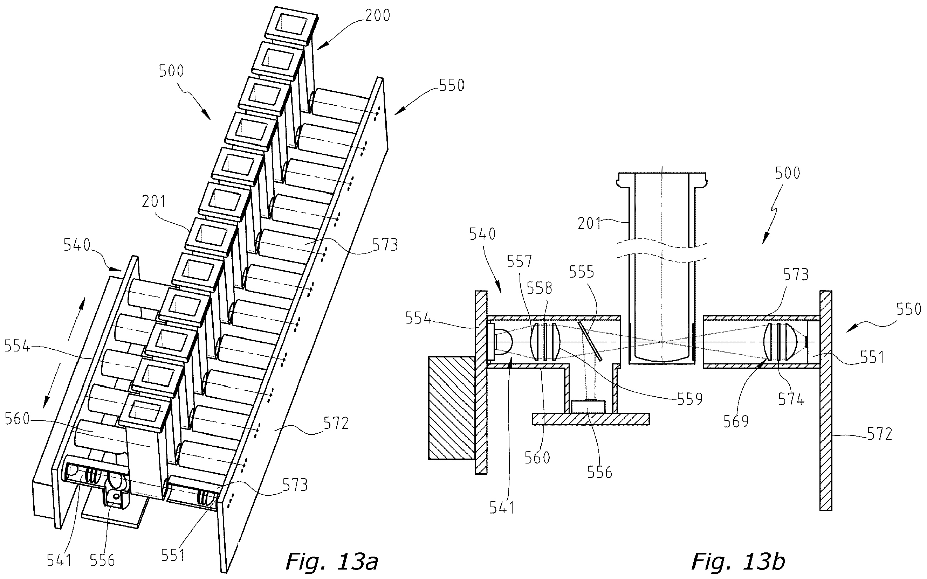

Bei der in den Fig. 13a und 13b dargestellten, zweiten Variante der erfindungsgemäßen, optischen Messeinheit 500 weist die Lichtbereitstellungseinheit 540 zumindest ein eindimensionales, stabförmiges Lichtquellenarray 554 mit mehreren LED-Lichtquellen 541 auf, das entlang des stationären Küvettenarrays 200, beispielsweise einer Analyseneinrichtung, ausgerichtet ist und entlang des stationären Küvettenarrays 200 verfahrbar ausgeführt ist. Jeder Küvette 201 des stationären Küvettenarrays 200 kann somit jede LED-Lichtquelle 541 des Lichtquellenarrays 554 zugeordnet werden.

Bei dieser Ausführungsvariante ist bevorzugt jeweils eine LED-Lichtquelle 541 zusammen mit einem Strahlteiler 555 und einem Referenzdetektor 556 in einem gemeinsamen, beispielsweise rohrförmigen, Gehäuse 560 angeordnet. Die Lichtwege der einzelnen, nebeneinander angeordneten LED-Lichtquelle 541 können dadurch separiert werden.

Einzelne LED-Lichtquellen 541 des stabförmigen Lichtquellenarrays 554 können zur Einspeisung des Lichts in die Küvetten 201 optische Elemente 557 zur Kollimation und zur Verbesserung der spektralen Charakteristik des Lichts ein schmalbandiges Filter 558 aufweisen. Weiterhin kann ein Kondensor, vorzugsweise eine Sammellinse 559, zur Bündelung des Lichts in die Küvette 201 vorgesehen sein.

Falls einzelne LED-Lichtquellen 541 als schmalbandig emittierende und parallel ausgerichtetes Licht abgebende Laserdioden ausgebildet sind, können die optischen Elemente 557 zur Kollimation, zur Filterung 558 und zur Bündelung 559 zur Gänze oder zumindest teilweise entfallen.

Die den einzelnen Küvetten 201 des stationären Küvettenarrays 200 fix zugeordneten Fotodioden 551 der Detektionseinheit 550 sind bevorzugt als Fotodiodenarray auf einer gemeinsamen Platine 572 angeordnet. Die Detektionseinheit 550 weist dabei - ausgehend von jeder Küvette 201 des stationären Küvettenarrays 200 eine beispielsweise rohrförmige Aufnahme 573 auf, in welcher - falls erforderlich - optische Elemente 569 zur Bündelung der Messstrahlung auf die Fotodiode 551 und - falls erforderlich - ein Filterelement 574 angeordnet sind.

Mit dieser Modulvariante können verschiedene photometrische und turbidimetrische Messungen an multiplen Küvetten 201 eines fest stehenden linearen Küvettenarrays 200 bei einzelnen und/oder multiplen Wellenlängen im Wellenlängenbereich des ultravioletten und sichtbaren Lichts durchgeführt werden, indem die einzelnen LED-Lichtquellen 541 unterschiedlicher Wellenlängen der Lichtbereitstellungseinheit 540 nacheinander vor den einzelnen Küvetten 201 positioniert werden. Danach wird dann jeweils die Intensität des durch die jeweilige Küvette 202 hindurchgetretenen Lichts durch die fix zugeordnete, stationäre Detektoreinheit 550 gemessen. Alternativ zur Positionierung ist auch eine Messung "on the fly" d.h. im Vorbeifahren möglich.

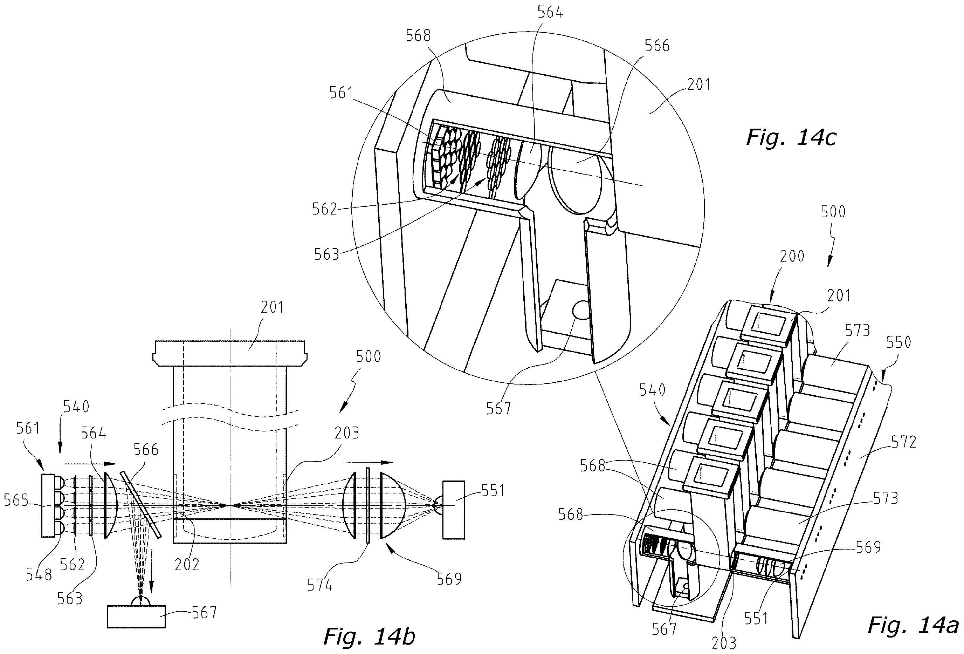

Bei der in den Fig. 14a bis 14c dargestellten, dritten Variante der erfindungsgemäßen, optischen Messeinheit 500 sind die LED-Lichtquellen 541 der Lichtbereitstellungseinheit 540 als 2D-LED-Array 561 angeordnet, wobei jeder Küvette 201 des stationären Küvettenarrays 200 ein stationäres 2D-LED-Array 561 fix zugeordnet ist. Bei dieser Ausführungsvariante findet - ähnlich wie bei der ersten Variante - keine Relativbewegung zwischen den Küvetten 201 des Küvettenarrays 200 einerseits und der Lichtbereitstellungseinheit 540 sowie der Detektionseinheit 550 andererseits statt, wodurch die Messvorgänge durch den Wegfall mechanischer Bewegungen innerhalb der optischen Messeinheit 500 wesentlich beschleunigt werden können.

Gemäß einer Untervariante der dritten Ausführungsvariante können die LED-Lichtquellen 541 in der Lichtbereitstellungseinheit 540 als einzelnes 2D-LED-Array 561 angeordnet sein (wie in der Detaildarstellung gemäß Fig. 14c), wobei die Lichtbereitstellungseinheit 540 entlang des gesamtem stationären Küvettenarrays 200 oder eines Segments 210 des Küvettenarrays 200 verfahrbar ausgeführt ist (ähnlich wie in Fig. 13a dargestellt), derart, dass jeder Küvette 201 des Küvettenarrays 200 oder jedem Segment 210 des Küvettenarrays 200 das 2D-LED-Array 561 zuordenbar ist. Bei einer Segmentierung des Küvettenarrays 200 ist für jedes Segment 210 eine Lichtbereitstellungseinheit 540 mit einem 2D-LED-Array 561 vorgesehen.

Zur Einspeisung des Lichts der einzelnen LEDs 548 des 2D-LED-Arrays 561 in die Küvetten 201 ist ein 2D-Linsenarray 562 zur Kollimation des Lichts der einzelnen LEDs vorgesehen. Weiters ist im Strahlengang zur Verbesserung der spektralen Charakteristik ein 2D-Filterarray 563 zur schmalbandigen Filterung des Lichts angeordnet. Das Filterarray 563 kann in einzelnen Positionen keine Filterfunktion aufweisen, beispielsweise wenn in dieser Position des 2D-LED-Arrays 561 eine schmalbandig und parallel emittierende Laserdiode angeordnet ist.

Weiters ist im Strahlengang zumindest ein Kondensor, vorzugsweise eine Sammellinse 564, zur Bündelung des Lichts in die einzelnen Küvetten 200 vorgesehen.

Besonders bevorzugt sind Ausführungsvarianten, bei welchen das 2D-LED-Array 561 aus auf einem einzigen Substrat 565 gebondeten LED-Emittern besteht, wobei das 2D-Linsenarray 562 ein 2D-Mikrolinsenarray ist und das 2D-Filterarray 563 ein 2D-Mikrointerferenzfilterarray ist.

Jeweils eine LED-Lichtquelle 541, aufweisend ein 2D-LED-Array 561, ein 2D-Linsenarray 562, ein 2D-Filterarray 563 und eine Sammellinse 564 können bevorzugt zusammen mit einem Strahlteiler 566 und einem Referenzdetektor 567 in einem gemeinsamen Gehäuse 568 angeordnet sein.

Bei dieser Variante verfügt jede Küvette 201 über eine individuelle Fotometereinheit bestehend aus einer Lichtbereitstellungseinheit für Licht mit bis zu 9, 12 oder 16 unterschiedlichen Wellenlängen ($\lambda1$ bis $\lambda n$) die durch einzelne LEDs 548 generiert werden. Bei der Verwendung von kommerziellen LEDs (Seitenlänge ca. 2 mm und einem Abstand von ca. 0,5 mm) die mittels Durchsteckmontage auf eine Platine gelötet werden, ist bei einem 4 x 4 Array mit einer Fläche von ca. 10 x 10 mm$^2$ zu rechnen.

Bei der Anordnung der Halbleiter der einzelnen LEDs als COB (Chip on Board) können diese auf einer platzsparenden Fläche von unter 5 x 5 mm$^2$ ausgeführt werden. Bei der COB-Technik werden die LED-Chips vorzugsweise direkt auf eine hoch wärmeleitende Aluminium-Platine gebondet.

Bei einer Kantenlänge von 300 bis 900 $\mu$m und einem Abstand von ca. 100 $\mu$m können beispielsweise 16 LED-Chips auf einer quadratischen Fläche von 1,6 bis 4 mm Kantenlänge untergebracht werden. Entsprechend weisen die einzelnen Kollimatorlinsen des 2D-Mikrolinsenarrays sowie die Interferenzfilter des 2D-Interferenzfilterarrays Durchmesser von bis zu 900 $\mu$m auf. Um die Kollimation (Parallelisierung) weiter zu verbessern kann auf das LED-Array ein Lochblendenarray aufgesetzt sein, so dass die Licht emittierenden Flächen unabhängig von der Größe der emittierenden Halbleiterflächen hinreichend punktförmig dargestellt werden können.

Die LED-Chips können auf dem 2D-Array in Spalten oder Reihen, z.B. 3 x 3, 3 x 4 oder 4 x 4, oder auch in konzentrischen Kreisen angeordnet sein.

Wie bereits im Zusammenhang mit der Variante gemäß Fig. 13a/b beschrieben, weist die Detektionseinheit 550 ausgehend von jeder Küvette 201 des stationären Küvettenarrays 200 eine beispielsweise rohrförmige Aufnahme 573 auf, in welcher optische Elemente 569 zur Bündelung der Messstrahlung auf die Fotodiode 551 und - falls erforderlich - ein Filterelement 574 angeordnet sind.

Die den einzelnen Küvetten 201 fix zugeordneten Fotodioden 551 der Detektionseinheit 550 sind bevorzugt als Fotodiodenarray auf einer gemeinsamen Platine 572 angeordnet.

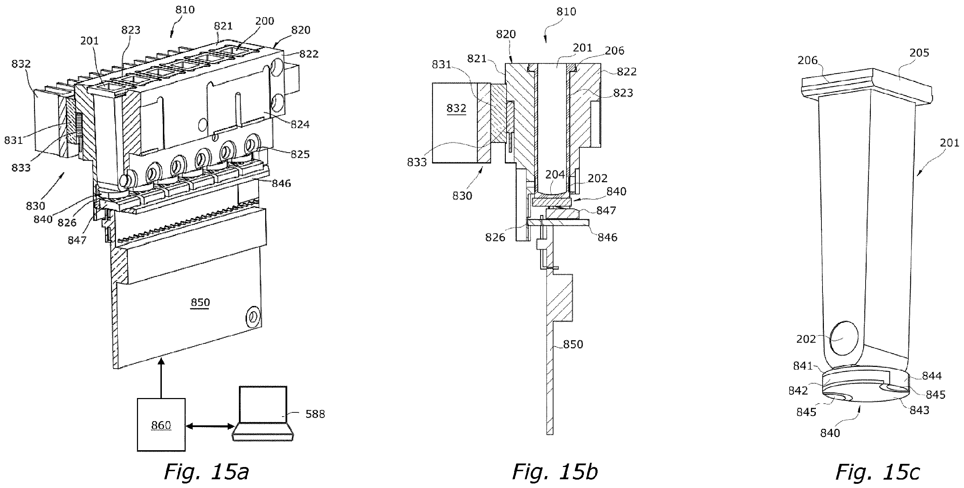

Die in den Fig. 15a bis 15c dargestellte, kombinierte Vorrichtung 810 zum Mischen und Thermostatisieren flüssiger Medien dient dazu, die in den aneinander gereihten Küvetten 201 eines Küvettenarrays 200 eingebrachten flüssigen Medien zu thermostatisieren. Im dargestellten Beispiel handelt es sich um ein lineares, stationäres Küvettenarray 200.

Die einzelnen Küvetten 201 des Küvettenarrays 200 sind in einem thermostatisierbaren Küvettenblock 820, beispielsweise aus Aluminium, angeordnet, wobei die Wände der trichterförmigen Aufnahmen 823 formschlüssig an den Wänden der Küvetten 201 anliegen, um eine optimale Wärmeübertragung zu gewährleisten. Der Küvettenblock 820 besteht aus einem Basisteil 821 mit den Aufnahmen 823 und einem durch eine seitliche Schubbewegung aufklappbaren Vorderteil 822.

Am Küvettenblock 820, beispielsweise am Basisteil 821, ist eine Thermostatisiereinrichtung 830 angeordnet, die eine Kühl- und Heizeinrichtung, beispielsweise in Form eines oder mehrerer Peltier-Elemente 831 sowie Kühlrippen 832 aufweist. Zur Regelung der Temperatur des Küvettenblocks 830 ist in einer Aufnahme zwischen dem Basisteil 821 und dem Peltier-Element 831 ein Temperatursensor 833 angeordnet.

Am aufklappbaren Vorderteil 822 des Küvettenblocks 820 sind Anschlussflächen 824 erkennbar, die ebenfalls für die Anbringung einer Kühl- und Heizeinrichtung, beispielsweise Peltier-Elemente, genutzt werden können. Weiterhin weist das Vorderteil 822 mit den Messfenstern 202 der Küvetten 202 korrespondierende Öffnungen 825 auf, um eine optische Vermessung der flüssigen Medien in den Küvetten 201 zu ermöglichen.

Am Boden 204 jeder Küvette 201 ist ein Ultraschall-Wandler 840, beispielsweise ein Dickenschwinger, befestigt, z.B. angeklebt oder bei der Herstellung der Küvette mitgespritzt, mit welchem Ultraschallenergie in die Küvette 201 eingebracht werden kann. Die eingebrachte Ultraschallenergie wird sowohl zum Mischen der flüssigen Medien als auch zum gezielten Zuheizen - neben der Grundlast aus der Thermostatisierung durch den Küvettenblock 820 - verwendet.

Der Ultraschall-Wandler 840 ist als piezoelektrischer Dickenschwinger ausgeführt, der - wie in Fig. 15c im Detail dargestellt - im Wesentlichen aus einem scheibenförmigen, piezoelektrischen Element 842 und beidseitigen Kontaktelektroden 841 und 843 besteht. Die küvettenseitige Elektrode 841 ist über seitliche Kontaktstreifen 844 zur unteren Elektrode 843 durchkontaktiert und bildet dort halbmondförmige Kontaktflächen 845.

Für jede Küvette 201 und deren Ultraschall-Wandler 840 ist ein von einer Federkontaktplatine 846 unterstützter Kontaktblock 847 vorgesehen, der vier Kontaktfedern 848 aufweist, von welchen zwei die halbmondförmige Kontaktflächen 845 und zwei die untere Kontaktelektrode 843 des Ultraschall-Wandlers 840 kontaktieren. Die Küvette 201 weist an der Füllöffnung 207 einen Kragen 205 sowie an gegenüberliegenden Seiten Anschlagleisten 206 auf, mit welcher die Küvette 201 - gegen den Druck der Kontaktfedern 848 - im Küvettenblock 820 gehalten wird.

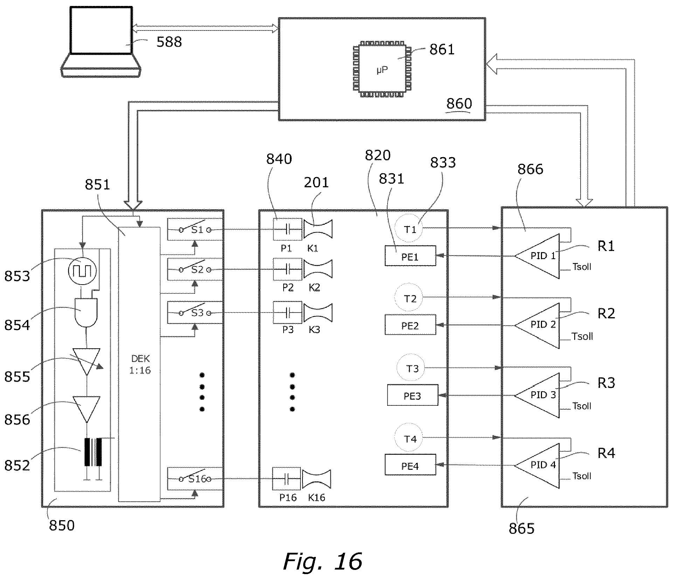

Die Federkontaktplatine 846 ist randseitig in einer waagrechten verlaufenden Nut 826 des Küvettenblocks 820 eingesteckt und stützt sich an der nach unten fortragenden Dekoderplatine 850 ab, deren Schaltungen in Fig. 16 näher erläutert werden.

In Fig. 16 ist ein Blockschaltbild zur elektronischen Ansteuerung der Vorrichtung zum Mischen und Thermostatisieren flüssiger Medien gemäß Fig. 15a dargestellt, welches die Funktionsblöcken Personal Computer 588, Controllerboard 860, Dekoderplatine 850, Küvettenblock 820, sowie eine Temperaturregelschaltung 870 umfasst.

Das Controllerboard 860 weist einen FPGA (Field-Programmable-Gate-Array) als Prozessor 861und dient zur Steuerung der Dekoderplatine 850 sowie der Temperaturregelschaltung 865. Der Personal Computer 588 kann beispielsweise über eine Ethernet Schnittstelle an das Controllerboard 860 angebunden sein und übermittelt je nach auszuführender Misch- und Thermostatisieraufgabe in einer der Küvetten 201 des Küvettenblocks 820 entsprechende Aufträge zur Ausführung von Firmware-Programmen auf dem Controllerboard 860, sowie dient zur Rückübermittlung von Kontrolldaten wie beispielsweise der gemessenen Temperaturen für die Thermostatisierung des Küvettenblocks 820.

Im Küvettenblock 820 sind jeweils an den Positionen K1 bis K16 bzw. P1 bis P16 Küvetten 201 samt den zugeordneten Ultraschall-Wandlern 840 angeordnet, wobei für die Thermostatisierung im dargestellten Beispiel in den Positionen PE1 bis PE4 bzw. T1 bis T4 jeweils ein Peltier-Element 831 samt zugeordnetem Temperatursensor 833 vorgesehen ist

Die Temperaturregelschaltung 865 weist somit vier Temperaturregelkreise 866 jeweils aus Peltier-Element 831, Temperatursensor 833 und PID (Proportional, Integral, Derivativ)-Regler R1 bis R4 auf und ist über eine Schnittstelle mit dem Controllerboard 860 zum Datenaustausch verbunden (Erhalt von Parametern wie Temperatur-Setpoints und Rückübermittlung gemessener Temperaturen der Temperaturregelschaltung 865 an das Controllerboard 860).

Die Dekoderplatine 850 ist ebenfalls über eine Schnittstelle an das Controllerboard 860 angebunden und erhält von diesem Steuersignale zur Auswahl einzelner Ultraschallwandler 840 über die auf der Dekoderplatine 850 implementierte Dekoderschaltung 851und den zugehörigen Optoschaltern 857 in den Positionen S1 bis S16, sowie Steuersignale zur Parametrisierung der Oszillatorschaltung 852. Die Oszillatorschaltung 852 erhält Steuersignale zur Anpassung von Frequenz, Tastgrad (duty ratio, duty factor, oder duty cycle), Burst-Muster (burst pattern), Amplitude, Phase sowie ON und OFF Zuständen der Signalerzeugung des Oszillators. Die Oszillatorschaltung 852 umfasst einen spannungskontrollierten Oszillator 853 (voltage-controlled oscillator, VCO), dessen Frequenzsignal über einen Burstgenerator 854 moduliert werden kann. Die Amplitude des modulierten Signals kann weiter über einen regelbaren Vorverstärker 855, sowie eine nachgeschaltete Verstärkerendstufe 856 angepasst werden. Das endverstärkte Signal wird über einen Überträger auf die benötigte Betriebsspannung der Ultraschall-Wandler 840 hochtransformiert, und über den jeweils von der Dekoderschaltung 851 selektierten Optoschalter 857 in S1 bis S16 einem der 16 piezoelektrischen Ultraschall-Wandler 840 an den Küvetten 201 auf dem Küvettenblock 820 zugeschaltet.

Das Diagramm gemäß Fig. 17a zeigt ein erstes Beispiels eines erfindungsgemäßen Thermostatisiervorganges eines Proben-Reagenzgemischs in einer Küvette, die in einem thermostatisierbaren Küvettenblock (siehe Fig. 15a) angeordnet ist.

Der Temperaturverlauf $\alpha$ zeigt die Erwärmung des Proben-Reagenzgemischs nur durch den auf die Temperatur $T_{BL}$ thermostatisierten Küvettenblock, wobei die Zieltemperatur, bei welcher das Proben-Reagenzgemischs vermessen werden kann, erst zum Zeitpunkt $t_2$ erreicht wird. Bereits wesentlich früher, zum Zeitpunkt $t_1$, wird die erforderliche Zieltemperatur erreicht, wenn Ultraschall-Boosts in den Zeitspannen M sowie A bis C eingebracht werden, wie im Temperaturverlauf $\beta$ dargestellt ist. Die Thermostatisierung des Küvettenblocks erfolgt bei einer im Wesentlichen konstanten elektrischen Leistung $P_{BL}$.

1) Vorerwärmung des Küvettenblocks mit darin befindlichen leeren Küvetten auf eine Blocktemperatur $T_{BL}$ (typischerweise 37.0 bis 37.5°C) und Stabilisierung der Blocktemperatur auf 0.1°C.

2) Befüllen einer leeren Küvette mit einem Proben-Reagenzgemisch der Temperatur $T_0$. Typischerweise hat das Proben-Reagenzgemisch nach Pipettierung in die Küvette eine Temperatur von 10-15°C, weil die zupipettierten Reagenzien aus einem auf 5°C gekühlten Lagerbereich stammen und sich im Pipettor und in den Zuführleitungen auf 10-15°C erwärmen.

3) Abgabe eines Ultraschallsignals für eine vordefinierte kumulierte Zeitdauer M, die bei einem Ultraschallsignal mit der gemittelten elektrischen Leistung $P_P$ eine Energiemenge M x $P_P$ in das Proben-Reagenzgemisch einbringt und einen errechneten Temperaturhub $\Delta T_M$ bewirkt, welcher aus variablen, aus den Daten der durchzuführenden Analyse bekannten Eigenschaften des Proben-Reagenziengemischs wie Wärmekapazität, Viskosität, Wärmeleitfähigkeit sowie dessen Volumen und konstanten, in der Vorrichtung hinterlegten (abgespeicherten) Daten errechnet wird. Die in der Zeitdauer M eingebrachte Energiemenge genügt, um das Proben-Reagenziengemisch ausreichend zu vermischen.

Typischerweise reicht für das homogene Vermischen eine Mischdauer von 1 bis 3 Sekunden, wobei der Temperaturhub $\Delta T_M$ eines beispielsweise 2-sekündigen Mischpulses etwa 3 °C betragen kann.

Alternativ kann die zum Erhalt eines stabilen Messsignals oder Inkubationsvorgangs erforderliche Mischdauer M bei gegebener Ultraschallleistung $P_P$ durch Versuche an verschiedenen Proben-Reagenzgemischen ermittelt und in der Vorrichtung abgespeichert werden.

Als weitere alternative Methode kann ein optisches Signal einer Analytmessung aus dem Proben-Reagenzgemisch laufend gemessen werden und der Mischvorgang abgebrochen werden, sobald eine stabiles Signal erhalten wird, wobei der Temperaturhub $\Delta T_M$ hierbei - wie erwähnt - aus bekannten thermischen Charakteristika errechnet wird.

4) Einhalten einer Pause >1 s (Zur Abkühlung des Küvettenbodens und der Klebestelle zum Ultraschall-Wandler)

5) Abgabe eines oder mehrerer gegebenenfalls durch Pausen > 1 s unterbrochenen Ultraschallsignals bei einer errechneten Temperatur $T_A$ für einer vordefinierte kumulierte Zeitdauer A + B + C + n, die einem zusätzlichen errechneten Temperaturhub $\Delta T_A, + \Delta T_B + \Delta T_C + \Delta T_N$ entspricht, wobei nach Abgabe des letzten Ultraschallpulses eine unter der Temperatur $T_{BL-x}$ liegende, Temperatur $T_{BL-y}$ erreicht wird. Ab dieser Temperatur erfolgt der Temperatureintrag in den Küvetteninhalt rein über Wärmeleitung zwischen dem Küvettenblock 820 und dem Küvetteninhalt.

6) Erreichen einer für die Analyse akzeptablen Temperatur $T_{BL-x}$, die um den Wert x unter der Temperatur des Küvettenblocks liegt, wobei x typischerweise bei einem festgelegten Wert von 0.1 - 0.5 °C liegt. Die akzeptable Temperatur ist festgelegt und liegt zwischen 36.5 und 37.5 °C. Die Temperaturkonstanz während der Zeitdauer einer darauf folgenden optischen Messung soll bei etwa 0.1°C liegen.

Das Diagramm gemäß Fig. 17b zeigt ein zweites Beispiels eines erfindungsgemäßen Thermostatisiervorganges eines Proben-Reagenzgemischs in einer Küvette, die in einem thermostatisierbaren Küvettenblock (siehe Fig. 15a) angeordnet ist.

1) (wie Beispiel 1) Vorerwärmung des Küvettenblocks mit darin befindlichen leeren Küvetten auf eine Blocktemperatur $T_{BL}$ (typischerweise 37.0 bis 37.5°C) und Stabilisierung der Blocktemperatur auf 0.1°K

2) (wie Beispiel 1) Befüllen einer leeren Küvette mit einem Proben-Reagenzgemisch der Temperatur $T_0$. Typischerweise hat das Proben-Reagenzgemisch nach Pipettierung in die Küvette eine Temperatur von 10-15°C, weil die zupipettierten Reagenzien aus einem auf 5°C gekühlten Lagerbereich stammen.

3) (wie Beispiel 1) Abgabe eines Ultraschallsignals für eine vordefinierte kumulierte Zeitdauer M, die bei einem Ultraschallsignal mit der gemittelten elektrischen Leistung $P_P$ eine Energiemenge M x $P_P$ in das Proben-Reagenzgemisch einbringt und einen errechneten Temperaturhub $\Delta T_M$ bewirkt, welcher aus variablen, aus den Daten der durchzuführenden Analyse bekannten Eigenschaften des Proben-Reagenziengemischs wie Wärmekapazität, Viskosität, Wärmeleitfähigkeit sowie dessen Volumen und konstanten, im Gerät hinterlegten Daten errechnet wird. Typischerweise reicht die geeignete kumulierte Zeitdauer benötigter Rührvorgänge je nach Rühraufgabe von 1 bis 3 Sekunden, wobei der Temperaturhub $\Delta T_M$ eines beispielsweise 2-sekündigen Rührpulses etwa 3 °K betragen kann. Alternativ kann die zum Erhalt eines stabilen Messsignals, eines Wasch- oder Inkubationsvorgangs erforderliche Mischdauer M bei gegebener Ultraschallleistung $P_P$ durch Versuche an verschiedenen Proben-Reagenzgemischen ermittelt und im Gerät gespeichert werden.
Als weitere alternative Methode kann ein optisches Signal aus dem Proben-Reagenzgemisch laufend gemessen werden, und der Mischvorgang abgebrochen werden, sobald eine stabiles Signal erhalten wird, wobei der Temperaturhub $\Delta T_M$ hierbei wie erwähnt aus bekannten thermischen Charakteristika errechnet wird.

4) (wie Beispiel 1) Einhalten einer Pause >1 s (Zur Abkühlung des Küvettenbodens und der Klebestelle zum Ultraschall-Wandler)

5) Abgabe eines oder mehrerer gegebenenfalls durch Pausen > 1 s unterbrochenen Ultraschallsignals erst bei einer errechneten Temperatur 0.5 x ($T_{BL}$ - $T_0$), für eine vordefinierte kumulierte Zeitdauer A + B + n, die einem zusätzlichen, errechneten Temperaturhub $\Delta T_A$, + $\Delta T_B$ + $\Delta T_n$ entspricht, wobei nach Abgabe des letzten Ultraschallpulses eine unter der akzeptablen Temperatur $T_{BL-x}$ liegende, sicher errechenbare Temperatur $T_{BL-y}$ erreicht wird. Ab dieser Temperatur erfolgt der Temperatureintrag in den Küvetteninhalt rein über Wärmeleitung zwischen dem Küvettenblock und dem Küvetteninhalt.

6) (wie Beispiel 1) Erreichen einer für die Analyse akzeptablen Temperatur $T_{BL-x}$, die um den Wert x unter der Temperatur des Küvettenblocks liegt, wobei x typischerweise bei einem festgelegten Wert von 0.1 - 0.5 °K liegt. Die akzeptable Temperatur ist festgelegt und liegt zwischen 36.5 und 37.5 °C. Die Temperaturkonstanz während der Zeitdauer einer darauffolgenden optischen Messung soll bei etwa 0.1°K liegen.

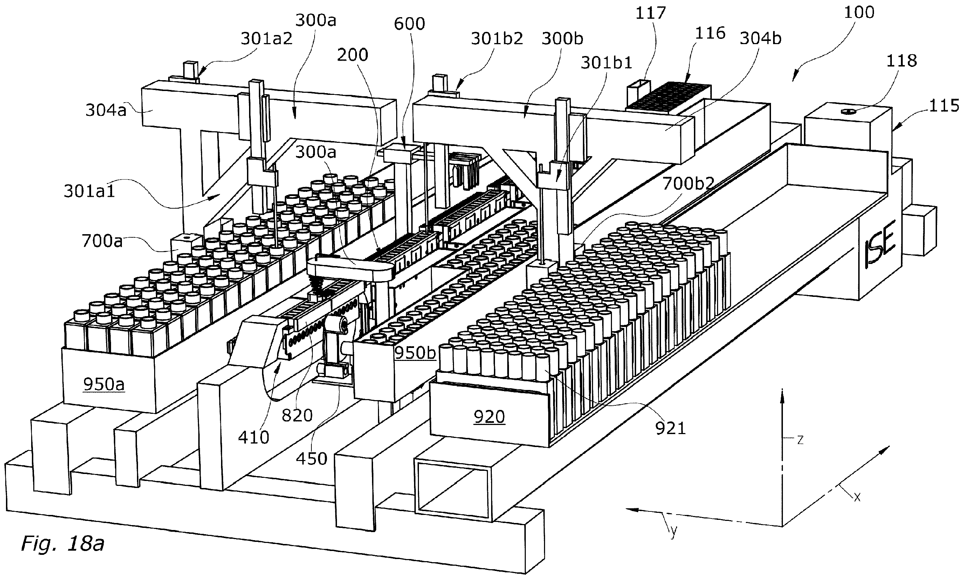

Die in den Fig. 18a, 18b sowie 19a bis 22 beschriebene, dritte Ausführungsvariante des automatischen Analysators 100 weist die schon im Zusammenhang mit der ersten und zweiten Ausführungsvariante ausführlich erläuterten Komponenten, wie entlang des stationären Küvettenarrays 200 verfahrbare Pipettoren 300a, 300b, bevorzugt mit den Pipettoren 300a, 300b mitfahrende Nadelwascheinheiten 700a1 bis 700b2, sowie eine entlang des Küvettenarrays 200 verfahrbare Küvettenwascheinheit 600 auf, sowie zusätzlich eine Vorrichtung zur Durchführung von heterogenen Immunoassays 410.

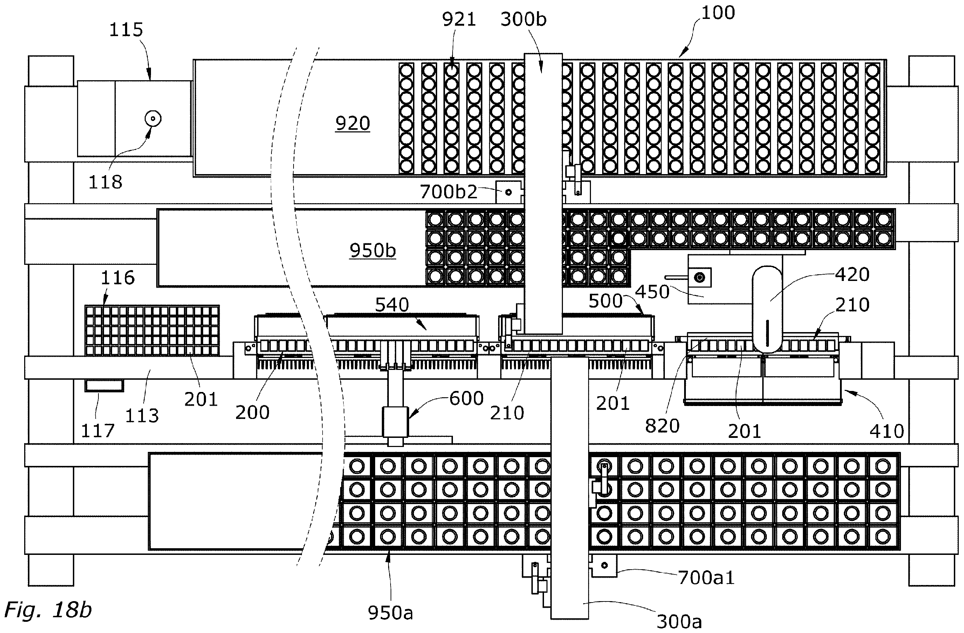

Der in den Fig. 18a und 18b dargestellte automatische Analysator 100 wird um eine Vorrichtung zur Durchführung von heterogenen Immunoassays 410 (HetIA-Modul) erweitert, die direkt in Verlängerung des stationären Küvettenarrays 200 angeordnet ist.

Die Küvetten 201 des HetIA-Moduls, die zur Aufnahme flüssiger Medien (Proben, Reagenzien, Suspensionen mit magnetischen Partikeln, Waschlösungen) in einem thermostatisierten Küvettenblock 820 angeordnet sind, bilden ein endständiges Segment 210 des stationären, linearen Küvettenarrays 200 des Analysators 100, derart, dass die entlang des Küvettenarrays 200 verfahrbaren Pipettoren 300a, 300b auch die Küvetten 201 des HetIA-Moduls mit Proben und Reagenzien aus dem Proben- und Reagenzienlager 920, 950a, 950b sowie mit magnetischen Partikeln und Waschlösungen versorgen können. Weiters hat auch die entlang des Küvettenarrays 200 verfahrbare Küvettenwaschstation 600 Zugriff auf die Küvetten 201 des HetIA-Moduls.

Falls Küvetten 201 des HetIA-Moduls oder in anderen Bereichen des Küvettenarrays 200 ausgewechselt werden müssen, können diese mit Hilfe eines Greifmechanismus (nicht dargestellt), beispielsweise des Pipettors 300b, oder der Küvettenwascheinheit 600 aus einem (beispielsweise am Ende des Küvettenarrays 200 angeordneten) Küvettenmagazin 116 entnommen werden, wobei gebrauchte Küvetten in einen Abfallschacht 117 entsorgt werden.

Der automatische Analysator 100 kann auch mit einer ISE-Messstation 115 ausgestattet sein, in welcher ionenselektive Messungen an den Proben durchgeführt werden. Die Proben werden mit dem Pipettor 300b aus dem Probenlager 920 entnommen und in die Eifüllöffnung 118 der ISE-Messstation 115 pipettiert.

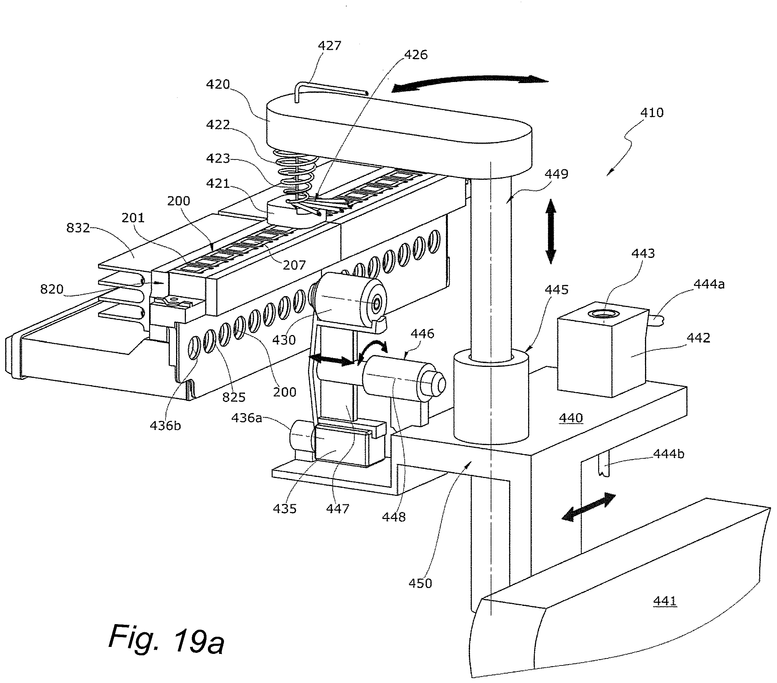

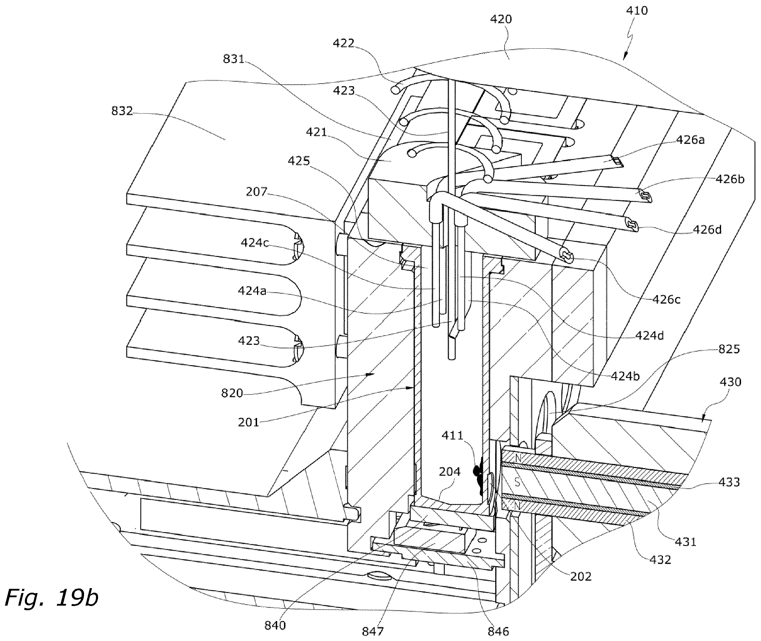

In den Fig. 19a und 19b wird die erfindungsgemäße Vorrichtung 410 (HetIA-Modul) im Detail dargestellt.

Ein verschwenkbarer Haltearm 420 der Vorrichtung 410 ist entlang des Küvettenarrays 200 verfahrbar ausgeführt und kann in Richtung der Füllöffnung 207 einer von der Steuerlogik der Vorrichtung ausgewählten Küvette 201 abgesenkt werden. Der Haltearm 420 ist mit einer, in Richtung Boden 204 der Küvette 201 absenkbaren Absaugnadel 423 samt Absaugleitung 427 ausgestattet, sowie mit zumindest einem, über oder in der jeweiligen Füllöffnung 207 positionierbaren Dispensor 424a bis 424d zur Abgabe der flüssigen Medien in die Küvette 201. Zumindest ein Dispensor 424a, 424b ist zur Abgabe einer Waschlösung für die magnetischen Partikel 411 ausgebildet.

Die Zuleitungen zu den Dispensoren 424a, 424b sind mit 426 bezeichnet, im Speziellen führt eine Waschleitung 426a zum Dispensor 424a, eine Waschleitung 426b zum Dispensor 424b, eine Zuleitung 426c zum Dispensor für eine Pretriggerlösung und eine Zuleitung 426d zum Dispensor 424d für eine Triggerlösung.

Weiterhin ist eine, entlang des Küvettenarrays 200 verfahrbare, auf den Inhalt der ausgewählten Küvette 201 wirkende Magnetanordnung 430 zur Separation der magnetischen Partikel 411 an einer Innenfläche der Küvette 201 vorgesehen, sowie eine, entlang des Küvettenarrays 200 verfahrbare optische Detektionseinrichtung 435, die auf das Messfenster 202 der ausgewählten Küvette 201 ausgerichtet werden kann, um ein zu der Analytkonzentration in der ausgewählten Küvette 201 proportionales Messsignal zu erhalten.

Zur Vereinfachung sind nur jene Komponenten der Vorrichtung 410 dargestellt, die für die gegenständliche Erfindung wesentlich sind, wobei auf Analysatorkomponenten, wie Proben- und Reagenzienlager, Pumpen, Ventile, Auswerte-, Steuer- und Antriebseinheiten, nicht näher eingegangen wird.

Das Küvettenarray 200 ist in einem thermostatisierbaren Küvettenblock 820 angeordnet, wobei insbesondere in Fig. 19b die für die Thermostatisierung vorgesehenen Peltier-Elemente 831 erkennbar sind, die zwischen Kühlrippen 832 und dem Küvettenblock 820 angeordnet sind. Der Küvettenblock 820 weist an der Vorderseite mit den Messfenstern 202 der Küvetten 201 fluchtende Zugangsöffnungen 825 auf.

Am verfahrbaren Haltearm 420 ist an einer federnden Halterung (siehe Federelement 422) eine auf die Füllöffnung 207 der Küvette 201 absenkbare Dispensorplattform 421 befestigt, die im dargestellten Beispiel vier Dispensoren 424a bis 424d zur Abgabe flüssiger Medien in die Küvette 201 aufweist. Die Dispensorplattform 421 wird in einer zentralen Öffnung von der am Haltearm 420 befestigten Absaugnadel 423 durchsetzt, sodass diese nach Anlage der Dispensorplattform 421 an der Füllöffnung 207 der Küvette 201 bis zum Boden 204 der Küvette 201 abgesenkt werden kann.

Die Dispensorplattform 421 weist an der der Küvette 201 zugewandten Seite eine Dichtfläche 425 aus einem lichtundurchlässigen Material auf, sodass bei abgesenkter Dispensorplattform 412 der Zutritt von Umgebungslicht bei der optischen Vermessung des Küvetteninhalts ausgeschlossen ist.

Erfindungsgemäß weist ein Dispensor 424a zur Abgabe einer Waschlösung für die magnetischen Partikel 411 eine parallel zur Längsachse der Küvette 201 ausgerichtete Ausströmungsrichtung (gerade Waschnadel) auf, und ein zweiter Dispensor 424b - ebenfalls zur Abgabe einer Waschlösung - eine auf eine innere Seitenfläche der Küvette 201 zielende Ausströmungsrichtung (schräge Waschnadel) auf.

Von weiteren Dispensoren 424c, 424d der Dispensorplattform 412, deren Ausströmungsrichtung parallel zur Längsachse der Küvette 201 ausgerichtet sind, ist ein optionaler dritter Dispensor 424c ggf. zur Abgabe einer Pretriggerlösung und ein vierter Dispensor 424d zur Abgabe einer Triggerlösung ausgebildet. Für auf Chemolumineszenz basierende Immunoassays, die mit nur einer Triggerlösung auskommen, kann der dritte Dispensor 424c ungenutzt bleiben oder entfallen.

Das in den Fig. 19a und 19b dargestellte Ausführungsbeispiel zeichnet sich durch eine entlang des Küvettenarrays 200 verfahrbare Plattform 440 aus, welche eine Hub- und Dreheinrichtung 445 aufweist, mit welcher der Haltearm 420 samt Absaugnadel 423 und den Dispensoren 424a bis 424d der Dispensorplattform 421 absenkbar ausgeführt ist. Bevorzugt ist auf der verfahrbaren Plattform 440 auch eine gemeinsame Aufhängung 446 für die Magnetanordnung 430 und die Detektionseinrichtung 435 angeordnet, sodass ein verfahrbares Mess- und Manipulationsmodul 450 realisiert wird, das sämtliche robotische, fluidische und messtechnische Komponenten für die Prozessschritte der magnetischen Separation der Beads, des sogenannten B/F - Waschens, sowie der Auslösung (Triggerung) und Messung der Lumineszenz vereint.

Die verfahrbare Plattform 440 des Mess- und Manipulationsmoduls 450 ist über eine parallel zum Küvettenarray 200 verlaufende, seitliche Schiene 441 mit dem Rahmen der Vorrichtung 410 verbunden, und kann über einen Verfahrmechanismus wie beispielsweise einem schrittmotorangetriebenen Zahnriemen, einer Spindel oder einen Linearmotor auf die Position einer ausgewählten Küvette 201 gebracht werden. Zur Versorgung und Steuerung des Mess- und Manipulationsmoduls 450 können hierbei flexible elektrische und fluidische Verbindungsleitungen beispielsweise in Form sog. Energieketten (nicht dargestellt) an die Plattform 440 herangeführt werden.

Gemäß einer Ausführungsvariante kann auf der verfahrbaren Plattform 440 auch eine Waschstation 442 für die Absaugnadel 423 und den zumindest einen Dispensor 424a bis 424d der Dispensorplattform 421 angeordnet sein, auf deren Öffnung 443 der Haltearm 420 nach einer Drehbewegung absenkbar ausgeführt ist, sodass die gesamte Nadelgruppe am Kopf des schwenkbaren Haltearms 420 in die Öffnung 443 eingeführt werden kann.

Die Nadelwaschstation 442 weist eine den Füllstand begrenzende, obere Absaugleitung 444a und eine untere Absaugleitung 444b auf. Hierbei ist ein Zufahren auf die Öffnung 443 durch eine Auf- und Abbewegung mit einem 90° Schwenk bei gleichzeitiger Absenkung des Haltearms 420 unter die Oberkante des Küvettenarrays 200 möglich, wodurch andere Robotikkomponenten, beispielsweise allfällige Pipettoren, etc., ungehindert entlang des Küvettenarrays 200 verfahren können.

Der schwenkbare Haltearm 420 des Mess- und Manipulationsmoduls 450 ist an einem in horizontaler Ebene um 90° schwenkbaren und zusätzlich vertikal beweglichen Turm 449 befestigt, wobei die Schwenkbewegung durch einen beispielsweise schrittmotorangetriebenen Drehaktuator ermöglicht wird. Zusätzlich ist der Turm mit einer Hebevorrichtung ausgestattet, die beispielsweise eine schrittmotorangetriebene Spindel oder einen Zahnriemen zur Erzeugung einer vertikalen Translationsbewegung des Haltearms 420 umfasst. Die beiden Bewegungsarten können in die kombinierte Hub- und Dreheinrichtung 445 an der Basis des vertikalen Turms 449 integriert sein.

Gemäß einer Ausführungsvariante kann die Nadelwaschstation auch stationär an einer Position unterhalb der verfahrbaren Plattform 440 entlang ihres horizontalen Verfahrraumes positioniert sein.

Eine Ausführungsvariante kann auch darin bestehen, dass die Nadelwaschstation stationär am Ende des Küvettenarrays 200 positioniert ist, wobei der Haltearm der Nadelgruppe in dieser Variante nicht schwenkbar ausgeführt sein muss.

Gemäß einer bevorzugten Ausführungsvariante ist die gemeinsame Aufhängung 446 für die Magnetanordnung 430 und die Detektionseinrichtung 435 geeignet, eine translatorische oder rotatorische Bewegung auszuführen, um die Positionen der Magnetanordnung 430 und der Detektionseinrichtung 435 vor der ausgewählten Küvette 201 auszutauschen.

Beispielsweise können die Magnetanordnung 430 und die Detektionseinrichtung 435 im gleichen Abstand zu einer gemeinsamen Drehachse 448 an einem in der Aufhängung 446 gelagerten Rotorarm 447 befestigt sein.

Bevorzugt kann dabei der in der Aufhängung 446 gelagerten Rotorarm 447 translatorisch in Richtung der Drehachse 448 verschiebbar ausgeführt sein, um die Magnetanordnung 430 oder die Detektionseinrichtung 435 an die Zugangsöffnung 825 im Küvettenblock 820 und somit an das Messfenster 202 der ausgewählten Küvette 201 heranzuführen. Der Fotomultiplier 435 sowie die Magnetanordnung 430 können mit ihrer jeweiligen optischen Haupt- bzw. Polachse auf die entsprechende Zugangsöffnung 825 im Küvettenblock ausgerichtet werden und durch eine Horizontalbewegung an die jeweilige Öffnung lichtdicht andocken, bzw. zur Erzeugung einer möglichst hohen magnetischen Flussdichte optimal an die Wand der Küvette 201 angenähert werden.

Die Magnetanordnung 430 kann aus einem oder mehreren Magneten bestehen, die vorzugsweise Seltenerdemagneten hoher Feldstärke, wie z.B. $Nd_2Fe_{14}B$ (Neodym-Eisenborat) sind, kann aber auch als Elektromagnet ausgeführt sein. Die Magnetanordnung 430 ist vorzugsweise aus Neodym-Stabmagneten mit zwei verschiedenen Stabradien ausgeführt, wobei im Wesentlichen ein innerer Stab 431 unter Zwischenlage einer nichtmagnetischen Zwischenschicht 433 von einem äußeren, hohlzylindrischen Stab 432 umfasst wird und die zwei Stäbe unterschiedlicher Länge und Durchmesser einen konischen Übergang aufweisen. Die Anordnung läuft in einen schlanken Endbereich mit punktuell hoher magnetischer Flussdichte aus, der durch die Öffnung 825 im Küvettenblock 820 nahe an das Fenster 201 der Küvette 201 herangebracht werden kann. Die Magnetanordnung 430 kann auch aus mehreren, einzelnen Magneten zusammengesetzt sein, um die für die magnetische Separation an einer Küvettenwand notwendige magnetische Feldstärke zu erhöhen, oder Streufelder in die Nachbarküvetten zu verringern. Ein Beispiel einer Magnetanordnung ist in Fig. 19b dargestellt, wobei ein bipolares Ende einer konzentrischen Magnetanordnung 430 mit einer nichtmagnetischen Zwischenschicht 433 auf die Küvette 201 gerichtet ist.

Gemäß einer Ausführungsvariante kann eine zweite, entlang des Küvettenarrays 200 verfahrbare, auf den Inhalt der ausgewählten Küvette 201 wirkende Magnetanordnung (nicht dargestellt) vorgesehen sein, die vorzugsweise mit zumindest einem der magnetischen Pole der ersten Magnetanordnung 430 eine magnetische N-S Brücke ausbildet. Die verfahrbare Plattform 440 des Mess- und Manipulationsmoduls 450 kann beispielsweise einen C-förmigen, unter dem stationären Küvettenarray 200 hindurchgeführten Ausleger aufweisen, der es erlaubt, einen zweiten Separationsmagneten entlang der magnetischen Wirkachse des ersten Separationsmagneten auszurichten, und auf der anderen Seite des Küvettenblocks 820 mitfahren zu lassen. Hierbei ist eine zur ersten Zugangsöffnung 825 der jeweiligen Küvette 201 vergleichbare zweite Öffnung nicht erforderlich, da die magnetischen Feldlinien der zweiten Magnetanordnung durch das Material des nicht aus ferromagnetischen Materials bestehenden Küvettenblocks (Aluminium) hindurchwirken. Idealerweise ist die Polarität der beiden Separationsmagneten entgegengesetzt orientiert, sodass ein magnetischer Reihenschluss (N-S) entsteht, der zu einer punktuellen Erhöhung der magnetischen Flussdichte und einer Verringerung des unerwünschten Streufeldes auf die benachbarten Küvetten führt. Das Streufeld beeinflusst die in Nachbarküvetten befindlichen magnetischen Beads in ungünstiger Weise, da sich die Beads in den Nachbarküvetten in anderen Prozessstadien befinden können, bei denen eine magnetische Separation oder Agglomeration unerwünscht ist.

Die zweite Magnetanordnung kann sowohl aus einem oder mehreren Elektromagneten, als auch aus Permanentmagneten bestehen, wobei bei Permanentmagneten ein Aktuator vorgesehen werden muss, um die Magnetanordnung zur Küvette wahlweise anzunähern, oder zu entfernen. Der Aktuatormechanismus kann analog zu dem für die erste Magnetanordnung 430 ausgeführt sein und in bekannter Weise über einen Riemenantrieb, eine Antriebsspindel oder ein Solenoid verfügen.

Gemäß einer weiteren, denkbaren Konfiguration, ist vorgesehen, dass die zweite Magnetanordnung auf einer eigenen Schiene von der ersten Magnetanordnung 430 unabhängig an den Komponenten des Mess- und Manipulationsmoduls 450 vorbei bewegbar ist, sodass zusätzlich zu den oben genannten Vorteilen eines mitfahrenden zweiten Separationsmagneten, zeitgleich eine magnetische Separation an einer anderen Küvette möglich ist, um magnetische Beads für einen Waschschritt eines zweiten Assays in der anderen Küvette bereits vorzuseparieren und damit Zeit zu sparen.

Die Detektionseinrichtung 435 ist vorzugsweise durch einen kompakt bauenden Fotomultiplier realisiert und dient zur Messung der Lichtmenge während der durch Zugabe der beiden Triggerlösungen ausgelösten Chemolumineszenz, und kann mit einer Peltier-Kühlung ausgestattet sein, um ein konstanteres, rauschärmeres Signal zu erhalten. Zur Vermeidung von Fehllicht bei der Messung an einer der Zugangsöffnung 825 des Küvettenblocks 820, können die Zugangsöffnungen 825 und die Lichteinlassöffnung des Fotomultipliers konzentrisch gestufte Kontaktflächen am Rand der beiden Öffnungen aufweisen. Weiters kann ein beispielsweise mechanisch betätigtes Blendenelement (Shutter) vorgesehen sein, um den Fotomultiplier im Ruhezustand vor dem Eintritt von Umgebungslicht zu schützen.

Zur Messung der Lumineszenz bei niedriger Analytkonzentration wird bevorzugt ein digitaler Fotomultiplier eingesetzt, der pro eintreffendem Photon triggert und einen digitalen Impuls von 10 ns auslöst. Diese kurzen Pulse werden mit dem FPGA des HetIA Controllers 460 gezählt und als Zählerstand über eine einstellbare Abtastzeit aufsummiert. Solange die Photonenanzahl klein ist, können die unregelmäßig entstehenden Impulse einzeln ausgegeben werden, die Impulsanzahl pro Zeiteinheit entspricht dann der Photonenanzahl pro Zeiteinheit.

Erfindungsgemäß kann auf der verfahrbaren Plattform 440 eine Referenzlichtquelle 436a für die Detektionseinrichtung 435 angeordnet sein. Die Referenzlichtquelle 436a dient zur Kalibration des Fotomultipliers und weist eine Lichtaustrittsöffnung auf, die in Richtung der Eintrittsöffnung der Detektionseinrichtung 435 (z.B. Fotomultiplier) ausgerichtet ist. Die Referenzlichtquelle 436a kann an beliebiger Stelle entlang der Bewegungslinie der Detektionseinrichtung 435 angeordnet sein, idealerweise jedoch derart, dass eine Kalibration des Fotomultipliers dann erfolgt, wenn sich die Magnetanordnung 430 gerade vor der jeweiligen Zugangsöffnung 825 des Küvettenblocks 820 befindet.

Alternativ zu dieser Variante kann eine Referenzlichtquelle 436b auch ortsfest am Ende des Küvettenblocks 820 angeordnet sein und eine Lichtaustrittsöffnung entlang der Zugangsöffnungen des Küvettenblocks 820 aufweisen, wodurch dessen Thermostatisiereinrichtung für die Referenzlichtquelle 436b mitgenutzt werden kann.

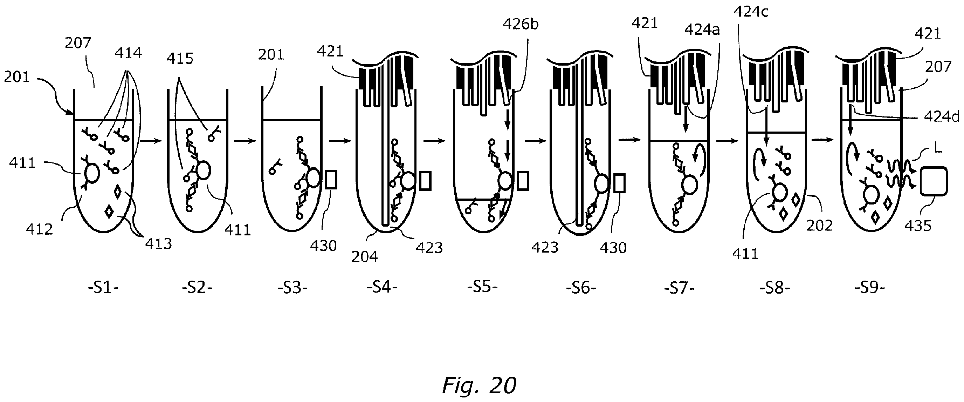

Das Ablaufbeispiel eines heterogenen Immunoassays wird beispielhaft in den Stufen S1 bis S9 in Fig. 20 dargestellt.

Das vorliegende Beispiel eines heterogenen Immunoassays bezieht sich auf die erforderlichen maschinellen Prozesse bei einem sogenannten "Sandwich-Assay". Hierbei bildet das Analytmolekül 413 (ein körpereigenes Protein, z.B. Prostataspezifisches Antigen) durch Antigen-Antikörper-Interaktionen eine Brücke zwischen einem auf der Oberfläche der magnetischen Partikel 411 immobilisierten, ersten Antikörper (Fängerantikörper 412) und einem zweiten Antikörper, an welchen Signalmoleküle gebunden sind (Tracer-Antikörper 414), die nach Zugabe einer Pretriggerflüssigkeit und einer Triggerflüssigkeit eine der Analytmenge proportionale, wenige Sekunden anhaltende Chemolumineszenz hervorruft. Die beiden Antikörper-Typen liegen im Vergleich zum Analyten im Überschuss vor. Bei Analytmolekülen, die zu klein sind, um Bindungsstellen für zwei verschiedene Antikörper aufzuweisen, werden sogenannte kompetitive Immunoassays eingesetzt, wobei die Tracer-Antikörper direkt mit den Analytmolekülen um Bindungsstellen an einem immobilisierten Antikörper konkurrieren.

Bei einem einfachen 1-Step-Assay gemäß Fig. 20 werden zunächst die Probe (enthält den Analyt 413), eine Suspension magnetischer Partikel 411 (Magnetic Beads) mit einer Beschichtung aus einem Fängerantikörper 412, sowie eine Lösung des Tracer-Antikörpers 414 in die Küvette 201 mittels eines hier nicht dargestellten Pipettors einpipettiert (S1, in Fig.20).

Während der nachfolgenden Inkubation (ca. 10 min) bei 37 °C wird die Lösung periodisch beispielsweise mittels Ultraschall gerührt, um ein Absinken und Agglomerieren der Beads zu verhindern. Nun ist jedes Analytmolekül "sandwichartig" zwischen einem auf den Beads 411 immobilisierten Fängerantikörper 412 und einem Tracer-Antikörper 414 gebunden. Weiters gibt es unspezifisch gebundene Tracer-Antikörper 415 (S2, in Fig. 20).

Die Beads 411 samt den daran gebundenen Substanzen werden nun mit Hilfe der Magnetanordnung 430 an der Innenwand der Küvette 201 fixiert (S3, in Fig. 20) und die gesamte Flüssigkeit mit der aus der der Dispensorplattform 421 abgesenkten Absaugnadel 423 entfernt (S4, in Fig. 20).

In weiterer Folge wird eine Waschlösung durch eine schräg auf die Innenwand der Küvette 201 gerichtete Waschnadel 424b eingebracht, um an den Beads 430 anhaftende, und in der Reaktionslösung verbliebene ungebundene Tracer-Antikörper durch vorsichtige Spülung der Beads zu entfernen, wobei die Beads 411 nach wie vor magnetisch an der Gefäßwand gehalten werden (S5, in Fig. 20).

Anschließend wird die Küvette 201 erneut trocken gesaugt, wobei die Beads 411 samt den daran gebundenen Substanzen noch immer magnetisch an der Innenwand der Küvetten 201 fixiert sind (S6, in Fig. 20).

Eine zweite, vertikal ausgerichtete Waschnadel 424a hingegen erzeugt beim Eindüsen von Waschlösung oder Verdünnungsflüssigkeit Turbulenzen in der Flüssigkeit, sodass die Beads 411 bei abgedockten Magneten in der Flüssigkeit resuspendiert werden (S7, in Fig. 20).

Nach diesem Waschschritt, der mehrere Male hintereinander durchgeführt werden kann, wird der Fotomultiplier 435 an die Küvette 201 herangefahren. Durch die beiden Dispensoren 424c und 424d werden nun in schneller unmittelbarer Abfolge Pretrigger- (S8, in Fig. 20) und Triggerlösung (S9, in Fig. 20) zugeführt. Damit wird eine nur wenige Sekunden andauernde Chemilumineszenz L (Flash Luminescence) ausgelöst, die vom Fotomultiplier 435 gemessen werden kann. Die hierfür auf die Füllöffnung 207 der Küvette 201 aufgesetzte Dispensorplattform 421 des Haltearms sorgt gleichzeitig für die hierzu benötigte Verdunkelung der Küvette 201.

Im Anschluss wird die benutzte Küvette 201 mit der Absaugnadel 423 leergesaugt und entweder durch eine Wegwerfküvette ausgetauscht, oder gereinigt und wiederverwendet, sodass eine neuer Immunoassay in der zuvor genutzten Küvettenposition stattfinden kann.

Zum Waschen der Küvette muss der Manipulator von der Küvette wegbewegt werden, sodass die Küvettenwaschstation heranfahren und mit dem Waschen beginnen kann.

Grundsätzlich können aber auch andere, etwas abgewandelte Immunoassays, die eine magnetische Separation mit B/F - Waschen als Prozessschritt aufweisen, mit der erfindungsgemäßen Vorrichtung durchgeführt werden, wobei für die Detektion gegebenenfalls auch eine andere Detektionsmethode als Messung der Chemolumineszenz vorgesehen sein kann.

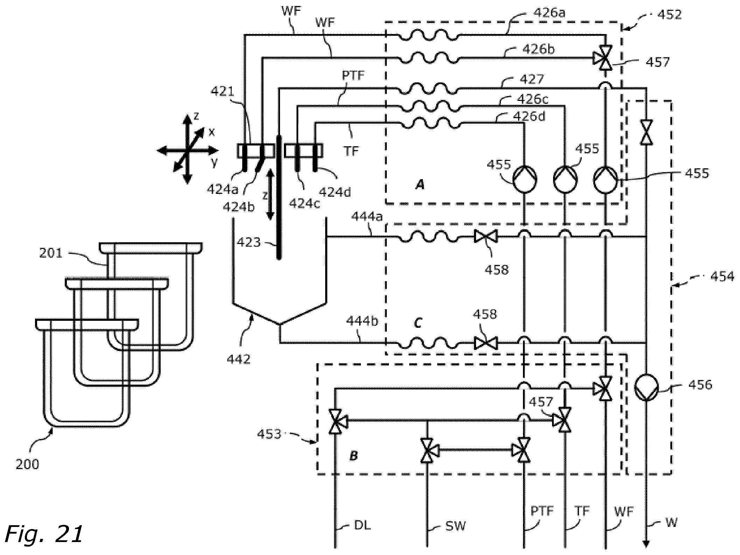

Wie in Fig. 21 schematisch dargestellt, verfügt das verfahrbare Mess- und Manipulationsmodul 450 der Erfindung gemäß Fig. 18a über ein fluidisches System 451 zur Versorgung der Dispensorplattform 421 mit Waschflüssigkeit WF, Pretriggerflüssigkeit PTF, Triggerflüssigkeit TF und Druckluft DL. Weiters sind Einrichtungen zur Absaugung von Reaktionsgemisch oder Waschflüssigkeit aus den Küvetten 201 des Küvettenarrays 200 sowie dem Behälter, bzw. Waschtrog, der Waschstation 442 vorgesehen.

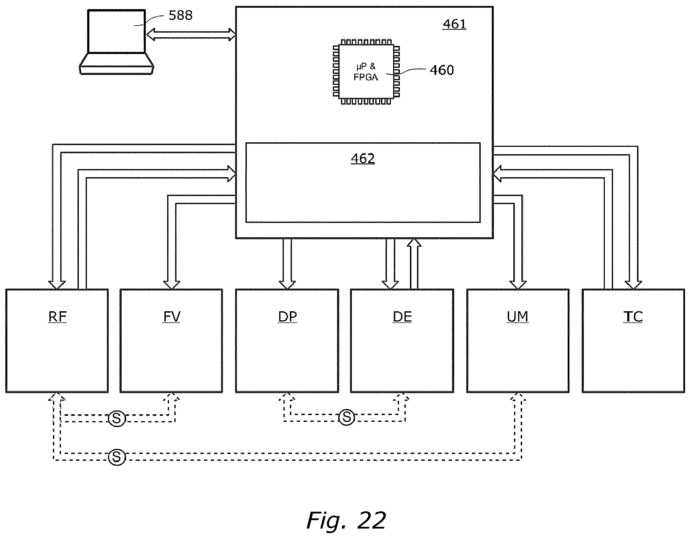

Das fluidische System 451 wird über den HetIA Controller 460 (siehe Fig. 22) gesteuert und umfasst eine Reihe von magnetisch betätigbaren 3-Wegeventilen 457 und Präzisionskolbenpumpen als Dispensierpumpen 455, welche an die verfahrbare Plattform 440 (siehe Fig. 19a) über flexible Schlauchverbindungen (mit Wellenlinien angedeutet) angebunden sind.

Die über die kombinierten Freiheitsgrade der verfahrbaren Plattform 440 sowie des verschwenkbaren Haltearms 420 in x, y und z Richtung verfahrbare Dispensorplattform 421 umfasst eine Gruppe von Dispensoren 424a bis 424d, die durch die absenkbare Absaugnadel 423 ergänzt wird.

Die Dispensiereinheit 452 umfasst jeweils eine eigene Dispensierpumpe 455 für die Bereitstellung von Waschflüssigkeit WF, Pretrigger- PTF und Triggerflüssigkeit TF, wobei der Flüssigkeitsstrom aus der Dispensierpumpe 455 für die Waschflüssigkeit über ein 3-Wegeventil 457 jeweils der geraden 424a oder der schrägen Waschnadel 424b zugeschaltet werden kann. Die vier selektiv beschickbaren Versorgungsleitungen sind an den bewegbaren Stellen aus einem flexiblen Kunststoff ausgeführt und in Energieketten (nicht dargestellt) geführt.

Die Dispensierpumpen 455 der Dispensiereinheit 452 sind jeweils über eigene Versorgungsleitungen an das Ventilnetzwerk 453 angebunden, wobei für Spül- und Reinigungszwecke, insbesondere zur Reinigung der Dispensoren 424a bis 424d und der Absaugnadel 423, jeweils anstelle des primären Fördermediums alternativ auch Druckluft DL oder Systemwasser SW (deionisiertes Wasser) über ein entsprechendes 3-Wegventil 457 zugeschaltet und den Dispensierpumpen 455 zugeführt werden kann.

Der Behälter der Waschstation 442 zur Reinigung der Dispensoren 424a bis 424d und der Absaugnadel 423 weist zwei Absaugleitungen 444a, 444b auf, von denen sich eine 444b im Boden des Behälters, eine zweite in der oberen Hälfte des Behälters befindet, um als Überlauf zur Einstellung eines stabilen Füllniveaus fungieren zu können. Die Absaugeinheit 454 steht sowohl mit den beiden Absaugleitungen 444a, 444b, als auch mit der Absaugnadel 423 über flexible Schlauchleitungen in Verbindung, die in Energieketten (nicht dargestellt) geführt sind. Um einen unerwünschten Rückfluss von abgesaugten Flüssigkeiten zu unterbinden, sind jeweils Absperrventile 458 vorgesehen. Die drei Abflussleitungen münden in eine gemeinsame Zuleitung einer Absaugpumpe 456 (z.B. einer selbstansaugenden Verdrängerpumpe), die die abgesaugten Abfallflüssigkeiten W einem Sammel- oder Aufbereitungsbereich in der Vorrichtung zuführt (nicht dargestellt)

Fig. 22 zeigt ein Blockschaltbild zur elektronischen Steuerung der erfindungsgemäßen Vorrichtung gemäß Fig. 19a. Der HetIA Controller 460 des Controllerboards 461 bedient die elektrischen und mechanischen Komponenten des HetIA Moduls und wird von einem Hauptrechner 588 (z.B. Personal Computer) gesteuert und programmiert. Der PC steuert den Ablauf und Reihenfolge der Teilprozesse, für die Ausführung der einzelnen Aktionen ist der HetIA Controller 460 verantwortlich.

Die Funktionen des HetIA Controllers 460 können wie folgt zusammengefasst werden (siehe Fig. 22):

- Kommunikation mit PC 588 über Ethernet-Schnittstelle

- Robotikfunktionen RF mittels Schrittmotoren

  ◦ Verfahren der Plattform 440 in x-Richtung zur jeweiligen Küvette 201 des stationären Küvettenarrays 200 (bzw. zur stationären Referenzlichtquelle 436b im Küvettenblock 820, falls keine mitfahrende Referenzlichtquelle 436a vorgesehen ist)

  ◦ Drehbewegung des Rotorarms 447 zum Positionstausch von Detektionseinrichtung 435 (Fotomultiplier) und Magnetanordnung 430

  ◦ y-Bewegung für das Andocken des Fotomultipliers 435 oder der Magnetanordnung 430 an das Messfenster der Küvette 201, bzw. an eine auf der Plattform 440 mitfahrende Referenzlichtquelle 436a

- Steuerung FV der Fluidikventile 457, 458 des Fluidiksystems 451

- Steuerung DP für die Dosierpumpen 455

- Steuerung UM für den Ultraschall-Wandler 840

  ◦ Weist einen eigenen, vom Controllerboard 461 unabhängigen US-Oszillator auf

  ◦ Dekoderfunktion für die piezoelektrischen Wandler 840 an den einzelnen Küvetten 201

- Steuerung DE für die Detektionseinrichtung 435 sowie die Referenzlichtquelle 436a

- Temperaturregelung TR für die Thermostatisierung (37°C)

  ◦ Peltier Regler für die Detektionseinrichtung 435 (Fotomultiplier)

  ◦ Peltier Regler für den Küvettenblock 820

Bestimmte Funktionen, die in Echtzeit exakt getriggert werden müssen (siehe Klammen "S" in Fig. 22), werden im FPGA des HetIA Controllers 460 realisiert. Es sind dies beispielsweise:

- zeitlich Triggerung der Steuerung DP der Dosierpumpe zeitlich mit der Steuerung DE für die Detektionseinrichtung 435 (Fotomultiplier Messung)

- Triggerung der Referenzlichtquelle zeitlich synchron mit der Messung des Fotomultipiers

- Ultraschall Mischprozess der jeweiligen Küvette.

**Patentansprüche**

1. Automatischer Analysator (100) zur Durchführung von chemischen, biochemischen und/oder immunchemischen Analysen von flüssigen Proben, mit einem Probenlager (920) zur Aufnahme der flüssigen Proben, und zumindest einem Reagenzienlager (950a, 950b) zur Aufnahme von flüssigen Reagenzien,

   mit Küvetten (201) zur Aufnahme der flüssigen Proben und Reagenzien, wobei eine Vielzahl von Küvetten (201) als stationäres, lineares Küvettenarray (200) im Analysator angeordnet ist,
   mit verfahrbaren und stationären Automatenkomponenten, zumindest umfassend:

   • einen entlang einer durch das lineare Küvettenarray (200) definierten Bewegungslinie in x-Richtung verfahrbar ausgeführten Pipettor (300a, 300b), der mit zumindest einer Pipettiernadel (301a1, 301a2, 301b1, 301b2) ausgestattet ist, die in z-Richtung in die Küvetten (201) absenkbar ausgeführt ist und in einer auf die x-Richtung im Wesentlichen normal stehenden y-Richtung zwischen den Küvetten (201), dem Probenlager (920) und dem Reagenzienlager (950a, 950b) verfahrbar ausgeführt ist,

• eine Vorrichtung zur Durchführung fotometrischer und/oder turbidimetrischer Messungen, mit einer optischen Messeinheit (500), welche mit einer Lichtbereitstellungseinheit (540) ausgestattet ist, die geeignet ist, Licht durch seitlich an den Küvetten (201) angeordnete Messfenster (202) einzustrahlen, sowie mit einer Detektionseinheit (550) zur Gewinnung eines Messsignals, die geeignet ist, durch seitlich an den Küvetten (201) angeordnete Messfenster (203) austretende Messstrahlung zu empfangen,

• eine Vorrichtung (410) zur Durchführung von heterogenen Immunoassays, mit einer entlang des Küvettenarrays (200) verfahrbaren optischen Detektionseinrichtung (435), die geeignet ist eine durch seitlich an den Küvetten (201) angeordnete Messfenster (203) austretende Messstrahlung zu empfangen,

• eine in x-Richtung verfahrbar ausgeführte Küvettenwascheinheit (600) zur Reinigung der Küvetten (201),

• eine Nadelwascheinheit (700a1, 700a2, 700b1, 700b2) zur Reinigung der zumindest einen Pipettiernadel (301a1, 301a2, 301b1, 301b2), sowie

• eine Thermostatisiereinheit (800) zur Einstellung einer Messtemperatur in den Küvetten (201),

wobei die Vorrichtung zur Durchführung fotometrischer und/oder turbidimetrischer Messungen Zugriff auf die Küvetten (201) eines ersten Bereichs des stationären, linearen Küvettenarrays (200) und die Vorrichtung (410) zur Durchführung von heterogenen Immunoassays Zugriff auf die Küvetten (201) eines zweiten Bereichs des stationären, linearen Küvettenarrays (200) hat, und

wobei der Pipettor (300a, 300b) und die Küvettenwascheinheit unabhängig voneinander entlang des ersten und des zweiten Bereichs des linearen Küvettenarrays (200) verfahrbar ausgeführt sind und jeweils Zugriff auf unterschiedliche Küvetten (201) beider Bereiche des Küvettenarrays (200) in frei wählbarer Reihenfolge aufweisen.

2. Analysator nach Anspruch 1, **dadurch gekennzeichnet, dass** der Analysator (100) zwei unabhängig voneinander in x-Richtung verfahrbare Pipettoren (300a, 300b) aufweist.

3. Analysator nach Anspruch 1 oder 2, **dadurch gekennzeichnet, dass** zumindest ein Pipettor (300a, 300b) zwei unabhängig voneinander, parallel zueinander in y-Richtung verfahrbare Pipettiernadeln (301a1, 301a2, 301b1, 301b2) aufweist.

4. Analysator nach einem der Ansprüche 1 bis 3, **dadurch gekennzeichnet, dass** die Nadelwascheinheit (700a1, 700a2, 700b1, 700b2) am Pipettor (300a, 300b) angeordnet und mit diesem verfahrbar ausgeführt ist.

5. Analysator nach einem der Ansprüche 1 bis 4, **dadurch gekennzeichnet, dass** die optische Messeinheit (500) eine entlang des ersten Bereichs des linearen, stationären Küvettenarrays (200) verfahrbare Einheit aus einer Lichtbereitstellungseinheit (520) und einem Spektrometer (535) aufweist.

6. Analysator nach einem der Ansprüche 1 bis 4, **dadurch gekennzeichnet, dass** die optische Messeinheit (500) mit einer Lichtbereitstellungseinheit (540) ausgestattet ist, die mehrere im UV/VIS/NIR-Wellenlängenbereich spektral unterschiedlich emittierende LED-Lichtquellen (541) aufweist, sowie mit einer stationären Detektionseinheit (550), die so ausgelegt ist, dass jeder Küvette (201) des ersten Bereichs des Küvettenarrays (200) zumindest eine Fotodiode (551) fix zugeordnet ist.

7. Analysator nach einem der Ansprüche 1 bis 4, **dadurch gekennzeichnet, dass** die Thermostatisiereinheit (800) zur Einstellung einer vorgebbaren Messtemperatur Heizfolien (891) umfasst, die einzelne Küvetten (201) oder Gruppen von Küvetten (201) thermisch kontaktieren und mit unterschiedlichen Temperaturniveaus beaufschlagbar sind.

8. Analysator nach einem der Ansprüche 1 bis 4, **dadurch gekennzeichnet, dass** die Thermostatisiereinheit (800) einen auf eine vorgegebene Zieltemperatur geregelten Küvettenblock (820) aufweist, der mit einer Thermostatisiereinrichtung (830) ausgestattet ist und mit den einzelnen Küvetten (201) in thermischem Kontakt steht.

9. Analysator nach einem der Ansprüche 1 bis 4, **dadurch gekennzeichnet, dass** der Analysator eine Mischereinheit (400) zur Vermischung der Proben und Reagenzien aufweist, wobei an jeder Küvette (201) des ersten Bereichs als stationäre Mischereinheit zumindest ein Ultraschall-Wandler (840) zum Einbringen von Ultraschallenergie in die Küvetten (201) befestigt ist, sowie dass der Ultraschall-Wandler (840) als piezoelektrischer Schwinger ausgeführt ist und mit einer Steuereinheit (860) in Verbindung steht, die den zumindest einen Ultraschall-Wandler (840) in Abhängigkeit von Parameterwerten der flüssigen Medien ansteuert.

10. Analysator nach Anspruch 8 und 9, **dadurch gekennzeichnet, dass** die stationären Vorrichtungen zum Mischen und Thermostatisieren der in die Küvetten (210) des stationären Küvettenarrays (200) eingebrachten flüssigen Medien als kombinierte Misch- und Thermostatisiervorrichtung (810) ausgeführt sind.

11. Analysator nach einem der Ansprüche 1 bis 10, **dadurch gekennzeichnet, dass** die Vorrichtung (410) zur Durchführung von heterogenen Immunoassays folgende Komponenten aufweist:

• zumindest einen, entlang des zweiten Bereichs des Küvettenarrays (200) verfahrbaren und in Richtung der Füllöffnung (207) einer ausgewählten Küvette (201) absenkbaren Haltearm (420) mit zumindest einer, in Richtung Boden (204) der Küvette (201) absenkbaren Absaugnadel (423), sowie mit zumindest einem, über oder in der jeweiligen Füllöffnung (207) positionierbaren Dispensor (424a bis 424d) zur Abgabe der flüssigen Medien in die Küvette (201), wobei zumindest ein Dispensor (424a, 424b) zur Abgabe einer Waschlösung für magnetische Partikel (411) ausgebildet ist,
• zumindest eine entlang des zweiten Bereichs des Küvettenarrays (200) verfahrbare, auf den Inhalt der ausgewählten Küvette (201) wirkende Magnetanordnung (430) zur Separation von magnetischen Partikel (411) an einer Innenfläche der Küvette (201), sowie
• die auf das Messfenster (202) der ausgewählten Küvette (201) des zweiten Bereichs ausrichtbare, optische Detektionseinrichtung (435) zur Aufnahme eines einer Analytkonzentration in der ausgewählten Küvette (201) proportionalen Messsignals.

12. Analysator nach Anspruch 11, **dadurch gekennzeichnet, dass** der zumindest eine Dispensor (424a bis 424d) zur Abgabe der flüssigen Medien in einer auf oder in die Füllöffnung (207) der Küvette (201) absenkbaren Dispensorplattform (421) angeordnet ist, welche von der absenkbaren Absaugnadel (423) durchsetzt wird.

13. Analysator nach Anspruch 11 oder 12, **dadurch gekennzeichnet, dass** der auf der verfahrbaren Plattform (440) angeordnete Haltearm (220) samt Dispensorplattform (421) zusammen mit der Magnetanordnung (430) und der Detektionseinrichtung (435) ein entlang des Küvettenarrays (200) verfahrbares Mess- und Manipulationsmodul (450) bildet, das sämtliche robotische, fluidische und messtechnische Komponenten für die Prozessschritte eines heterogenen Immunoassays vereint.

14. Verfahren zur automatischen chemischen, biochemischen und/oder immunchemischen Analyse von flüssigen Proben, zur Ermittlung zumindest einer Analytkonzentration in der Probe, mit einem automatischen Analysator nach Anspruch 1 **gekennzeichnet durch** folgende Schritte:

a) Transferieren einer vorbestimmten Menge einer flüssigen Probe von einem Probengefäß (921) im Probenlager (920) in eine Küvette (201) des ersten Bereichs des stationären, linearen Küvettenarrays (200) mittels eines entlang des Küvettenarrays verfahrbaren, ersten Pipettors (300b);
b) Transferieren einer vorbestimmten Menge einer Reagenzflüssigkeit von einem Reagenziengefäß (951a) des Reagenzienlagers (950a) in die Küvette (201) des ersten Bereichs des stationären, linearen Küvettenarrays (200) mittels des ersten Pipettors (300b) oder mittels eines zweiten, unabhängig vom ersten verfahrbaren Pipettors (300a);
c) Vermischen und Thermostatisieren der Flüssigkeiten in der Küvette (201) ;
d) gegebenenfalls Transferieren einer vorbestimmten Menge einer weiteren Reagenzflüssigkeit von einem Reagenziengefäß (951b) des Reagenzienlagers (950b) in die Küvette (201) des ersten Bereichs des stationären, linearen Küvettenarrays (200) mittels des ersten oder eines zweiten Pipettors (300a, 300b);
e) gegebenenfalls nochmaliges Vermischen und Thermostatisieren der Flüssigkeiten in der Küvette (201);
f) optische Vermessung des Inhalts der Küvette (201) mittels der optischen Messeinheit (500); und Ermittlung zumindest eines Messwertes;
g) Berechnen und Anzeigen der Analytkonzentration basierend auf den in Punkt f) ermittelten Messwerten und vorbekannten oder vorbestimmten Referenz- und Kalibrierwerten;
h) Waschen und Trocknen der Küvette (201) mittels der entlang des Küvettenarrays (200) verfahrbaren Küvettenwascheinheit (600); sowie
i) Bereitstellen der Küvette (201) für eine nachfolgende Analyse.

**15.** Verfahren nach Anspruch 14, **dadurch gekennzeichnet, dass** in einer gemeinsamen Abfolge zum Mischen und Thermostatisieren des Inhalts der Küvette (201) folgende Schritte durchgeführt werden:

a) Erwärmen der Küvette (201) auf eine vorgegebene Zieltemperatur mit Hilfe des thermostatisierbaren Küvettenblocks (820),
b) Erwärmen der flüssigen Medien mit Hilfe des thermostatisierten Küvettenblocks (820) um die vorgegebene Zieltemperatur zu erreichen,
c) in der Erwärmungsphase gemäß Punkt b), vor dem Erreichen der Zieltemperatur, zusätzliche Einbringung einer vorbestimmten Menge an Ultraschallenergie mit Hilfe zumindest eines an jeder Küvette (201) befestigten Ultraschall-Wandlers (840) zur Erhöhung der Erwärmungsrate, sowie
d) gleichzeitiges Mischen der flüssigen Medien mit Hilfe der in Punkt c) eingebrachten Ultraschallenergie.

**16.** Verfahren nach Anspruch 15, **dadurch gekennzeichnet, dass** zur Unterstützung des Mischvorganges zumindest ein Teil des in die Küvette (201) eingebrachten Flüssigkeitsvolumens zumindest einmal abgesaugt und wieder in die Küvette (201) dispensiert wird.

**17.** Verfahren zur Bestimmung eines Antigens mittels eines heterogenen Immunoassays mit einem automatischen Analysator nach Anspruch 1, **dadurch gekennzeichnet, dass** zunächst in einer ersten Schrittfolge A

eine Probe zur Bestimmung des Antigens,
eine Suspension magnetischer Partikel mit einem Fängerantikörper, sowie
ggf. ein Tracer-Antikörpers oder ein markiertes Antigen

in eine ausgewählte Küvette (201) des zweiten Bereichs des stationären Küvettenarrays (200) einpipettiert werden, sowie dass die folgenden Schritte B einer immunchemischen Analyse, wie

a) Separieren der magnetischen Partikel,
b) ein oder mehrmaliges Einbringen und Absaugen einer Waschlösung,
c) Zudosieren zumindest einer Trigger-Flüssigkeit, sowie
d) luminometrische Vermessung der Probe,

mit Hilfe eines entlang des zweiten Bereichs des Küvettenarrays verfahrbaren Mess- und Manipulationsmoduls (450) erfolgen, das zur Durchführung einzelner oder aller Schritte a) bis d) bei der ausgewählten Küvette (201) angehalten wird.

**18.** Verfahren nach Anspruch 17, **dadurch gekennzeichnet, dass** die in einer Mess- und Manipulationseinheit (450) angeordnete Magnetanordnung (430) und Detektionseinrichtung (435) während des Ablaufs zeitaufwändiger Schritte bei der immunchemischen Analyse, wie Inkubation, etc., in der ausgewählten Küvette, zu zumindest einer weiteren Küvette (201) des zweiten Bereichs des Küvettenarrays (200) verfahren wird, um einzelne oder alle Schritte B einer immunchemischen Analyse durchzuführen.

Fig. 1a (PRIOR ART)

Fig. 1b (PRIOR ART)

Fig. 1c (PRIOR ART)

Fig. 2a (PRIOR ART)

Fig. 2b (PRIOR ART)

Fig. 2c (PRIOR ART)

Fig. 2d (PRIOR ART)

Fig. 2f (PRIOR ART)

Fig. 2h (PRIOR ART)

Fig. 2e (PRIOR ART)

Fig. 2g (PRIOR ART)

*Fig. 3a*

*Fig. 3b*

*Fig. 3c*

Fig. 5

Fig. 4

Fig. 8

Fig. 7

Fig. 6

*Fig. 9a*

*Fig. 9b*

*Fig. 9c*

Fig. 10a

Fig. 10b

Fig. 10c

Fig. 11b

Fig. 11a

Fig. 11f

Fig. 11e

Fig. 11c

Fig. 11d

EP 3 769 842 A1

Fig. 12a

Fig. 12b

Fig. 12c

*Fig. 13b*

*Fig. 13a*

Fig. 14a

Fig. 14b

Fig. 14c

Fig. 15c

Fig. 15b

Fig. 15a

*Fig. 16*

Fig. 17b

Fig. 17a

63

Fig. 18a

Fig. 18b

EP 3 769 842 A1

*Fig. 19a*

Fig. 19b

Fig. 20

Fig. 21

EP 3 769 842 A1

*Fig. 22*

**Europäisches Patentamt**

**European Patent Office**

**Office européen des brevets**

# EUROPÄISCHER RECHERCHENBERICHT

Nummer der Anmeldung

EP 20 19 4310

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 322 939 A1 (BIT ANALYTICAL INSTR GMBH [DE]) 18. Mai 2011 (2011-05-18) * Zusammenfassung * * Absätze [0065] - [0086] * * Abbildungen 1,3 * ----- | 1-18 | INV. B01L3/02 G01N35/10 G01N21/25 G01N35/00 G01N35/04 |
| X | EP 0 644 426 A1 (HOFFMANN LA ROCHE [CH]) 22. März 1995 (1995-03-22) * Zusammenfassung * * Seite 1, Zeilen 5-10 * * Seite 1, Zeile 38 - Seite 4, Zeile 22 * * Abbildung 1 * ----- | 1-18 | G01N35/02 B01L3/00 G01N21/00 |
| X | EP 1 615 037 A1 (ROCHE DIAGNOSTICS GMBH [DE]; HOFFMANN LA ROCHE [CH]) 11. Januar 2006 (2006-01-11) * Zusammenfassung * * Absätze [0032] - [0036], [0041] - [0043] * * Abbildung 1 * ----- | 1-18 | |
| A,D | WO 99/46601 A1 (HITACHI LTD [JP]; SHIMIZU HAYATO [JP] ET AL.) 16. September 1999 (1999-09-16) * das ganze Dokument * ----- | 1-18 | **RECHERCHIERTE SACHGEBIETE (IPC)** B01L G01N |

Der vorliegende Recherchenbericht wurde für alle Patentansprüche erstellt

| Recherchenort | Abschlußdatum der Recherche | Prüfer |
|---|---|---|
| Den Haag | 18. Dezember 2020 | Sinn, Cornelia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

 
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 20 19 4310

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-12-2020

| Im Recherchenbericht angeführtes Patentdokument | Datum der Veröffentlichung | Mitglied(er) der Patentfamilie | Datum der Veröffentlichung |
|---|---|---|---|
| EP 2322939 A1 | 18-05-2011 | DE 212010000179 U1 | 24-08-2012 |
| | | DK 2502082 T3 | 27-07-2015 |
| | | EP 2322939 A1 | 18-05-2011 |
| | | EP 2502082 A1 | 26-09-2012 |
| | | ES 2541215 T3 | 16-07-2015 |
| | | WO 2011058169 A1 | 19-05-2011 |
| EP 0644426 A1 | 22-03-1995 | AT 266206 T | 15-05-2004 |
| | | CA 2131844 A1 | 18-03-1995 |
| | | DK 0644426 T3 | 06-09-2004 |
| | | EP 0644426 A1 | 22-03-1995 |
| | | ES 2219643 T3 | 01-12-2004 |
| | | JP 2825766 B2 | 18-11-1998 |
| | | JP H07174763 A | 14-07-1995 |
| | | PT 644426 E | 31-08-2004 |
| | | US 5482863 A | 09-01-1996 |
| | | US 5482864 A | 09-01-1996 |
| EP 1615037 A1 | 11-01-2006 | KEINE | |
| WO 9946601 A1 | 16-09-1999 | JP 3578084 B2 | 20-10-2004 |
| | | WO 9946601 A1 | 16-09-1999 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8911685 B2
- DE 112009002702 B4
- US 5178833 A
- GB 1321754 A
- US 20140287523 A1
- WO 99046601 A1
- EP 2309251 A1
- EP 0259386 B1
- DE 102004057450 B4
- EP 2410342 A2
- EP 1230553 B1
- US 5897837 A

- US 8675187 B2
- US 20130301051 A1
- US 8064062 B2
- AT 510631 B1
- WO 2010122203 A1
- US 4234539 A
- DE 2726498 A1
- JP 2007303964 A
- EP 1995597 A1
- JP 2007010345 A
- US 7998432 B2
- US 6333008 B1